# EUROPEAN PATENT APPLICATION

(11) **EP 1 738 752 A1**
(43) Date of publication of application: **03.01.2007**
(21) Application number: 06090154.3
(22) Date of filing: 11.12.2003
(51) Int. Cl.: A61K 31/00, A61K 31/4406

(54) **Pharmaceutical combinations comprising cis-retine acid**

(30) Priority: 27.12.2002 EP 02090431; 12.03.2003 EP 03090061
(62) Divisional of application: 03782372.1
(71) Applicant: Schering Aktiengesellschaft, 13353 Berlin (DE)
(72) Inventor: Schuppan, Detlef, 91988 Bubenreuth (DE); Herold, Christoph, 90518 Altdorf (DE); Ganslmayer, Marion, 91054 Erlangen (DE); Ocker, Matthias, 91083 Baiersdorf (DE); Thierauch, Karl-Heinz, 14169 Berlin (DE)

(57) **Abstract**

Pharmaceutical combinations comprising at least 9cis-retine acid (CRA), 13cis-retine acid, or a derivative thereof, at least one compound of general formula II), and an anti-hormone, and their use for the treatment of different diseases resulting by persistent angiogenesis are described.

## Description

The invention concerns pharmaceutical combination and their use for the treatment of diseases resulting from persistent angiogenesis.

Persistent angiogenesis can be the source for different diseases, such as for example psoriasis, arthritis, such as rheumatoid arthritis, haemeangioma, angiofribroma, diseases of the eyes, such as diabetic retinopathie, neovascular glaucoma, diseases of the kidney, such as glomerulonephritis, diabetic nephropatic desease, malignant nephrosclerosis, thrombotic microangiopatic syndrome, disposes of transplants and glomerulopathy, fibrotic diseases, such as liver cirrhosis, mesangial cell proliferative diseases and artheriosclerosis, or can be change for the worse of these diseases.

Histone modification, catalized by the histone acetyltansferase (HAT) and histone deacetylase (HDAC) enzymes, plays an important role in regulating gene expression by altering chromatin structure. Histone acetylation results in charge neutralization and separation of DNA from the histones. Thus, transcriptionally activated genes are typically associated with hyperacetylated loci. Because of the profound effect of histone modification on gene transcription, manipulation of the activities of histone- modifing HAT and HDAC enzymes has the potential for modifing the cell cycle and the neoplastic transfomation of cells.
Some compounds with deacetylase inhibitory activity have been shown to be effective in suppressing tumor growth in animal models and in initial clinical trials, although the efficacy is limited, possibly due to their stability, low retention, or nonspecific toxicity in the body.

In addition, HDAC enzymes play a role in the regulation of hypoxia-induced angiogenesis. Hypoxia enhances HDAC function and HDAC is closely involved in angiogenesis through suppression of hypoxia-responsive tumor suppressor genes. A specific HDAC inhibitor upregulates p53 and von Hippel-Lindau expression and downregulates hypoxia-induced factor-1alpha and vascular endothelial growth factor (VEGF). HDAC inhibitors are also shown to inhibit angiogenesis both in vitro and in vivo.

HDAC inhibitors are currently of major interest as potential anti-cancer therapeutics, largely because of their well-documented properties of inhibiting proliferation and induce apotosis of tumour cells. Furthermore, the finding that HDAC appears to be a critical regulator of angiogenesis in addition to tumour cell growth will cause further interest in the development of HDAC inhibitors as potential anticancer drugs.

In Kim M. S. et al. (Nature med. Apr. 2001 vol. 7, no. 4) and Deroanne Christopher et al. (Oncogene, 17 Jan. 2002, vol. 21, no. 3) the HDAC-inhibitor Trichostatin A which downregulates VEGF and blocks angiogenesis in vitro and in vivo is described.

In EP 0847992 (US 6,174,905) benzamide derivatives are described which show differentiation-inducing effects, and which are useful a therapeutic or improving agent for malignant tumors, autoimmune diseases, dermatologic diseases and parasitism. In particular, they are highly effective as an anticancer drug, specifically to a hematologic malignancy and a solid carcinoma.

These are compounds of general formula II 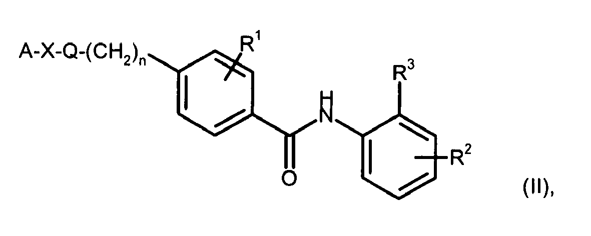 wherein
A is an optionally substituted phenyl group or an optionally substituted heterocyclic group wherein the substituent(s) for the phenyl group or the heterocyclic group is (are) 1 to 4 substituents selected from the group consisting of a halogen atom, a hydroxyl group, an amino group, a nitro group, a cyano group, an alkyl group having 1 to 4 carbons, an alkoxy group having 1 to 4 carbons, an aminoalkyl group having 1 to 4 carbons, an alkylamino group having 1 to 4 carbons, an acyl group having 1 to 4 carbons, an acylamino group having 1 to 4 carbons, an alkylthio group having 1 to 4 carbons, a perfluoroalkyl group having 1 to 4 carbons, a perfluoroalkyloxy group having 1 to 4 carbons, a carboxyl group, an alkoxycarbonyl group having 1 to 4 carbons, a phenyl group and a heterocyclic group;
X is a bond or a moiety having the following structure 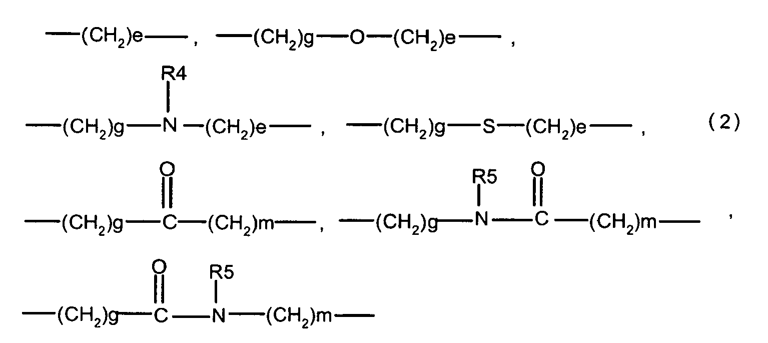 wherein e is an integer of 1 to 4; g and m are independently an integer of 0 to 4; R⁴ is a hydrogen atom or an optionally substituted alkyl group having 1 to 4 carbons, or the acyl group represented by formula (3) 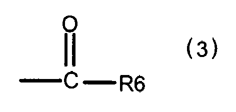 wherein R⁶ is an optionally substituted alkyl group having 1 to 4 carbons, a perfluoroalkyl group having 1 to 4 carbons, a phenyl group or a heterocyclic group; R⁵ is a hydrogen atom or an optionally substituted alkyl group having 1 to 4 carbons;
n is an integer of 0 to 4, provided that when X is a bond, n is not zero;
Q is a moiety having a structure selected from those illustrated in formula (4) 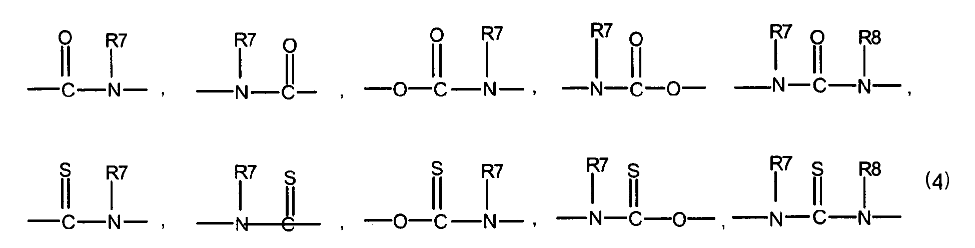 wherein R⁷ and R⁸ are independently a hydrogen atom or an optionally substituted alkyl group having 1 to 4 carbons;
R¹ and R² are independently a hydrogen atom, a halogen atom, a hydroxyl group, an amino group, an alkyl group having 1 to 4 carbons, an alkoxy group having 1 to 4 carbons, an aminoalkyl group having 1 to 4 carbons, an alkylamino group having 1 to 4 carbons, an acyl group having 1 to 4 carbons, an acylamino group having 1 to 4 carbons, an alkylthio group having 1 to 4 carbons, a perfluoroalkyl group having 1 to 4 carbons, a perfluoroalkyloxy group having 1 to 4 carbons, a carboxyl group or an alkoxycarbonyl group having 1 to 4 carbons;
R³ is a hydroxyl or amino group or a pharmaceutically acceptable salt thereof.

Of special interest are those compounds of formula II), wherein n is an integer of 1 to 4.

Of further interest are those compounds wherein Q is selected from the structures illustrated in formula (5): 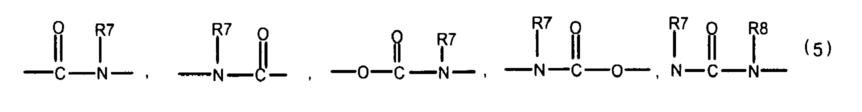 wherein R7 and R8 are as defined above.

Of further interest are also those compounds wherein A is an optionally substituted hetero ring, especially an optionally substituted pyridyl group.

Of special interest are also those compounds of general formula II), wherein n is 1 to 4; Q is selected from the structures illustrated in formula (5); A is an optionally substituted hetero ring, especially optionally substituted pyridyl group; most preferred, wherein X is direct bond, most preferred wherein R¹ and R² are a hydrogen atom, most preferred, wherein R³ is an amino group.

Of special interest are also those compounds of general formula II), wherein n is 1 to 4; wherein Q is selected from the structures illustrated in formula (5); A is an optionally substituted hetero ring, especially optionally substituted pyridyl group; most preferred, wherein X is the structure represented by formula (6):

-(CH₂)e- (6)

wherein e is an integer of 1 to 4; most preferred wherein n is 1 and R¹ and R² are a hydrogen atom; most preferred, wherein R³ is an amino group.

Of special interest are also those compounds of general formula II), wherein Q is selected from the structures illustrated in formula (5); A is an optionally substituted hetero ring, especially optionally substituted pyridyl group; most preferred, wherein X is selected from the structures illustrated in formula (7):
wherein e, g and R4 are as defined above; most preferred wherein n is 1 and R¹ 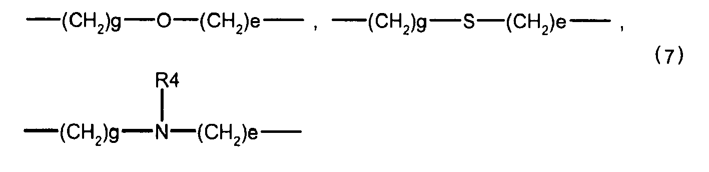 and R² are a hydrogen atom; most preferred, wherein R³ is an amino group.

Of special interest are also those compounds of general formula II, wherein Q is selected from the structures illustrated in formula (5); A is an optionally substituted hetero ring, especially optionally substituted pyridyl group; most preferred, wherein X is selected from the structures illustrated in formula (8): 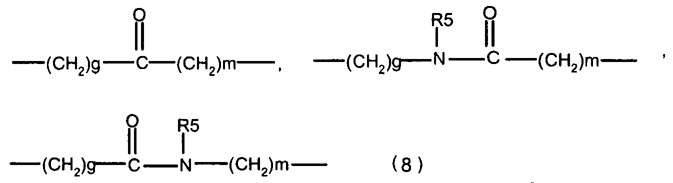 wherein g, m and R5 are as defined above; most preferred wherein n is 1 and R¹ and R² are a hydrogen atom; most preferred, wherein R³ is an amino group.

Of special interest are also those compounds of general formula II), wherein n is zero, and further of interest, wherein Q is selected from the structures illustrated in formula (5), and further of interest, wherein A is an optionally substituted hetero ring, most preferred, wherein A is anoptionally substituted pyridyl group, most preferred, wherein R¹ and R² are a hydrogen atom, most preferred,
wherein R³ is an amino group.

Selected compounds of general formula II are for example the following compounds: 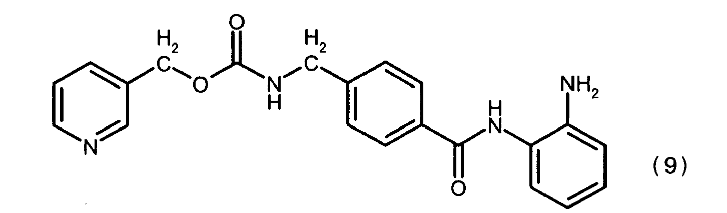 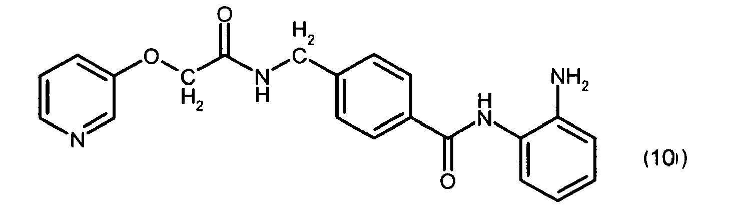 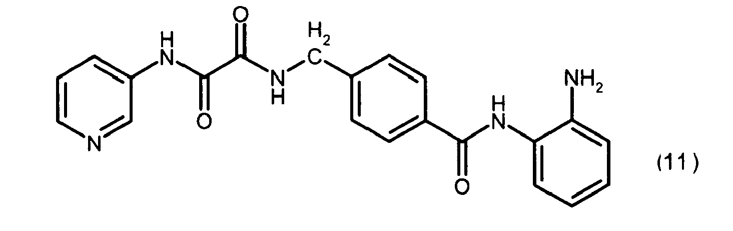 and

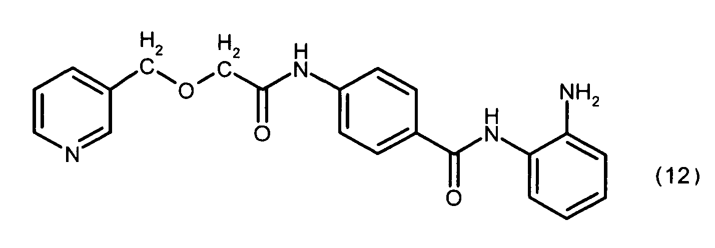

In the above formula (II), n may be zero or an integer of 1 to 4.

Q in the above formula (II) may be any structure illustrated in formula (5); 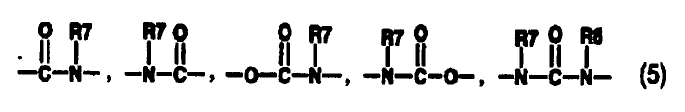 wherein R7 and R8 are as defined above.

X in the above formula (II) may be a moiety having the structure represented by formula (6);

**―(CH₂)e―** **(6)**

wherein e is as defined above.

X in the above formula (II) may be also a moiety having any structure illustrated in formula (7); 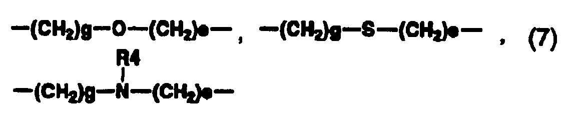 wherein e, g and R⁴ are as defined above.

X in the above formula (II) may be also a moiety having any structure illustrated in formula (8); 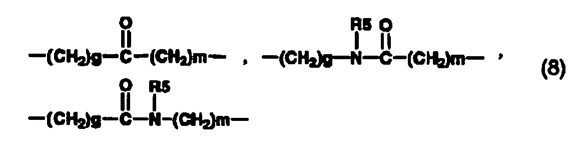 wherein g, m and R⁵ are as defined above.

As used herein, "1 to 4 carbons" means a carbon number per a single substituent; for example, for dialkyl substitution it means 2 to 8 carbons.

A heterocycle in the compound represented by formula (II) or (IIa) is a monocyclic heterocycle having 5 or 6 members containing 1 to 4 nitrogen, oxygen or sulfur atoms or a bicyclic-fused heterocycle. The monocyclic heterocycle includes pyridine, pyrazine, pyrimidine, pyridazine, thiophene, furan, pyrrole, pyrazole, isoxazole, isothiazole, imidazole, oxazole, thiazole, piperidine, piperazine, pyrrolidine, quinuclidine, tetrahydrofuran, morpholine, thiomorpholine and the like. The bicyclic fused heterocycle includes quinoline; isoquinoline; naphthyridine; fused pyridines such as furopyridine, thienopyridine, pyrrolopyridine, oxazolopyridine, imidazolopyridine and thiazolopyridine; benzofuran; benzothiophene; benzimidazole and the like.

A halogen may be fluorine, chlorine, bromine or iodine.

An alkyl having 1 to 4 carbons includes methyl, ethyl, n-propyl, isopropyl, n-butyl, isobutyl, sec-butyl and tert-butyl.

An alkoxy having 1 to 4 carbons includes methoxy, ethoxy, n-propoxy, isopropoxy, allyloxy, n-butoxy, isobutoxy, sec-butoxy, tert-butoxy and the like.

An aminoalkyl having 1 to 4 carbons includes aminomethyl, 1-aminoethyl, 2-aminopropyl and the like.

An alkylamino having 1 to 4 carbons includes N-methylamino, N,N-dimethylamino, N,N-diethylamino, N-methyl-N-ethylamino, N,N-diisopropylamino and the like.

An acyl having 1 to 4 carbons includes acetyl, propanoyl, butanoyl and like.

An acylamino having 1 to 4 carbons includes acetylamino, propanoylamino, butanoylamino and the like.

An alkylthio having 1 to 4 carbons includes methylthio, ethylthio, propylthio and the like.

A perfluoroalkyl having 1 to 4 carbons includes trifluoromethyl, pentafluoroethyl and the like.

A perfluoroalkyloxy having 1 to 4 carbons includes trifluoromethoxy, pentafluoroethoxy and the like.

An alkoxycarbonyl having 1 to 4 carbons includes methoxycarbonyl and ethoxycarbonyl.

An optionally substituted alkyl having 1 to 4 carbons includes methyl, ethyl, n-propyl, isopropyl, n-butyl, isobutyl, sec-butyl and tert-butyl and these having 1 to 4 substituents selected from the group consisting of a halogen, hydroxyl, amino, nitro, cyano, phenyl and a heterocycle.

A basic cyclic structure includes cyclic moieties having 4 to 7 members containing carbons and/or hetero atoms or their fused cycles. For example it may be cyclobutane, cyclopentane, cyclohexane, cycloheptane, oxetane, oxolane, oxane, oxepane, pyrrolidine, imidazolidine, pyrazolidine, piperidine, piperazine, indoline, isoindoline, thiolane, thiazolidine and oxazolidine rings, which may contain unsaturated bonds, hydrogen bond acceptors and/or substituents.

In the above analyses, other commercially available program packages such as CATALYST(MSI), Cerius 2/QSAR+(MSI) and SYBYL/DISCO(Tripos) may be used, and the information on distance obtained here is not limited to that from a particular calculation program.

The ring centroid used in definition of the spatial configuration may be defined as an average of X, Y and Z axes of the ring-forming atoms. When a ring structure to be calculated is fused-polycyclic, the centroid of either the overall fused ring or of a partial ring may be used as that for defining the space.

"Possibility of formation of a configuration" means that a conformer filling the spatial configuration is within 15 kcal/mol, preferably 8 kcal/mol from the energetically most stable structure.

Specific calculation can be performed as described in the instructions for Sybyl (M.Clark) or J.Comput.Chem. 10, 982(1989).

A pharmaceutically acceptable salt of the compound of this invention includes salts with an inorganic acid such as hydrochloric acid, hydrobromic acid, sulfuric acid and phosphoric acid; and with an organic acid such as acetic acid, lactic acid, tartaric acid, malic acid, succinic acid, fumaric acid, maleic acid, citric acid, benzoic acid, trifluroacetic acid, p-toluenesulfonic acid and methanesulfonic acid. Such a salt includes N-(2-aminophenyl)-4-[N-(pyridin-3-yl)methoxycarbonylaminomethyl]benzamide hydrochloride, N-(2-aminophenyl)-4-[N-(pyridin-3-yl)methoxycarbonylaminomethyl]benzamidehydrobromide, N-(2-aminophenyl)-4-[N-(pyridin-3-yl)methoxycarbonylaminomethyl]benzamide sulfate, N-(2-aminophenyl)-4-[N-(pyridin-3-yl)methoxycarbonylaminomethyl]benzamide phosphate, N-(2-aminophenyl)-4-[N-(pyridin-3-yl)methoxycarbonylaminomethyl]benzamide acetate, N-(2-aminophenyl)-4-[N-(pyridin-3-yl)methoxycarbonylaminomethyl]benzamide lactate, N-(2-aminophenyl)-4-[N-(pyridin-3-yl)methoxycarbonylaminomethyl]benzamide tartrate, N-(2-aminophenyl)-4-[N-(pyridin-3-yl)methoxycarbonylaminomethyl]benzamide malate, N-(2-aminophenyl)-4-[N- (pyridin-3-yl)methoxycarbonylaminomethyl]benzamide succinate, N-(2-aminophenyl)-4-[N-(pyridin-3-yl )methoxycarbonylaminomethyl]benzamide fumarate, N-(2-aminophenyl)-4-[N-(pyridin-3-yl)methoxycarbonylaminomethyl]benzamide maleate, N-(2-aminophenyl)-4-[N-(pyridin-3-yl)methoxycarbonylaminomethyl]benzamide citrate, N-(2-aminophenyl)-4-[N-(pyridin-3-yl)methoxycarbonylaminomethyl]benzamide trifluoroacetate, N-(2-aminophenyl)-4-[N-(pyridin-3- yl)methoxycarbonylaminomethyl]benzamide p-toluenesulfonate and N-(2-aminophenyl)-4-[N-(pyridin-3-yl)methoxycarbonylaminomethyl]benzamide methanesulfonate.

When having asymmetric carbon or carbons, the compound represented by formula (II) may be obtained as an individual stereoisomer or a mixture of stereoisomers including a racemic modification. This invention encompasses the above-specified different forms, which may be also used as an active ingredient.

Representative compounds of formula (II) are specifically shown in Tables 1 to 4, but this invention is not intended to be limited to these.

**Table 1**

| | | | | | | | |
|---|---|---|---|---|---|---|---|
| 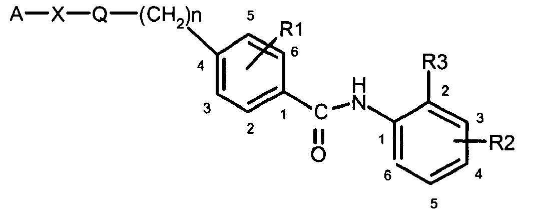 | | | | | | | |

| Compound No. | A | X | Q | n | R1 | R2 | R3 |
|---|---|---|---|---|---|---|---|
| 1 | 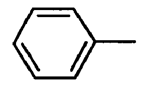 | Direct bond | 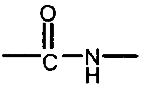 | 1 | H | H | NH₂ |
| 2 | 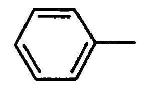 | -CH₂- | 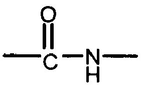 | 0 | H | H | NH₂ |
| 3 | 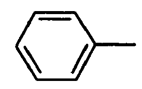 | -(CH₂)₂- | 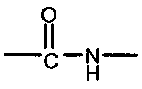 | 0 | H | H | NH₂ |
| 4 | 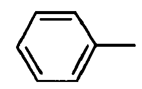 | -(CH₂)₃- | 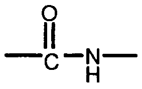 | 0 | H | H | NH₂ |
| 5 | 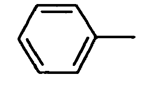 | -(CH₂)₄- | 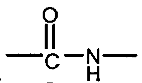 | 0 | H | H | NH₂ |
| 6 | 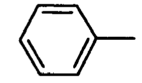 | -CH₂- | 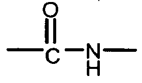 | 1 | H | H | NH₂ |
| 7 | 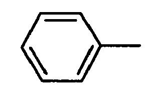 | -(CH₂)₂- | 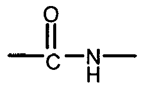 | 1 | H | H | NH₂ |
| 8 | 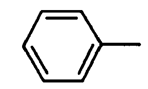 | -CH₂- | 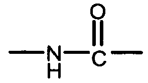 | 0 | H | H | NH₂ |
| 9 | 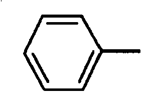 | -(CH₂)₂- | 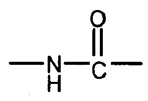 | 0 | H | H | NH₂ |
| 10 | 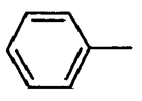 | Direct bond | 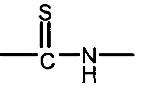 | 1 | H | H | NH₂ |
| 11 | 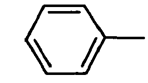 | -CH₂- | 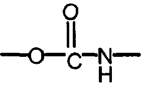 | 1 | H | H | NH₂ |
| 12 | 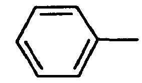 | Direct bond | 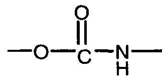 | 1 | H | H | NH₂ |
| 13 | 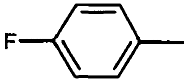 | Direct bond | 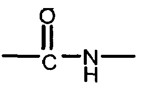 | 1 | H | H | NH₂ |
| 14 | 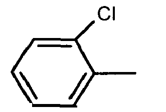 | Direct bond | 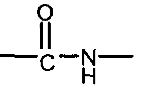 | 1 | H | H | NH₂ |
| 15 | 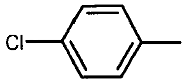 | -CH₂- | 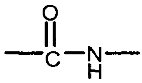 | 0 | H | H | NH₂ |
| 16 | 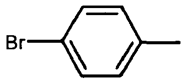 | Direct bond | 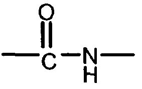 | 1 | H | H | NH₂ |
| 17 | 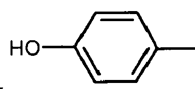 | Direct bond | 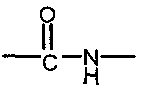 | 1 | H | H | NH₂ |
| 18 | 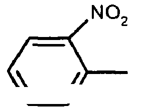 | Direct bond | 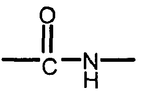 | 1 | H | H | NH₂ |
| 19 | 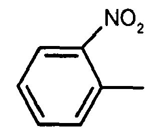 | -CH₂- | 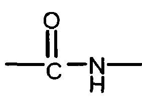 | 0 | H | H | NH₂ |
| 20 | 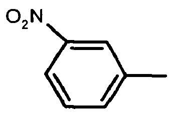 | Direct bond | 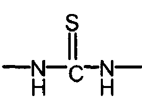 | 1 | H | H | NH₂ |
| 21 | 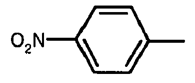 | -CH₂- | 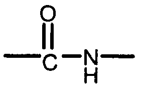 | 0 | H | H | NH₂ |
| 22 | 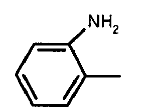 | -CH₂- | 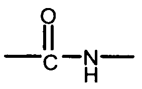 | 0 | H | H | NH₂ |
| 23 | 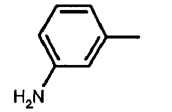 | -CH₂- | 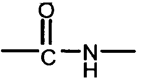 | 1 | H | H | NH₂ |
| 24 | 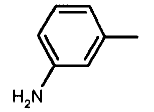 | Direct bond | 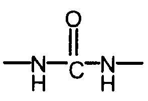 | 1 | H | H | NH₂ |
| 25 | 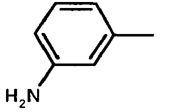 | Direct bond | 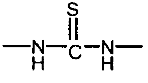 | 1 | H | H | NH₂ |
| 26 | 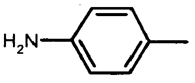 | -CH₂- | 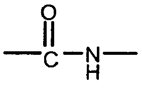 | 0 | H | H | NH₂ |
| 27 | 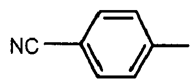 | Direct bond | 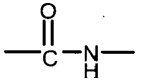 | 1 | H | H | NH₂ |
| 28 | 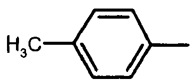 | Direct bond | 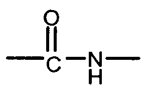 | 1 | H | H | NH₂ |
| 29 | 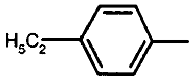 | Direct bond | 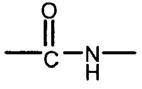 | 1 | H | H | NH₂ |
| 30 | 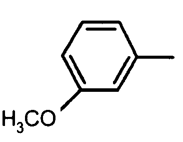 | Direct bond | 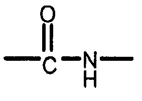 | 1 | H | H | NH₂ |
| 31 | 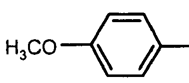 | Direct bond | 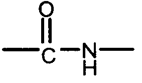 | 1 | H | H | NH₂ |
| 32 | 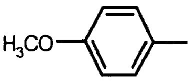 | -CH₂- | 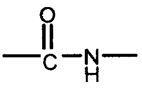 | 0 | H | H | NH₂ |
| 33 | 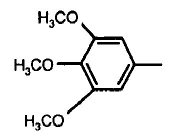 | Direct bond | 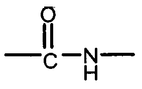 | 1 | H | H | NH₂ |
| 34 | 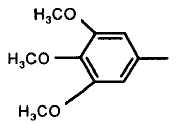 | -CH₂- | 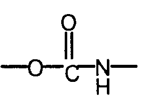 | 1 | H | H | NH₂ |
| 35 | 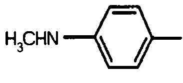 | Direct bond | 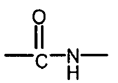 | 1 | H | H | NH₂ |
| 36 | 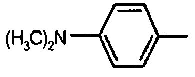 | Direct bond | 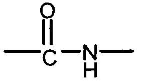 | 1 | H | H | NH₂ |
| 37 | 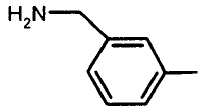 | Direct bond | 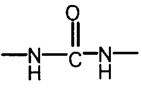 | 1 | H | H | NH₂ |
| 38 | 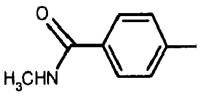 | -CH₂- | 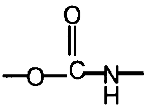 | 1 | H | H | NH₂ |
| 39 | 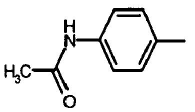 | -CH₂- | 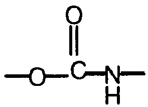 | 1 | H | H | NH₂ |
| 40 | 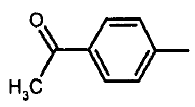 | Direct bond | 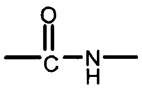 | 1 | H | H | NH₂ |
| 41 | 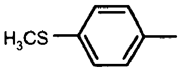 | Direct bond | 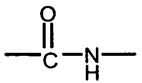 | 1 | H | H | NH₂ |
| 42 | 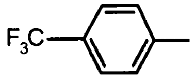 | Direct bond | 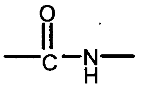 | 1 | H | H | NH₂ |
| 43 | 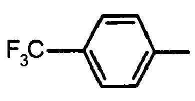 | -CH₂- | 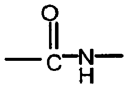 | 0 | H | H | NH₂ |
| 44 | 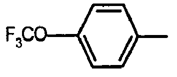 | Direct bond | 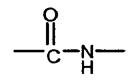 | 1 | H | H | NH₂ |
| 45 | 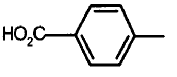 | Direct bond | 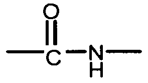 | 1 | H | H | NH₂ |
| 46 | 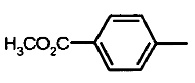 | Direct bond | 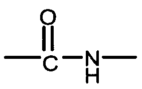 | 1 | H | H | NH₂ |
| 47 | 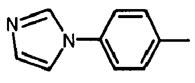 | -CH₂- | 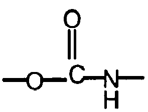 | 1 | H | H | NH₂ |
| 48 | 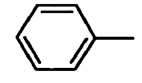 | -O-CH₂- | 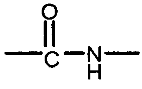 | 1 | H | H | NH₂ |
| 49 | 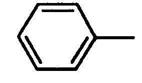 | -S-CH₂⁻ | 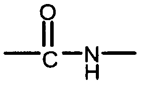 | 1 | H | H | NH₂ |
| 50 | 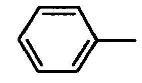 | 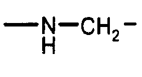 | 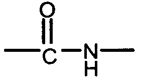 | 1 | H | H | NH₂ |
| 51 | 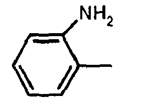 | -CH₂- | 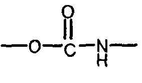 | 1 | H | H | NH₂ |
| 52 | 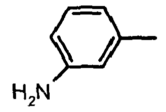 | -CH₂- | 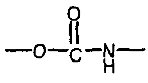 | 1 | H | H | NH₂ |
| 53 | 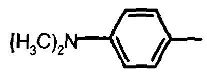 | -CH₂- | 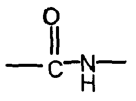 | 0 | H | H | NH₂ |
| 54 | 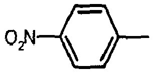 | -O-CH₂- | 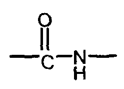 | 0 | H | H | NH₂ |
| 55 | 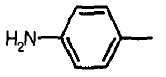 | -O-CH₂- | 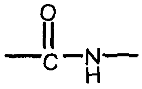 | 0 | H | H | NH₂ |
| 56 | 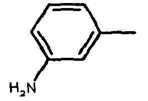 | -O-CH₂- | 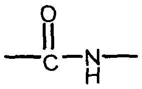 | 1 | H | H | NH₂ |
| 57 | 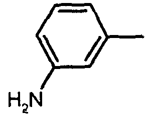 | -O-CH₂- | 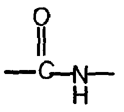 | 1 | H | H | NH₂ |
| 58 | 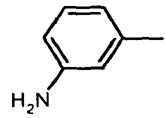 | -CH₂-O-CH₂- | 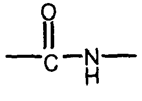 | 0 | H | H | NH₂ |
| 59 | 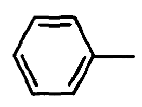 | 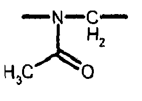 | 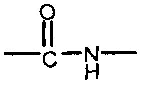 | 1 | H | H | NH₂ |
| 60 | 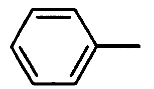 | 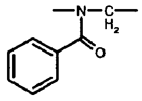 | 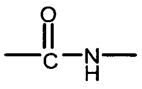 | 1 | H | H | NH₂ |
| 61 | 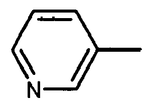 | -O-CH₂- | 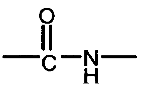 | 1 | H | H | NH₂ |
| 62 | 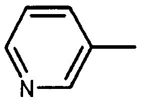 | -O-(CH₂)₂- | 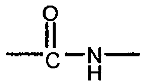 | 1 | H | H | NH₂ |
| 63 | 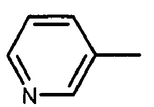 | 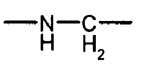 | 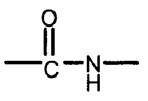 | 1 | H | H | NH₂ |
| 64 | 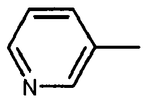 | -S-CH₂- | 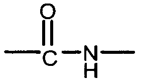 | 1 | H | H | NH₂ |
| 65 | 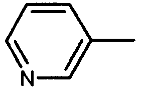 | -O-CH₂- | 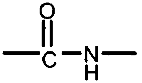 | 0 | H | H | NH₂ |
| 66 | 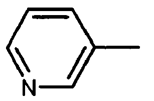 | -O-(CH₂)₂- | 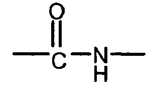 | 0 | H | H | NH₂ |
| 67 | 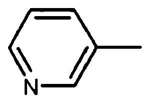 | -O-(CH₂)₂- | 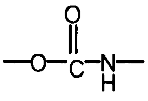 | 0 | H | H | NH₂ |
| 68 | 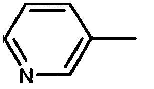 | -CH₂- | 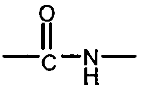 | 0 | H | H | NH₂ |
| 69 | 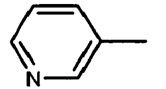 | -(CH₂)₂- | 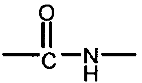 | 0 | H | H | NH₂ |
| 70 | 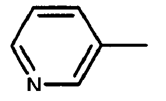 | -(CH₂)₃- | 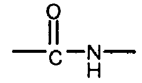 | 0 | H | H | NH₂ |
| 71 | 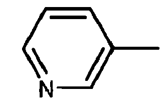 | Direct bond | 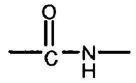 | 1 | H | H | NH₂ |
| 72 | 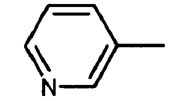 | Direct bond | 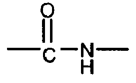 | 2 | H | H | NH₂ |
| 73 | 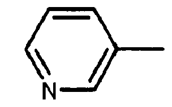 | Direct bond | 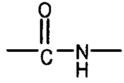 | 3 | H | H | NH₂ |
| 74 | 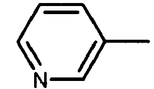 | -CH₂- | 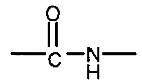 | 1 | H | H | NH₂ |
| 75 | 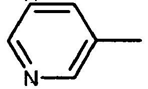 | -(CH₂)₂- | 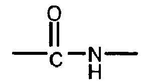 | 1 | H | H | NH₂ |
| 76 | 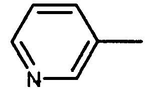 | -(CH₂)₃- | 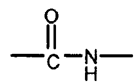 | 1 | H | H | NH₂ |
| 77 | 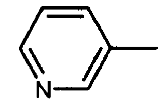 | -CH₂- | 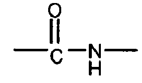 | 2 | H | H | NH₂ |
| 78 | 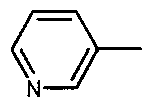 | -CH₂- | 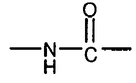 | 1 | H | H | NH₂ |
| 79 | 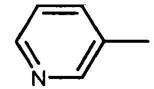 | Direct bond | 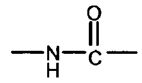 | 2 | H | H | NH₂ |
| 80 | 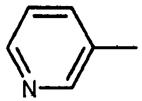 | -CH₂- | 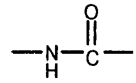 | 2 | H | H | NH₂ |
| 81 | 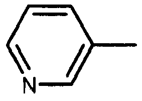 | Direct bond | 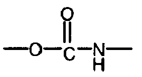 | 1 | H | H | NH₂ |
| 82 | 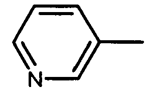 | -CH₂- | 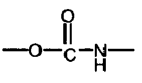 | 1 | H | H | NH₂ |
| 83 | 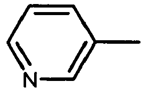 | -(CH₂)₂- | 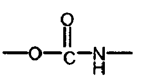 | 1 | H | H | NH₂ |
| 84 | 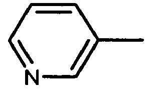 | -(CH₂)₃- | 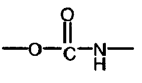 | 1 | H | H | NH₂ |
| 85 | 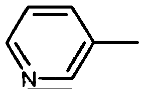 | -CH₂- | 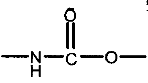 | 1 | H | H | NH₂ |
| 86 | 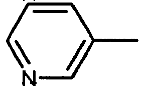 | -CH₂- | 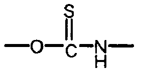 | 1 | H | H | NH₂ |
| 87 | 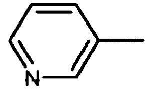 | Direct bond | 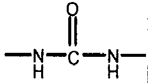 | 1 | H | H | NH₂ |
| 88 | 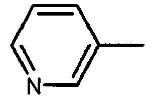 | -CH₂- | 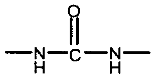 | 1 | H | H | NH₂ |
| 89 | 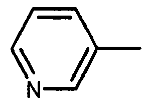 | -(CH₂)₂- | 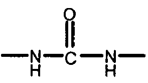 | 1 | H | H | NH₂ |
| 90 | 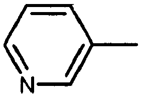 | -CH₂- | 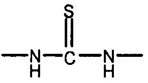 | 1 | H | H | NH₂ |
| 91 | 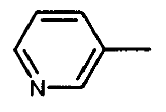 | -O-CH₂- | 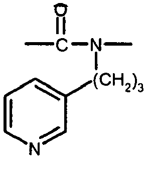 | 1 | H | H | NH₂ |
| 92 | 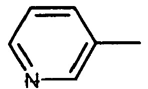 | -O-CH₂- | 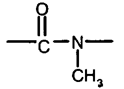 | 1 | H | H | NH₂ |
| 93 | 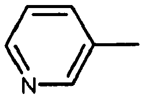 | -O-CH₂- | 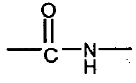 | 1 | H | H | NH₂ |
| 94 | 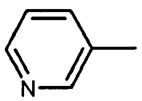 | 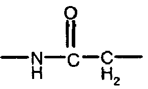 | 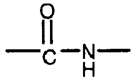 | 0 | H | H | NH₂ |
| 95 | 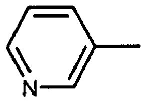 | 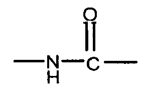 | 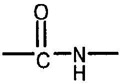 | 1 | H | H | NH₂ |
| 96 | 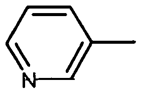 | 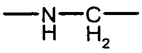 | 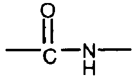 | 1 | H | H | NH₂ |
| 97 | 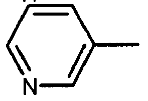 | 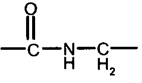 | 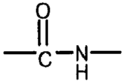 | 0 | H | H | NH₂ |
| 98 | 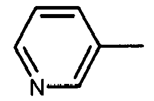 | 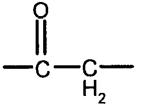 | 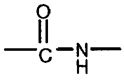 | 1 | H | H | NH₂ |
| 99 | 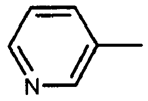 | 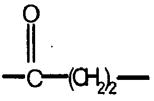 | 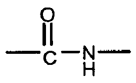 | 0 | H | H | NH₂ |
| 100 | 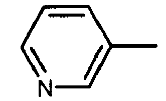 | 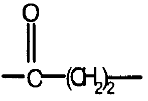 | 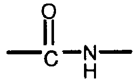 | 1 | H | H | NH₂ |
| 101 | 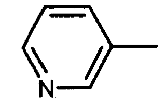 | -CH₂-O-CH₂- | 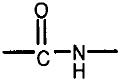 | 0 | H | H | NH₂ |
| 102 | 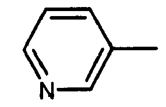 | -CH₂-O-CH₂- | 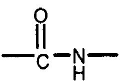 | 0 | 3-CH₃ | H | NH₂ |
| 103 | 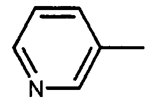 | -CH₂-O-CH₂- | 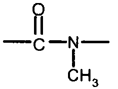 | 0 | H | H | NH₂ |
| 104 | 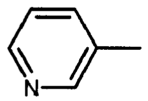 | 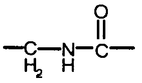 | 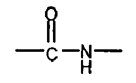 | 0 | H | H | NH₂ |
| 105 | 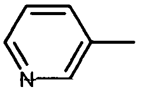 | 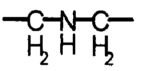 | 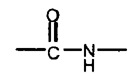 | 0 | H | H | NH₂ |
| 106 | 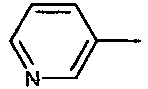 | 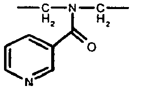 | 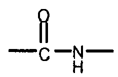 | 0 | H | H | NH₂ |
| 107 | 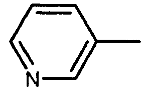 | 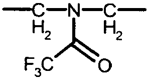 | 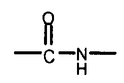 | 1 | H | H | NH₂ |
| 108 | 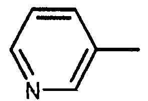 | 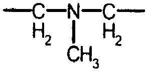 | 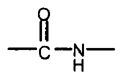 | 0 | H | H | NH₂ |
| 109 | 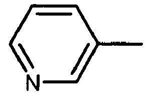 | -CH₂- | 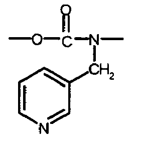 | 1 | H | H | NH₂ |
| 110 | 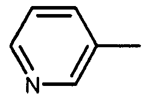 | -CH₂- | 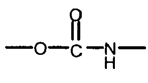 | 1 | H | 5-F | NH₂ |
| 111 | 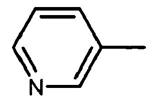 | -CH₂- | 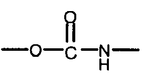 | 1 | H | H | OH |
| 112 | 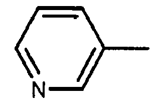 | -CH₂- | 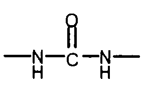 | 1 | H | 5-F | NH₂ |
| 113 | 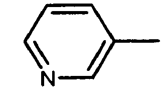 | -CH₂- | 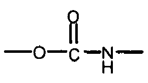 | 1 | H | 4-Cl | NH₂ |
| 114 | 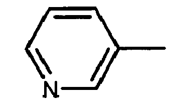 | -CH₂- | 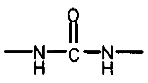 | 1 | H | H | OH |
| 115 | 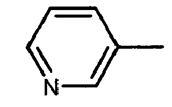 | -CH₂- | 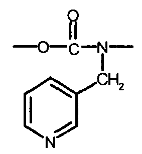 | 1 | H | H | OH |
| 116 | 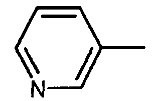 | -CH₂- | 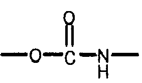 | 1 | H | 4-OH | OH |
| 117 | 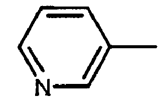 | -CH₂- | 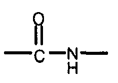 | 1 | H | H | OH |
| 118 | 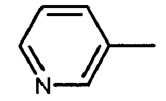 | -CH₂- | 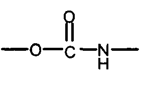 | 1 | H | 5-CH₃ | OH |
| 119 | 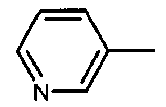 | -CH₂- | 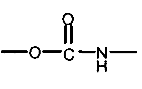 | 1 | H | 5-OCH₃ | OH |
| 120 | 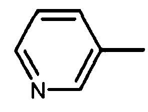 | -CH₂- | 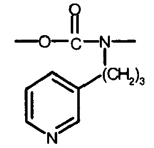 | 1 | H | H | NH₂ |
| 121 | 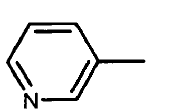 | -CH2- | 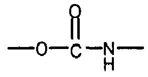 | 1 | H | 5-OCH₃ | NH₂ |
| 122 | 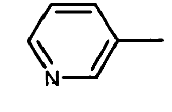 | -(CH₂)₃- | 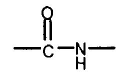 | 0 | H | 5-F | NH₂ |
| 123 | 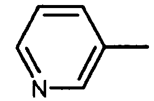 | -(CH₂)₂- | 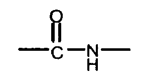 | 0 | 3-Cl | H | NH₂ |
| 124 | 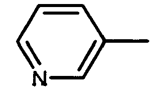 | -(CH₂)₂- | 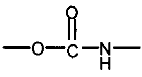 | 0 | H | H | NH₂ |
| 125 | 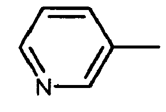 | -(CH₂)₂- | 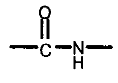 | 1 | H | H | OH |
| 126 | 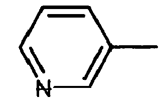 | 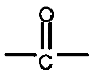 | 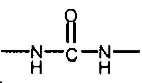 | 1 | H | H | NH₂ |
| 127 | 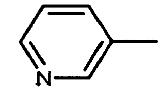 | 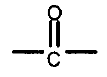 | 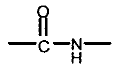 | 1 | H | H | NH₂ |
| 128 | 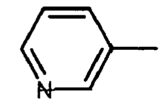 | -O-CH₂- | 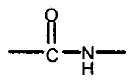 | 1 | 2-Cl | H | NH₂ |
| 129 | 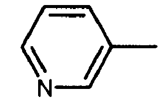 | -O-CH₂- | 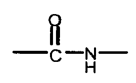 | 1 | H | 5-F | NH₂ |
| 130 | 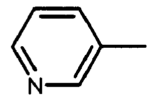 | -O-CH₂- | 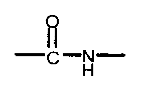 | 1 | H | 5-OCH₃ | NH₂ |
| 131 | 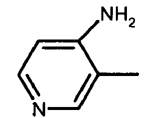 | -CH₂- | 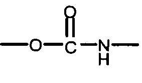 | 1 | H | H | NH₂ |
| 132 | 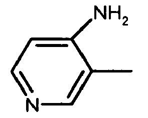 | -O-CH₂- | 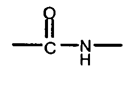 | 1 | H | H | NH₂ |
| 133 | 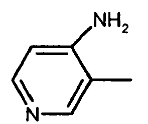 | -CH₂-O-CH₂- | 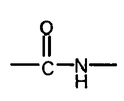 | 1 | H | H | NH₂ |
| 134 | 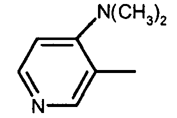 | -CH₂- | 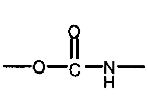 | 1 | H | H | NH₂ |
| 135 | 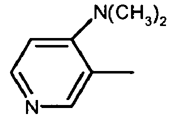 | -O-CH₂- | 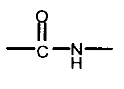 | 1 | H | H | NH₂ |
| 136 | 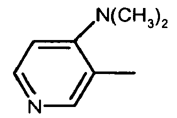 | -CH₂-O-CH₂- | 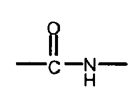 | 1 | H | H | NH₂ |
| 137 | 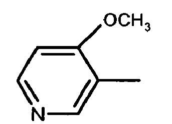 | -CH₂- | 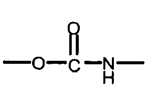 | 1 | H | H | NH₂ |
| 138 | 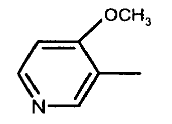 | -O-CH₂- | 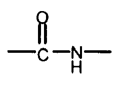 | 1 | H | H | NH₂ |
| 139 | 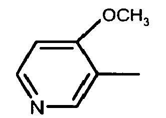 | -CH₂-O-CH₂- | 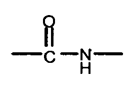 | 1 | H | H | NH₂ |
| 140 | 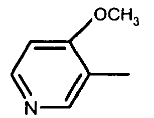 | -CH₂- | 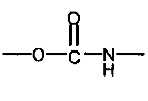 | 1 | H | 5-F | NH₂ |
| 141 | 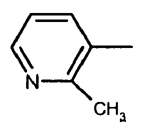 | Direct bond | 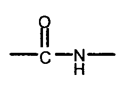 | 1 | H | H | NH₂ |
| 142 | 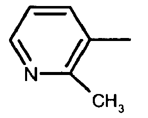 | -CH₂- | 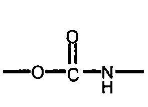 | 1 | H | H | NH₂ |
| 143 | 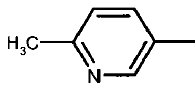 | Direct bond | 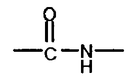 | 1 | H | H | NH₂ |
| 144 | 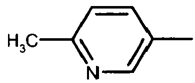 | -CH₂- | 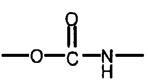 | 1 | H | H | NH₂ |
| 145 | 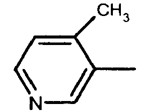 | -CH₂- | 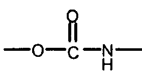 | 1 | H | H | NH₂ |
| 146 | 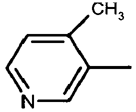 | -CH₂- | 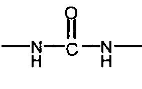 | 1 | H | H | NH₂ |
| 147 | 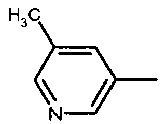 | -CH₂- | 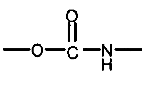 | 1 | H | H | NH₂ |
| 148 | 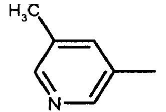 | -CH₂- | 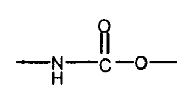 | 1 | H | H | NH₂ |
| 149 | 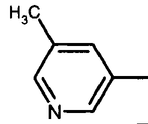 | -CH₂- | 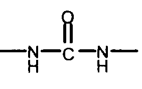 | 1 | H | H | NH₂ |
| 150 | 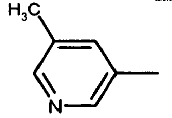 | -(CH₂)₂- | 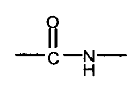 | 1 | H | H | NH₂ |
| 151 | 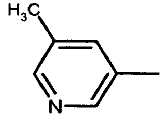 | -(CH₂)₂- | 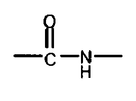 | 1 | H | H | NH₂ |
| 152 | 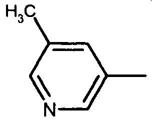 | -(CH₂)₂- | 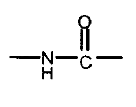 | 1 | H | H | NH₂ |
| 153 | 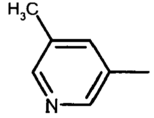 | -CH₂- | 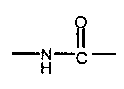 | 1 | H | H | NH₂ |
| 154 | 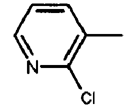 | Direct bond | 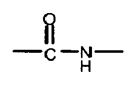 | 1 | H | H | NH₂ |
| 155 | 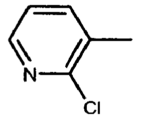 | -CH₂- | 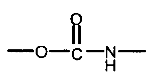 | 1 | H | H | NH₂ |
| 156 | 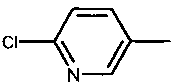 | Direct bond | 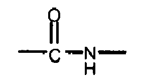 | 1 | H | H | NH₂ |
| 157 | 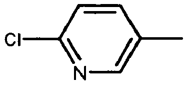 | -CH₂- | 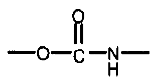 | 1 | H | H | NH₂ |
| 158 | 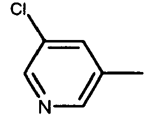 | -O-CH₂- | 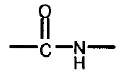 | 1 | H | H | NH₂ |
| 159 | 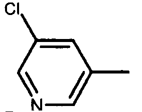 | -O-CH₂- | 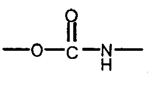 | 1 | H | H | NH₂ |
| 160 | 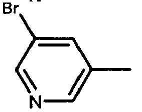 | -CH₂- | 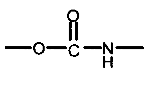 | 1 | H | H | NH₂ |
| 161 | 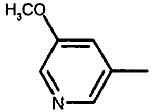 | -CH₂- | 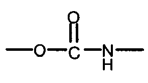 | 1 | H | H | NH₂ |
| 162 | 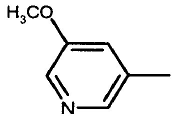 | -CH₂- | 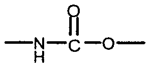 | 1 | H | H | NH₂ |
| 163 | 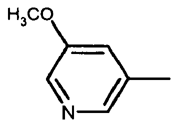 | -CH₂- | 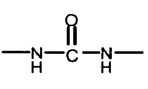 | 1 | H | H | NH₂ |
| 164 | 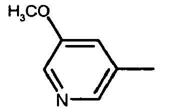 | -(CH₂)₂- | 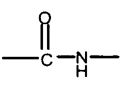 | 1 | H | H | NH₂ |
| 165 | 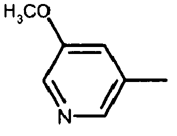 | -(CH₂)₂- | 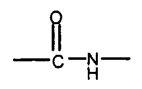 | 1 | H | H | NH₂ |
| 166 | 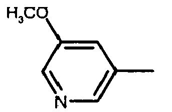 | -(CH₂)₂- | 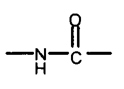 | 0 | H | H | NH₂ |
| 167 | 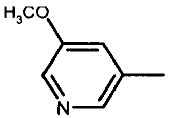 | -CH₂- | 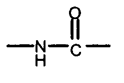 | 2 | H | H | NH₂ |
| 168 | 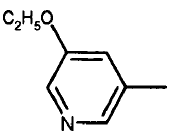 | -CH₂- | 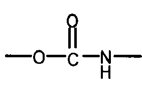 | 1 | H | H | NH₂ |
| 169 | 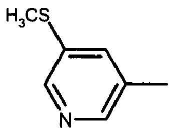 | -CH₂- | 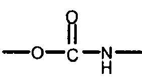 | 1 | H | H | NH₂ |
| 170 | 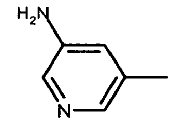 | -CH₂- | 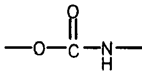 | 1 | H | H | NH₂ |
| 171 | 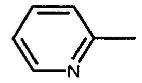 | -CH₂- | 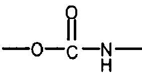 | 1 | H | H | NH₂ |
| 172 | 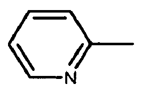 | -(CH₂)₂- | 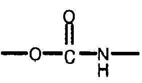 | 1 | H | H | NH₂ |
| 173 | 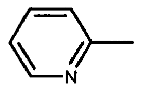 | Direct bond | 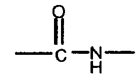 | 1 | H | H | NH₂ |
| 174 | 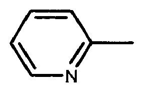 | -CH₂- | 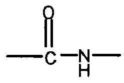 | 0 | H | H | NH₂ |
| 175 | 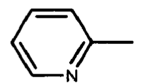 | -O-CH₂- | 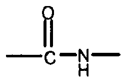 | 1 | H | 5-OCH₃ | NH₂ |
| 176 | 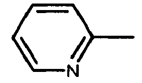 | -CH₂-O-CH₂- | 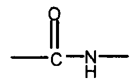 | 0 | H | H | NH₂ |
| 177 | 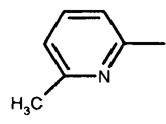 | -CH₂- | 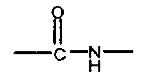 | 0 | H | H | NH₂ |
| 178 | 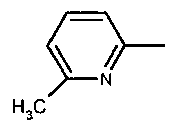 | Direct bond | 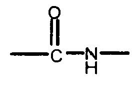 | 1 | H | H | NH₂ |
| 179 | 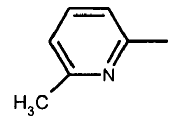 | -CH₂- | 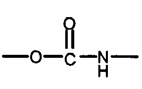 | 1 | H | H | NH₂ |
| 180 | 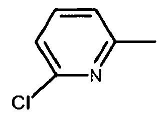 | -CH₂- | 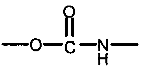 | 1 | H | H | NH₂ |
| 181 | 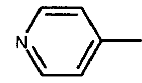 | -CH₂- | 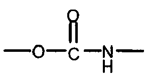 | 1 | H | H | NH₂ |
| 182 | 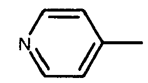 | -(CH₂)₂- | 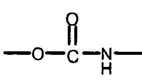 | 1 | H | H | NH₂ |
| 183 | 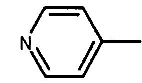 | Direct bond | 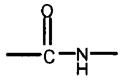 | 1 | H | H | NH₂ |
| 184 | 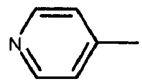 | -CH₂- | 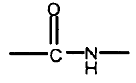 | 0 | H | H | NH₂ |
| 185 | 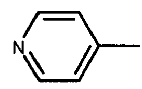 | -CH₂- | 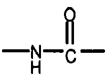 | 0 | H | H | NH₂ |
| 186 | 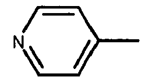 | -CH₂- | 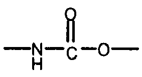 | 1 | H | H | NH₂ |
| 187 | 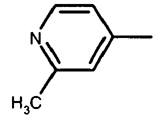 | -CH₂- | 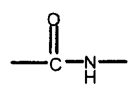 | 0 | H | H | NH₂ |
| 188 | 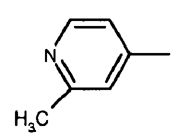 | Direct bond | 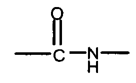 | 1 | H | H | NH₂ |
| 189 | 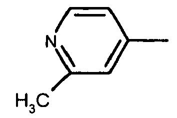 | -CH₂- | 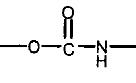 | 1 | H | H | NH₂ |
| 190 | 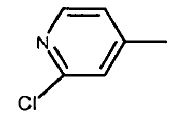 | -CH₂- | 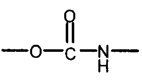 | 1 | H | H | NH₂ |
| 191 | 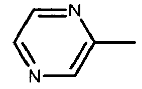 | Direct bond | 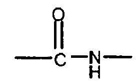 | 1 | H | H | NH₂ |
| 192 | 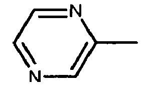 | -CH₂- | 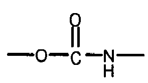 | 1 | H | H | NH₂ |
| 193 | 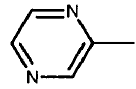 | -CH₂-O-CH₂- | 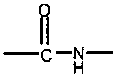 | 1 | H | H | NH₂ |
| 194 | 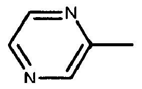 | -CH₂-O-CH₂- | 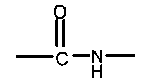 | 0 | H | H | NH₂ |
| 195 | 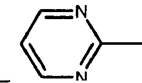 | Direct bond | 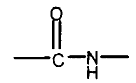 | 1 | H | H | NH₂ |
| 196 | 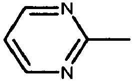 | -CH₂- | 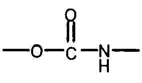 | 1 | H | H | NH₂ |
| 197 | 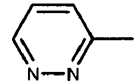 | Direct bond | 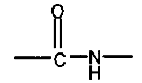 | 1 | H | H | NH₂ |
| 198 | 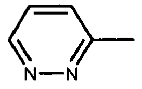 | -CH₂- | 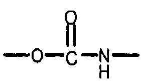 | 1 | H | H | NH₂ |
| 199 | 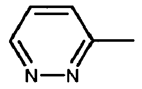 | -CH₂-O-CH₂- | 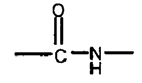 | 1 | H | H | NH₂ |
| 200 | 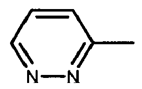 | -CH₂-O-CH₂- | 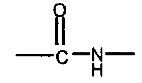 | 0 | H | H | NH₂ |
| 201 | 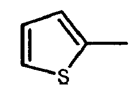 | Direct bond | 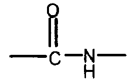 | 1 | H | H | NH₂ |
| 202 | 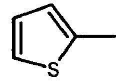 | -CH₂- | 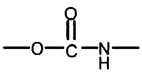 | 1 | H | H | NH₂ |
| 203 | 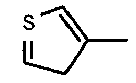 | -(CH₂)₂- | 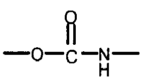 | 1 | H | H | NH₂ |
| 204 | 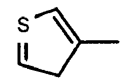 | -CH₂-O-CH₂- | 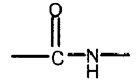 | 0 | H | H | NH₂ |
| 205 | 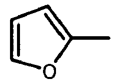 | Direct bond | 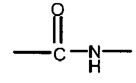 | 1 | H | H | NH₂ |
| 206 | 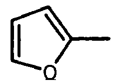 | -CH₂- | 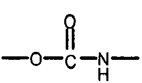 | 1 | H | H | NH₂ |
| 207 | 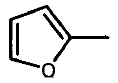 | -CH₂-O-CH₂- | 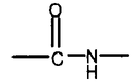 | 1 | H | H | NH₂ |
| 208 | 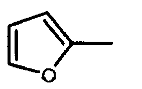 | -CH₂-O-CH₂- | 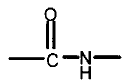 | 0 | H | H | NH₂ |
| 209 | 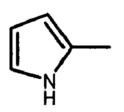 | Direct bond | 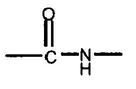 | 1 | H | H | NH₂ |
| 210 | 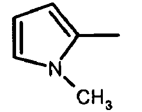 | Direct bond | 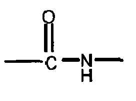 | 1 | H | H | NH₂ |
| 211 | 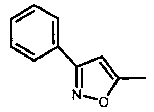 | -CH₂- | 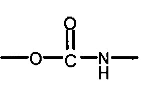 | 1 | H | H | NH₂ |
| 212 | 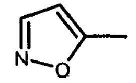 | Direct bond | 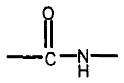 | 1 | H | H | NH₂ |
| 213 | 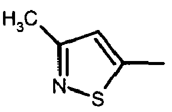 | Direct bond | 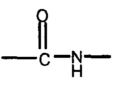 | 1 | H | H | NH₂ |
| 214 | 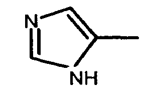 | Direct bond | 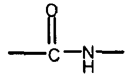 | 1 | H | H | NH₂ |
| 215 | 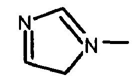 | -(CH₂)₃- | 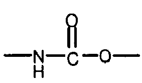 | 1 | H | H | NH₂ |
| 216 | 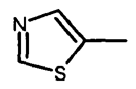 | -CH₂- | 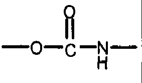 | 1 | H | H | NH₂ |
| 217 | 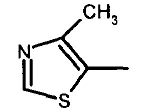 | -(CH₂)₂- | 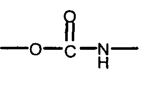 | 1 | H | H | NH₂ |
| 218 | 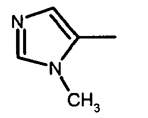 | -CH₂- | 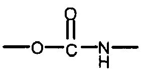 | 1 | H | H | NH₂ |
| 219 | 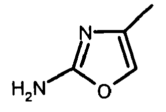 | -CH₂- | 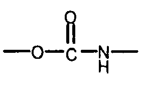 | 1 | H | H | NH₂ |
| 220 | 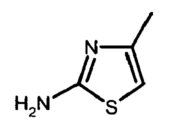 | -CH₂- | 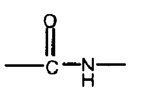 | 1 | H | H | NH₂ |
| 221 | 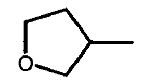 | -CH₂- | 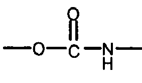 | 1 | H | H | NH₂ |
| 222 | 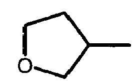 | -CH₂-O-CH₂- | 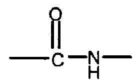 | 1 | H | H | NH₂ |
| 223 | 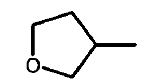 | -CH₂-O-CH₂- | 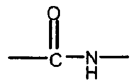 | 1 | H | H | NH₂ |
| 224 | 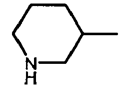 | Direct bond | 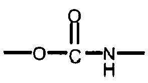 | 1 | H | H | NH₂ |
| 225 | 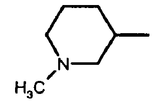 | -CH₂- | 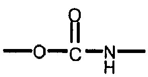 | 1 | H | H | NH₂ |
| 226 | 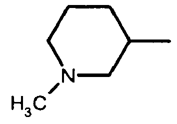 | -CH₂-O-CH₂- | 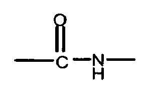 | 1 | H | H | NH₂ |
| 227 | 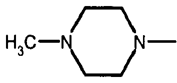 | -(CH₂)₃- | 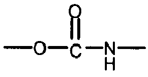 | 1 | H | H | NH₂ |
| 228 | 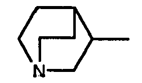 | Direct bond | 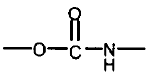 | 1 | H | H | NH₂ |
| 229 | 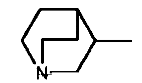 | -CH₂- | 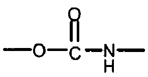 | 1 | H | H | NH₂ |
| 230 | 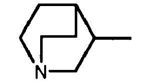 | -CH₂-O-CH₂- | 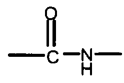 | 1 | H | H | NH₂ |
| 231 | 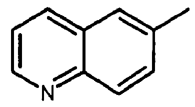 | Direct bond | 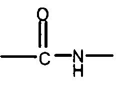 | 1 | H | H | NH₂ |
| 232 | 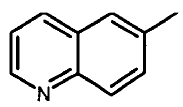 | Direct bond | 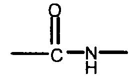 | 1 | H | H | NH₂ |
| 233 | 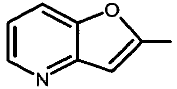 | Direct bond | 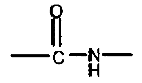 | 1 | H | H | NH₂ |
| 234 | 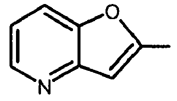 | Direct bond | 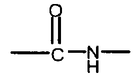 | 1 | H | H | NH₂ |
| 235 | 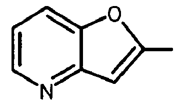 | Direct bond | 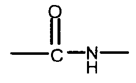 | 1 | H | H | NH₂ |
| 236 | 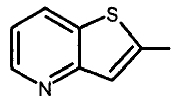 | Direct bond | 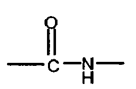 | 1 | H | H | NH₂ |
| 237 | 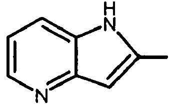 | Direct bond | 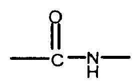 | 1 | H | H | NH₂ |
| 238 | 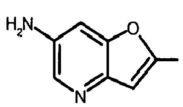 | Direct bond | 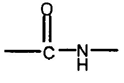 | 1 | H | H | NH₂ |
| 239 | 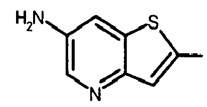 | Direct bond | 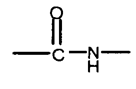 | 1 | H | H | NH₂ |
| 240 | 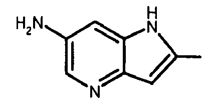 | Direct bond | 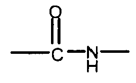 | 1 | H | H | NH₂ |

**Table 2**

| Compound No. | Structural formula |
|---|---|
| 1 | 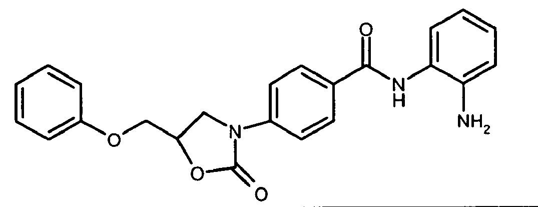 |
| 2 | 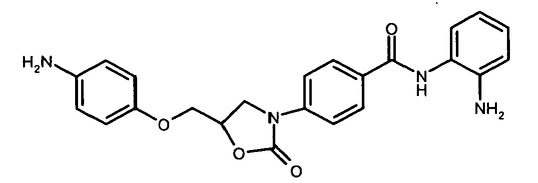 |
| 3 | 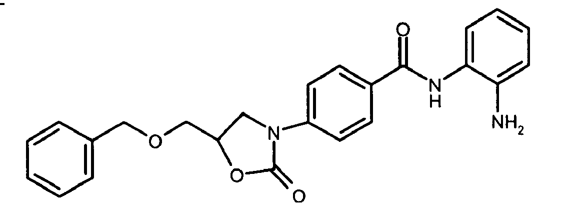 |
| 4 | 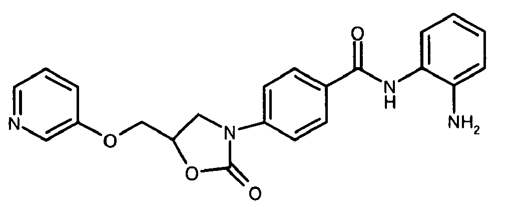 |
| 5 | 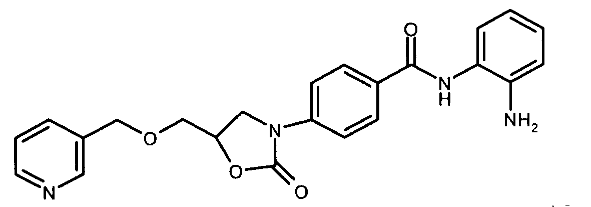 |
| 6 | 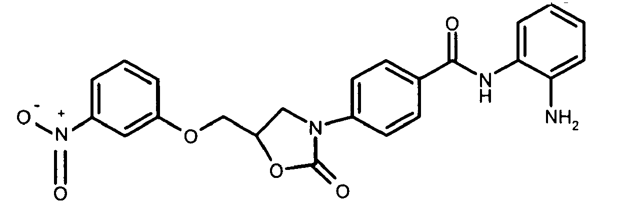 |
| 7 | 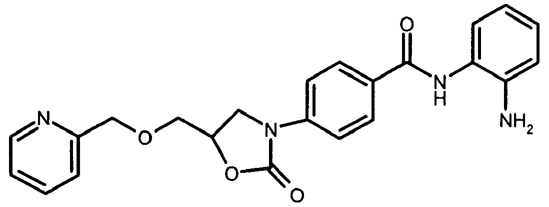 |
| 8 | 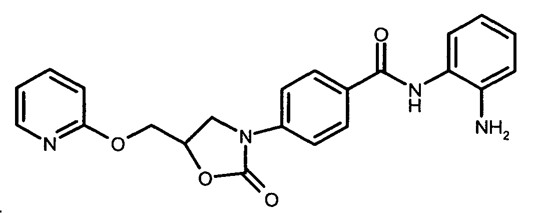 |
| 9 | 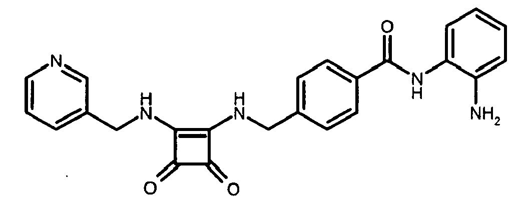 |
| 10 | 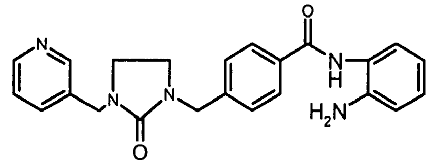 |
| 11 | 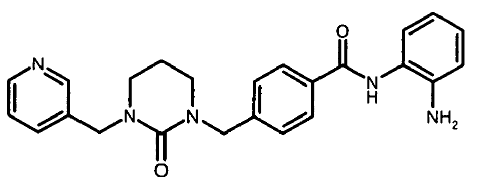 |
| 12 | 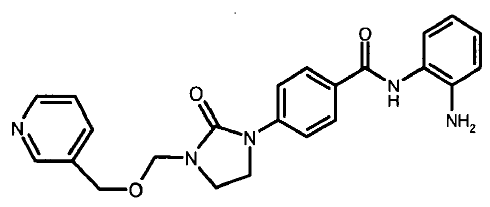 |
| 13 | 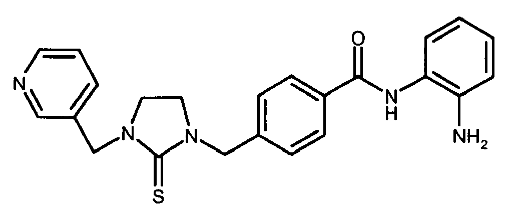 |
| 14 | 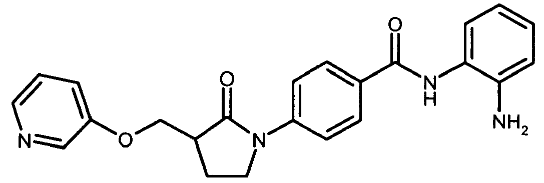 |
| 15 | 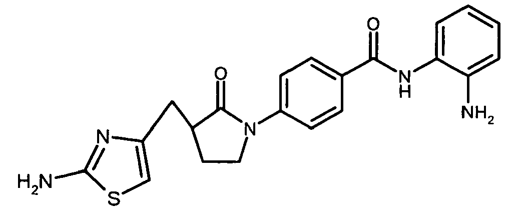 |
| 16 | 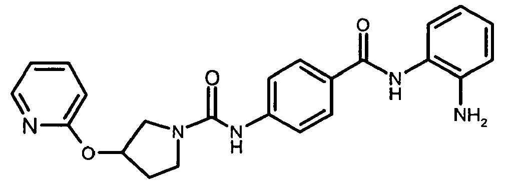 |
| 17 | 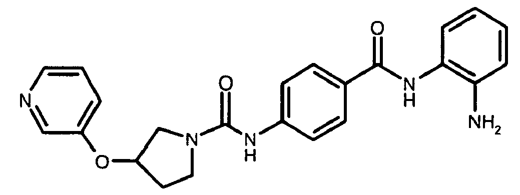 |
| 18 | 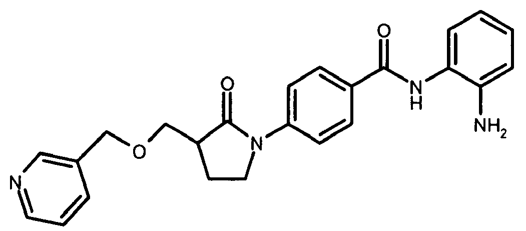 |
| 19 | 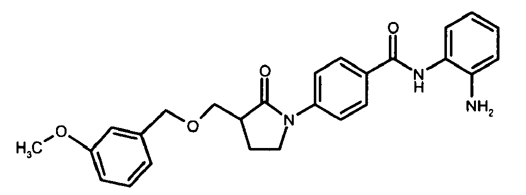 |
| 20 | 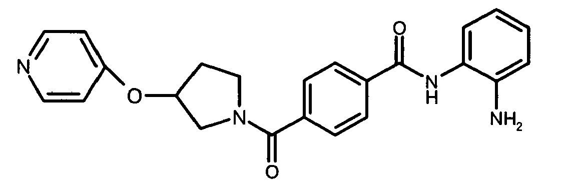 |

**Table 3**

| Compound No. | Structural formula |
|---|---|
| 1 | 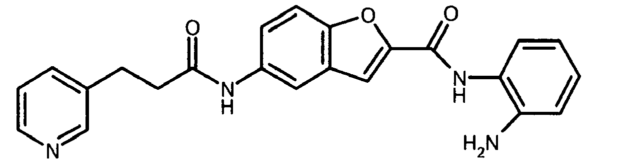 |
| 2 | 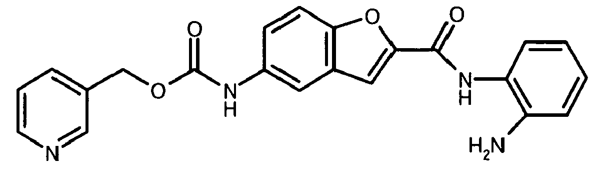 |
| 3 | 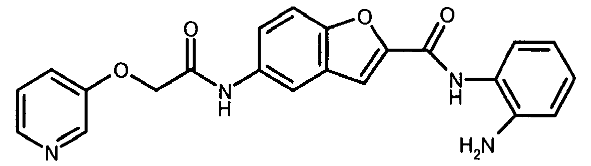 |
| 4 | 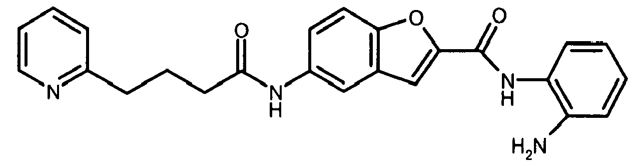 |
| 5 | 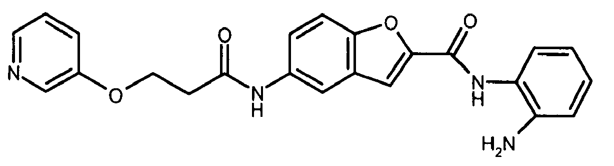 |
| 6 | 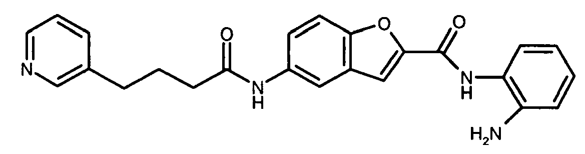 |
| 7 | 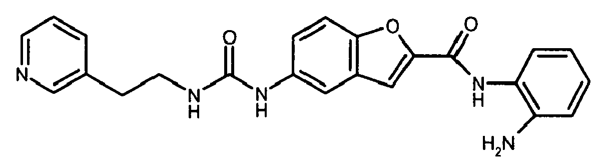 |
| 8 | 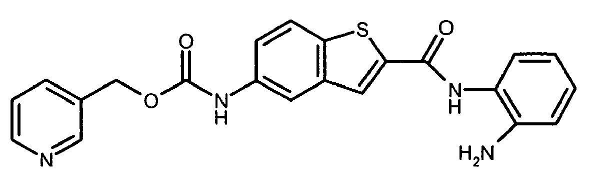 |
| 9 | 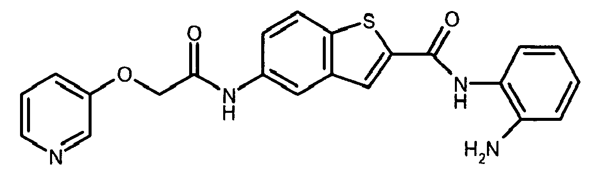 |
| 10 | 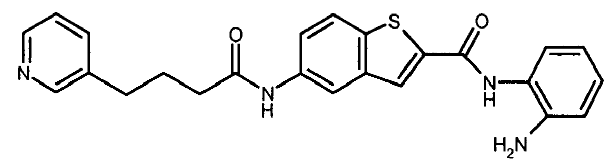 |
| 11 | 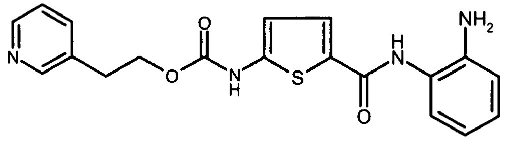 |
| 12 | 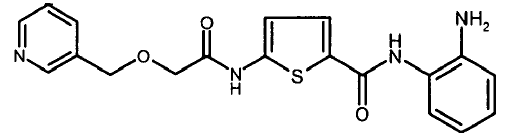 |
| 13 | 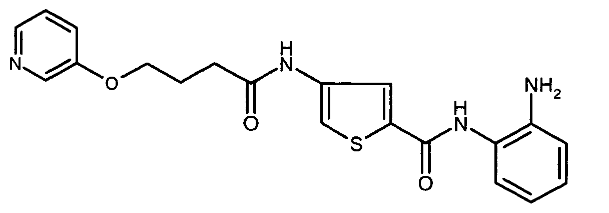 |
| 14 | 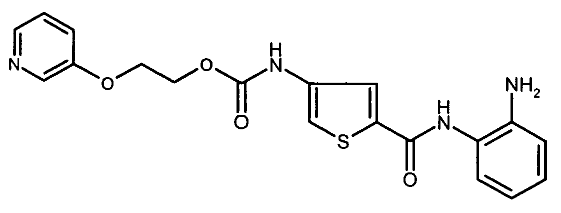 |
| 15 | 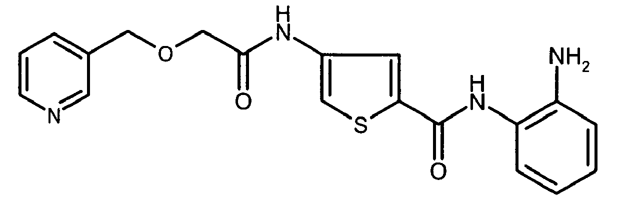 |
| 16 | 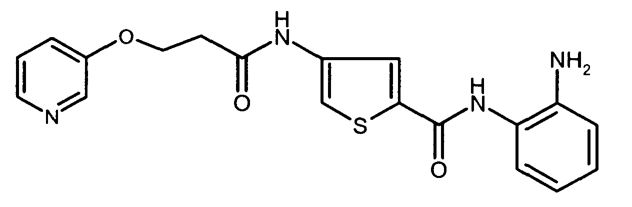 |

**Table 4**

| Compound No. | Structural formula |
|---|---|
| 1 | 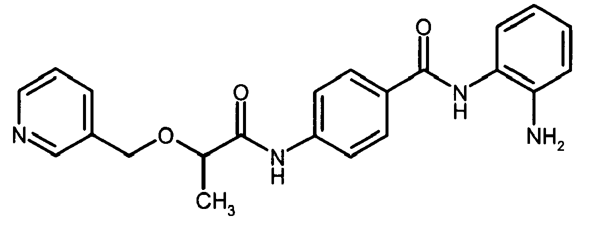 |
| 2 | 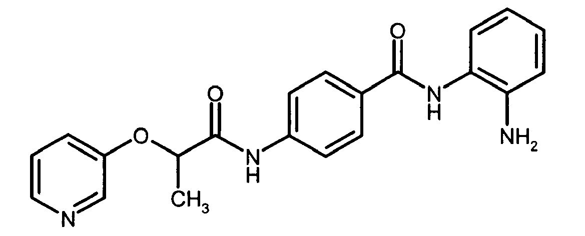 |
| 3 | 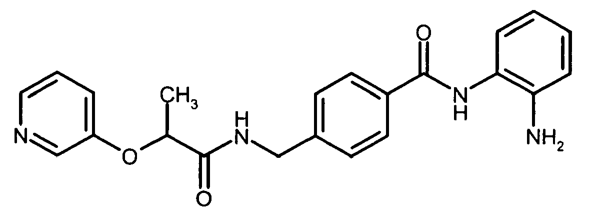 |
| 4 | 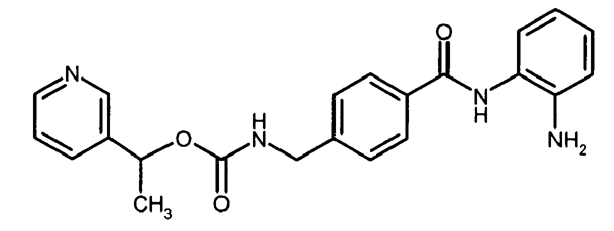 |
| 5 | 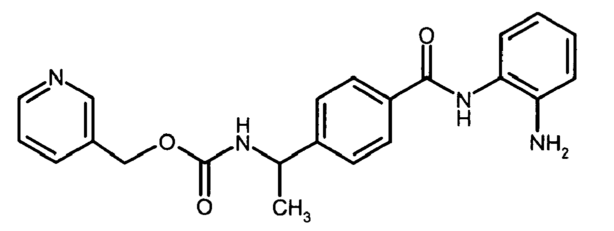 |
| 6 | 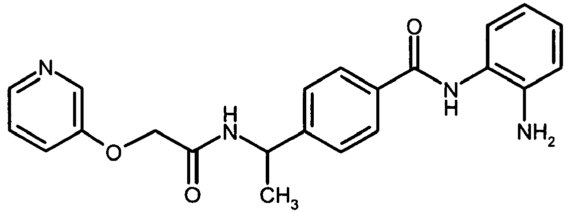 |
| 7 | 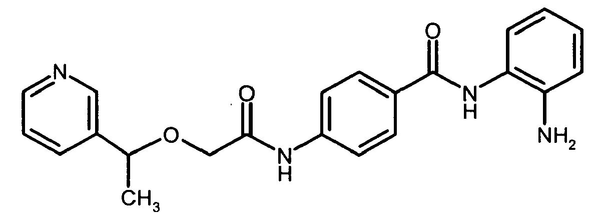 |
| 8 | 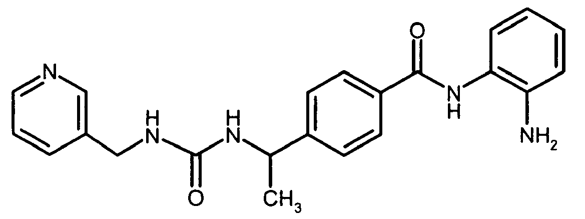 |
| 9 | 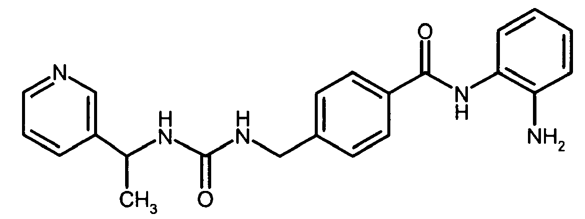 |
| 10 | 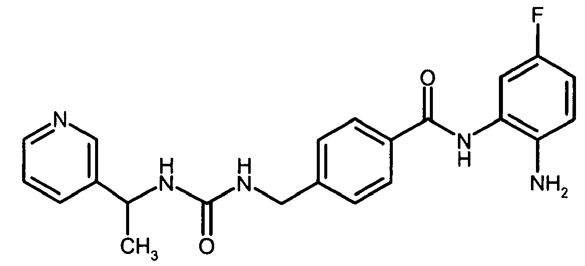 |
| 11 | 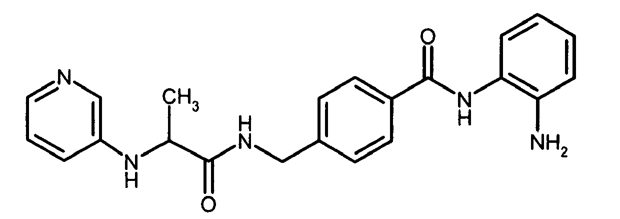 |
| 12 | 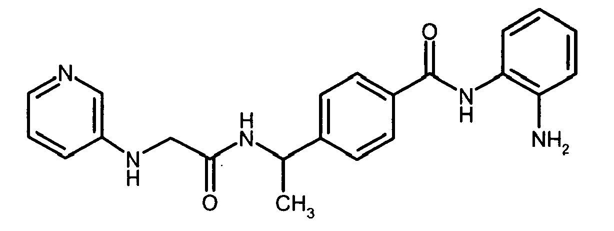 |
| 13 | 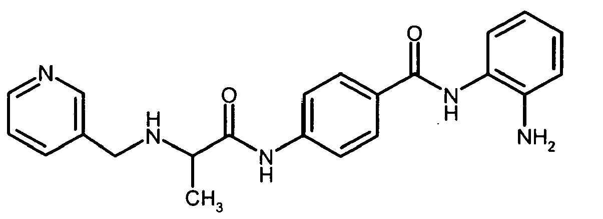 |
| 14 | 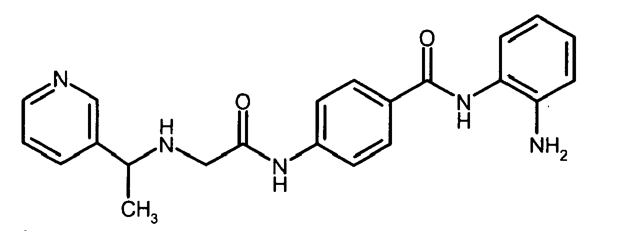 |

The compounds of this invention may be prepared as described in US 6,174,905.

### Examples for compound b) of the instant inventive combination are described as follows:

It should however be noted that these examples do not limit the inventive combination only to these compounds b).

### Example b-1

### Preparation of N-(2-aminophenyl)-4-(N-benzoylaminomethyl)benzamide hydrochloride (Table 1: hydrochloride of Compound 1):

### Process 1-1

To a suspension of 21.16 g of 4-aminomethylbenzoic acid(140 mmol) in 450 ml of dichloromethane was added 42 ml of triethylamine (300 mmol). Under ice-cooling, 60.4 g of trifluoroacetic anhydride (287 mmol) in 50 ml of dichloromethane were added dropwise, maintaining the inner temperature at 3 to 8 °C., and then the mixture was stirred four 3 hours. The reaction mixture was poured into a saturated aqueous sodium bicarbonate solution, and was acidified with 10% hydrochloric acid. The gel precipitatem was collected by filtration and dried to give 30.4 g of 4-(N-trifluoroacetylaminomethyl)benzoic acid (Yield: 87.8%) as an opalescent solid.
¹H NMR (270 MHz, DMSO-d₆) . δ. ppm: 4.47(2H, d, J=5.8 Hz), 7.39(2H, d, J=8.1 Hz), 7.93(2H, d, J=8.1 Hz), 10.08(1H, t, J=5.8 Hz), 12.95(1H, br.s.)

### Process 1-2

To a solution of 108 g of o-phenylenediamine (1.0 mol) in 1000 ml of dioxane was added 500 ml of 1N sodium hydroxide aq., and then 218 g of tert-butyldicarbonate (1.1 mol) in 500 ml of dioxane under ice-cooling. After stirring for 6 hours at room temperature, the mixture was left overnight. The mixture was concentrated to 1/2 volume by evaporation, and extracted with ethyl acetate. The organic layer was washed with saturated brine, dried and evaporated. The residue was purified by column chromatography (eluent: chloroform) to give a solid, which was then washed with diethyl ether to give 68.4 g of N-tertbutoxycarbonyl-o-phenylenediamine (Yield: 32.8%) as a white solid.
¹H NMR(270 MHz, CDCl₃) .δ. ppm: 1.51 (9H, s), 3.75(2H, s), 6.26(1H, s), 6.77(1H, d, J=8.1 Hz), 6.79(1H, dd, J=7.3, 8.1 Hz), 7.00(1H, dd, J=7.3, 8.1 Hz), 7.27(1H, d, J=8.1 Hz)

### Process 1-3

To a suspension of 30 g of the compound from the process (1-1) (121 mmol) in 200 ml of dichloromethane were slowly added dropwise 21 g of oxalyl chloride (165 mmol) with intermittently adding DMF (0.1 ml per 2 ml addition), maintaining the inner temperature within 10 to 15 °C by ice-cooling. After completion of the addition, the mixture was stirred until bubble generation ceased, and then at 40 °C. for an additional hour. After evaporation, excess oxalyl chloride was azeotropically removed with toluene, and then the residue was redissolved in 100 ml of dichloroethane. The prepared acid chloride solution was added dropwise to a solution of 22.88 g of the compound from the process (1-2) (110 mmol) in 100 ml of dichloromethane and 200 ml of pyridine, maintaining the inner temperature within 7 to 9 °C. by ice-cooling.

After addition, the mixture was warmed to room temperature, and was left overnight. After adding saturated sodium bicarbonate aq. to the reaction mixture, the resulting mixture was extracted with chloroform, and the organic layer was washed with saturated brine, dried and evaporated. To the residue was added methanol-diisopropyl ether, and the precipitated solid was collected by filtration and dried to give 28.1 g of N-([2-(N-tert-butoxycarbonyl)aminophenyl]-4-(N-trifluoroacetylaminomethyl) benzamide (Yield: 58%) as a light yellow solid.
¹ H NMR (270 MHz, DMSO-d₆) .delta. ppm: 1.44(9H, s), 4.48(2H, d, J=5.9 Hz), 7.12-7.23(2H, m), 7.44(2H, d, J=8.1 Hz), 7.54(2H, d, J=8.1 Hz) 7.94(2H, d, J=8.1 Hz), 8.68(1H, br.s), 9.83(1H, s), 10.10(1H, br.t, J=5.9 Hz)

### Process 1-4

To a suspension of 13.12 g of the compound from the process (1-3) (30 mmol) in 120 ml of methanol and 180 ml of water were added 4.70 g of potassium carbonate (34.0 mmol), and the mixture was heated with stirring at 70 °C. for 4 hours. It was extracted with chloroform, and the organic layer was washed with saturated brine, dried, evaporated and dried to give 10.3 g of 4-aminomethyl-N-[2-(N-tertbutoxycarbonyl)aminophenyl]benzamide (Yield: quantitative) as a light yellow amorphous solid.
¹ H NMR (270 MHz, DMSO-d₆) .delta. ppm: 3.80(2H, s), 7.13-7.23(2H, m), 7.48-7.58(4H, m), 7.90(2H, d, J=8.1 Hz), 8.69(1H, br.s), 9.77(1H, br.s)

### Process (1-5)

To a solution of 0.11 g of the compound from the process (1-4) (0.44 mmol) in 5 ml of pyridine was added 0.08 g of benzoyl chloride (0.53 mmol), and the mixture was gradually warmed to room temperature and then stirred for 8 hours. Saturated sodium bicarbonate aq. was added, and then the mixture was extracted with ethyl acetate. The organic layer was washed with saturated brine, dried and evaporated. The residue was washed with diisopropyl ether, and the solid obtained was dried to give 0.14 g of N-[2-(N-tertbutoxycarbonyl)aminophenyl]-4-(N-benzoylaminomethyl)benzamide (Yield: 71.4%) as a white solid.
¹ H NMR (270 MHz, DMSO-d₆) .delta. ppm: 1.44(9H, s), 4.56(2H, d, J=5.9 Hz), 7.11-7.22(2H, m), 7.46-7.56(7H, m), 7.90-7.94(4H, m), 8.67(1H, s), 9.15(1H, t, J=5.9 Hz), 9.81(1H, s)

### Process (1-6)

To a solution of 0.10 g of the compound from the process (1-5) (0.224 mmol) in 5 ml of dioxane and 1 ml of methanol was added 5 ml of 4N hydrochloric acid-dioxane, and the mixture was stirred at room temperature for 7 hours. To the residue after evaporation was added diisopropyl ether, and the formed solid was collected by filtration and dried to give 0.08 g of N-(2-aminophenyl)-4-(N-benzoylaminomethyl)benzamide hydrochloride (Yield: 93%) as a light brown solid.
mp: 206-209 °C.
¹ H NMR (270 MHz, DMSO-d₆) .delta. ppm: 4.57(2H, d, J=5.8 Hz), 7.27-7.38(4H, m), 7.47-7.59(5H, m), 7.92(1H, d, J=8.1 Hz), 8.05(1H, d, J=8.1 Hz), 9.19(1H, t, J=5.8 Hz), 10.38(1H, br.s)
IR(KBr, cm⁻¹): 3286, 3003(br.), 1630, 1551, 1492, 1306, 1250, 749, 695.

As described in Example 1, the compounds of Examples 2 to 44 were prepared, each of whose melting point (mp), ¹H NMR data and/or IR data are described below.

### Example 2

### N-(2-aminophenyl)-4-[N-(2-chlorobenzoyl)aminomethyl]benzamide (Table 1: Compound 14)

mp: 201-204 °C. (dec.)
¹ H NMR (270 MHz, DMSO-d₆) .delta. ppm: 4.52(2H, t, J=5.9 Hz), 4.89(2H, br.s), 6.60(1H, ddd, J=1.5, 7.3, 8.1 Hz), 6.78(1H, dd, J=1.5, 8.1 Hz), 6.97(1H, ddd, J=1.5, 7.3, 8.1 Hz), 7.17(1H, d, J=8.1 Hz), 7.38-7.54(6H, m), 7.97(2H, d, J=8.1 Hz), 9.06(1H, br.t, J=5.9 Hz), 9.63(1H, br.s)
IR (KBr) cm⁻¹ : 3268, 1649, 1458, 1304, 748

### Example 3

### N-(2-aminophenyl)-4-[N-(2-nitrobenzoyl)aminomethyl]benzamide hydrochloride(Table 1: hydrochloride of Compound 18)

mp: 210-212 °C.(dec.)
¹ H NMR(270 MHz, DMSO-d₆) ppm: 4.55(2H, t, J=5.9 Hz), 7.20-7.40(3H, m), 7.50-7.60(1H, m), 7.53(2H, d, J=8.1 Hz), 7.60-7.70(2H, m), 7.83(1H, ddd, J=1.5, 8.1, 8.1 Hz), 8.00-8.10(3H, m), 9.34(1H, t, J=5.9 Hz), 10.43(1H, br.s)
IR(KBr)cm⁻¹ : 3283, 2500-3000(br.), 1648, 1534, 1461, 1362, 1314, 754, 701

### Example 4

### N-(2-aminophenyl)-4-[N-(4-methylbenzoyl)aminomethyl]benzamide hydrochloride (Table 1: hydrochloride of Compound 28)

mp: (amorphous).
¹ H NMR (270 MHz, DMSO-d₆) .delta. ppm: 2.37(3H, s), 4.56(2H, d, J=5.0 Hz), 7.20-7.30(6H, m), 7.47(4H, d, J=8.8 Hz), 7.82(2H, d,J=8.8 Hz), 8.03(2H, d, J=8.8 Hz), 9.09(1H, t, J=5 Hz), 10.36(1H, br.s)
IR(KBr)cm⁻¹ : 3269(br.), 2861(br.), 1743, 1636, 1534, 1505, 1456, 1308, 1120, 753.

### Example 5

### N-(2-aminophenyl)-4-[N-(3-methoxybenzoyl)aminomethyl]benzamide (Table 1: Compound 30)

mp: 182-185 °C.
¹ H NMR(270 MHz, DMSO-d₆). delta. ppm: 3.81 (3H, s), 4.54(2H, d, J=5.9 Hz), 4.88(2H, br.s), 6.60(1H, dd, J=6.6, 7.3 Hz), 6.78(1H, d, J=7.3 Hz), 6.97(1H, dd, J=6.6, 7.3 Hz), 7.11(1H, dd, J=1.5, 8.1 Hz), 7.16(1H, d, J=7.3 Hz), 7.35-7.51 (5H, m), 7.94(2H, d, J=8.1 Hz), 9.12(1H, br.t, J=5.9 Hz), 9.63(1H, br.s)
IR(KBr)cm⁻¹ : 3301, 1637, 1524, 1489, 1457, 1314, 1248, 752

### Example 6

### N-(2-aminophenyl)-4-[N-(4-methoxybenzoyl)aminomethyl]benzamide (Table 1: Compound 31)

mp: 149-151 °C.
¹ H NMR(270 MHz, DMSO-d₆) .delta. ppm: 3.82(3H, s), 4.53(2H, d, J=5.9 Hz), 4.88(2H, s), 6.59(1H, dd, J=7.3, 7.3 Hz), 6.77(1H, d, J=8.1 Hz), 6.94-7.00(1H, m), 7.02(2H, d, J=8.8 Hz), 7.16(1H, d, J=8.1 Hz), 7.43(2H, d, J=8.1 Hz), 7.89(2H, d, J=8.8 Hz), 7.94(2H, d, J=8.1 Hz), 8.98(1H, br.t, J=5.9 Hz), 9.61(1H, br.s)
IR(KBr)cm⁻¹ : 3297, 1630, 1527, 1505, 1457, 1256, 1177, 1024,843, 749

### Example 7

### N-(2-aminophenyl)-4-[N-(3,4,5-trimethoxybenzoyl)aminomethyl]benzamide (Table 1: Compound 33)

mp: 208-210 °C.(dec.)
¹ H NMR(270 MHz, DMSO-d₆) .delta. ppm: 3.71(3H, s), 3.83(6H, s), 4.55(2H, d, J=5.9 Hz), 4.88(2H, br.s), 6.60(1H, dd, J=7.3, 8.1 Hz), 6.78(1H, d, J=8.1 Hz), 6.97(1H, dd, J=6.6, 8.1 Hz), 7.16(1H, d, J=8.1 Hz), 7.26(2H, s), 7.44(2H, d, J=8.1 Hz), 7.95(2H, d, J=8.8 Hz), 9.07(1H, t, J=5.9 Hz), 9.62(1H, br.s)
IR(KBr)cm⁻¹ : 3267, 1635, 1582, 1457, 1237, 1132, 755

### Example 8

### N-(2-aminophenyl)-4-[N-[4-(N,N-dimethyl)aminobenzoyl]aminomethyl]benzamide (Table 1: Compound 36)

mp: 216-219°C(dec.)
¹ H NMR(270 MHz, DMSO-d6) .delta. ppm: 2.98(6H, s), 4.51(2H, d, J=5.9 Hz), 4.88(2H, br.s), 6.60(1H, dd, J=8.1, 8.1 Hz), 6.71(2H, d, J=8.8 Hz), 6.97(1H, dd, J=7.3, 8.1 Hz), 7.16(1H, d, J=7.3 Hz), 7.41(2H, d, J=8.1 Hz), 7.78(2H, d, J=8.8 Hz), 7.93(2H, d, J=8.1 Hz), 8.77(1H, t, J=5.9 Hz), 9.63(1H, br.s).
IR(KBr)cm-1 : 3301, 1632, 1519, 1457, 1298, 754

### Example 9

### N-(2-aminophenyl)-4-[N-(4-trifluoromethylbenzoyl)aminomethyl]benzamide (Table 1: Compound 42)

mp: 243-246°C
¹ H NMR(270 MHz, DMSO-d6) .delta. ppm: 4.58(2H, d, J=5.9 Hz), 4.88(2H, br.s), 6.59(1H, dd, J=6.6, 7.3 Hz), 6.77(1H, d, J=8.1 Hz), 6.94(1H, dd, J=5.9, 6.6 Hz), 7.16(1H, d, J=8.1 Hz), 7.45(2H, d, J=8.1 Hz), 7.88(2H, d, J=8.8 Hz), 7.95(2H, d, J=8.1 Hz), 8.11(2H, d, J=8.1 Hz), 9.38(1H, t, J=5.9 Hz), 9.64 (1H, br.s)
IR(KBr)cm-1 : 3301, 1640, 1549, 1523, 1458, 1334, 1162, 1120, 1070, 856, 750

### Example 10

### N-(2-aminophenyl)-4-[N-(4-carboxybenzoyl)aminomethyl]benzamide hydrochloride (Table 1: hydrochloride of Compound 45)

mp: (amorphous).
¹ H NMR(270 MHz, DMSO-d6) .delta. ppm: 4.58(2H, d, J=5.9 Hz), 7.29-7.37(3H, m), 7.49(3H, d, J=8.1 Hz), 8.02-8.06(6H, m), 9.36(1H, t, J=5.9 Hz), 10.4(1H, br.s)
IR(KBr)cm-1 : 3432(br.), 1718, 1637, 1542, 1499, 1303(br.), 1116, 1018, 757

### Example 11

### N-(2-aminophenyl)-4-[N-(4-methoxycarbonylbenzoyl)aminomethyl]benzamide (Table 1: Compound 46)

mp: 204-209°C(dec.)
¹ H NMR(270 MHz, DMSO-d6) .delta. ppm: 3.89(3H, s), 4.57(2H, d, J=5.9 Hz), 4.88(2H, br.s), 6.60(1H, dd, J=6.6, 7.3 Hz), 6.78(2H, d, J=7.3 Hz), 6.97(1H, ddd, J=1.5, 6.6, 7.3 Hz), 7.16(1H, d, J=7.3 Hz), 7.45 (2H, d, J=8.1 Hz), 7.95(2H, d, J=8.1 Hz), 8.03(2H, d, J=8.8 Hz), 8.07(2H, d, J=8.8 Hz), 9.35(1H, t, J=5.9 Hz), 9.64(1H, br.s)
IR(KBr)cm-1 : 3287(br.), 1721, 1634, 1281, 1113, 750, 703

### Example 12

### N-(2-aminophenyl)-4-(N-picolinoylaminomethyl)benzamide (Table 1: Compound 173)

mp: 173-178°C(dec.
¹ H NMR(270 MHz, DMSO-d6) .delta. ppm: 4.57(2H, d; J=6.6 Hz), 4.88(2H, br.s), 6.59(1H, dd, J=7.3, 8.1 Hz), 6.77(1H, d, J=8.1 Hz), 6.96(1H, dd, J=7.3, 8.1 Hz), 7.16(1H, d, J=7.3 Hz), 7.44(2H, d, J=8.1 Hz), 7.60-7.65(1H, m), 7.93(2H, d, J=8.1 Hz), 7.98-8.08(2H, m), 8.67(1H, d, J=4.4 Hz), 9.45(1H, t, J=6.6 Hz), 9.61(1H, br s)
IR(KBr)cm-1 : 3330, 1656, 1634, 1523, 1456, 1294, 752

### Example 13

### N-(2-aminophenyl)-4-[N-(6-methylpicolinoyl)aminomethyl]benzamide (Table 1: Compound 178)

mp: 172-173°C
¹ H NMR(270 MHz, DMSO-d6) .delta. ppm: 2.51(3H, s), 4.57(2H, d, J=6.6 Hz), 5.0(2H, br.s), 6.61(1H, dd, J=7.3, 8.1 Hz), 6.79(1H, d, J=7.3 Hz), 6.98(1H, dd, J=7.3, 8.1 Hz), 7.17(1H, d, J=7.3 Hz), 7.44(2H, d, J=8.1 Hz), 7.43-7.49(1H, m), 7.84-7.90(2H, m), 7.94(2H, d, J=8.1 Hz), 9.27(1H, t, J=5.9 Hz), 9.64(1H, br.s)
IR(KBr)cm-1 : 3331, 1675, 1634, 1594, 1523, 1454, 1307, 1292, 750

### Example 14

### N-(2-aminophenyl)-4-(N-nicotinoylaminomethyl)benzamide (Table 1: Compound 71)

mp: 193-196°C
¹ H NMR(270 MHz, DMSO-d6) .delta. ppm: 4.58(2H, d), 4.88(2H, br.s), 6.60(1H, t), 6.78(1H, d), 6.97(1H, t), 7.16(1H, d), 7.46(2H, d), 7.53(1H, dd), 7.95(2H, d), 8.24(1H, ddd), 8.73(1H, dd), 9.07(1H, d), 9.32(1H, br.t), 9.63(1H, br.s)
IR(KBr)cm-1 : 3301, 1639, 1522, 1457, 1314, 749, 705

### Example 15

### N-(2-aminophenyl)-4-[N-(2-methylnicotinoyl)aminomethyl]benzamide (Table 1: Compound 141)

mp: 191-194°C(dec.
¹ H NMR(270 MHz, DMSO-d6) .delta. ppm: 2.53(3H, s), 4.53(2H, d, J=5.9 Hz), 4.88(2H, br.s), 6.60(1H, dd, J=6.6, 8.1 Hz), 6.78(1H, d, J=7.3 Hz), 6.97(1H, dd, J=7.3, 8.1 Hz), 7.17(1H, d, J=7.3 Hz), 7.29(1H, dd, J=5.1, 8.1 Hz), 7.47(2H, d, J=8.1 Hz), 7.77(1H, dd, J=1.5, 8.1 Hz), 7.97(2H, d, J=8.1 Hz), 8.51(1H, dd, J=1.5, 5.1 Hz), 9.06(1H, t, J=5.9 Hz), 9.64(1H, s)
IR(KBr)cm-1 : 3261, 1642, 1523, 1310, 753

### Example 16

### N-(2-aminophenyl)-4-[N-(6-methylnicotinoyl)aminomethyl]benzamide (Table 1: Compound 143)

mp: 186-190°C(dec.)
¹ H NMR(270 MHz, DMSO-d6).delta. ppm: 2.36(3H, s), 4.56(2H, d, J=5.9 Hz), 4.88(2H, s), 6.60(1H, dd, J=7.4, 7.8 Hz), 6.78(1H, d, J=7.8 Hz), 6.97(1H, dd, J=6.9, 6.9 Hz), 7.16(1H, d, J=7.4 Hz), 7.37(1H, d, J=8.3 Hz), 7.45(2H, d, J=8.3 Hz), 7.95(2H, d, J=8.3 Hz), 8.13(1H, dd, J=2.0, 8.3 Hz), 8.96(1H, s), 9.24(1H, t, J=5.9 Hz), 9.63(1H, br.s)
IR(KBr)cm-1 : 3302, 1636, 1602, 1523, 1489, 1457, 1313, 751

### Example 17

### N-(2-aminophenyl)-4-[N-(2-chloronicotinoyl)aminomethyl]benzamide (Table 1: Compound 154)

mp: 176-178°C(dec.)
¹ H NMR(270 MHz, DMSO-d6).delta. ppm: 4.54(2H, t, J=5.9 Hz), 4.90(2H, br.s), 6.60(1H, ddd, J=1.5, 7.3, 7.3 Hz), 6.78(1H, d, J=8.1 Hz), 6.97(1H, ddd, J=1.5, 7.3, 7.3 Hz), 7.18(1H, d, J=8.1 Hz), 7.48-7.54(3H, m), 7.94-7.99(3H, m), 8.49(1H, dd, J=2.1, 5.1 Hz), 9.23(1H, br.t, J=5.9 Hz), 9.65(1H, br.s)
IR(KBr)cm-1 : 3264, 1649, 1524, 1400, 1309, 751

### Example 18

### N-(2-aminophenyl)-4-[N-(6-chloronicotinoyl)aminomethyl]benzamide (Table 1: Compound 156)

mp: 205-208°C(dec.)
¹ H NMR(270 MHz, DMSO-d6) .delta. ppm: 5.57(2H, d, J=5.9 Hz), 6.60(1H, dd, J=7.3, 7.3 Hz), 6.78(1H, d, J=8.1 Hz), 6.96(1H, dd, J=7.3, 8.1 Hz), 7.16(1H, d, J=8.1 Hz), 7.45(2H, d, J=8.1 Hz), 7.66(1H, d, J=8.8 Hz), 7.95(2H, d, J=8.1 Hz), 8.27-8.32(1H, m), 8.90(1H, d, J=2.1 Hz), 9.38(1H, t, J=5.9 Hz), 9.63(1H, s)
IR(KBr)cm-1 : 3318(br.), 2929, 1646, 1590, 1525, 1503, 1454, 1108, 745

### Example 19

### N-(2-aminophenyl)-4-(N-isonicotinoylaminomethyl)benzamide (Table 1: Compound 183)

mp: 234-237°C(dec.)
¹ H NMR(270 MHz, DMSO-d6) .delta. ppm: 4.57(2H, t, J=5.9 Hz), 4.88(2H, br.s), 6.59(1H, dd, J=6.6, 7.3 Hz), 6.78(1H, d, J=8.1 Hz), 6.96(1H, dd, J=7.3, 7.3 Hz), 7.16(1H, d, J=7.3 Hz), 7.45(2H, d, J=8.1 Hz), 7.81(2H, d, J=1.5, 4.4 Hz), 7.95(2H, d, J=8.1 Hz), 8.75(2H, d, J=6.6 Hz), 9.41(1H, t, J=5.9 Hz), 9.62(1H, br.s)
IR(KBr)cm-1 : 3298, 1646, 1550, 1525, 1457, 1304, 843, 760, 695

### Example 20

### N-(2-aminophenyl)-4-[N-(pyrazin-2-yl)carbonylaminomethyl]benzamide (Table 1: Compound 191)

mp: 207°C(dec.)
¹ H NMR(270 MHz, DMSO-d6).delta. ppm: 4.58(2H, d, J=5.9 Hz), 4.88(2H, br.s), 6.59(1H, dd, J=7.3, 7.3 Hz), 6.77(1H, d, J=8.1 Hz), 6.94(1H, ddd, J=1.5, 7.3, 8.1 Hz), 7.15(1H, d, J=7.3 Hz), 7.45(2H, d, J=8.1 Hz), 7.93(2H, d, J=8.1 Hz), 8.77(1H, d, J=1.5 Hz), 8.90(1H, d, J=2.1 Hz), 9.21(1H, s), 9.55-9.61(2H, m)
IR(KBr)cm -1 : 3368(br.), 1657, 1524, 1455, 1295, 1023, 751

### Example 21

### N-(2-aminophenyl)-4-[N-(thiophen-2-yl)carbonylaminomethyl]benzamide (Table 1: Compound 201)

mp: 202-205°C(dec.)
¹H NMR(270 MHz, DMSO-d6) delta. ppm: 4.52(2H, t, J=5.9 Hz), 4.88(2H, br.s), 6.60(1H, dd,J=6.6.7.3 Hz), 6.78(1H, d, J=8.1 Hz), 6.97(1H, dd, J=7.3, 8.1 Hz), 7.15-7.18(2H, m), 7.43(2H,d, J=8.1Hz), 7.78(1H, d, J=4.4), 7.82(1H, d, J=3.7 Hz), 7.95(2H, d, J=8.1 Hz), 9.12(1H, br.t, J=5.9 Hz), 9.62(1H, br.s)
IR(KBr)cm-1 : 3306, 1633, 1523, 1456, 1297, 750, 716

### Example 22

### N-(2-aminophenyl)-4-[N-(furan-2-yl)carbonylaminomethyl]benzamide (Table 1: Compound 205)

mp: 197°C(dec.)
¹H NMR(270 MHz. DMSO-d6).delta. ppm: 4.59(2H, d, J=6.6 Hz), 4.86(2H, br.s), 6.59(1H, dd, J=6.6, 6.6 Hz), 6.63(1H, dd, J=1.5, 3.6 Hz), 6.78(1H, d, J=8.1 Hz), 6.96(1H, dd, J=7.3, 6.6 Hz), 7.10-7.20(2H, m), 7.41 (2H, d, J=8.1 Hz), 7.84(1H, s), 7.94(2H, d, J=8.1 Hz), 9.00(1H, br. t, J=5.9 Hz), 9.62(1H, s)
IR(KBr)cm-1 : 3245, 1651, 1573, 1545, 1323, 1241, 745

### Example 23

### N-(2-aminophenyl)-4-[N-(pyrrol-2-yl)carbonylaminomethyl]benzamide (Table 1: Compound 209)

mp: 216-220°C(dec.)
¹ H NMR(270 MHz, DMSO-d6) .delta. ppm: 4.50(2H, d, J=5.9 Hz), 4.88(2H, br.s), 6.10(1H, dd, J=2.1, 5.9 Hz), 6.59(1H, dd, J=7.3, 7.3 Hz), 6.77(1H, dd, J=1.5, 8.1 Hz), 6.84-6.88(2H, m), 6.97(1H, ddd, J=1.5, 7.3, 8.1 Hz), 7.16(1H, d, J=7.3 Hz), 7.41(2H, d, J=8.1 Hz), 7.94(2H, d, J=8.1 Hz), 8.62(1H, br.t, J=5.9 Hz), 9.62(1H, br.s)
IR(KBr)cm-1 : 3275, 1655, 1584, 1534, 1458, 1316, 747

### Example 24

### N-(2-aminophenyl)-4-[N-(N'-methyl-1 H-pyrrol-2-yl)carbonylaminomethyl]benzam ide (Table 1: Compound 210)

mp: 177-179°C(dec.)
¹ H NMR(270 MHz, DMSO-d6).delta. ppm: 3.84(3H, s), 4.46(2H, d, J=5.9 Hz), 4.88(2H, br.s), 6.03(1H, dd, J=2.1, 4.4 Hz), 6.59(1H, dd, J=8.1, 8.1 Hz), 6.77(1H, d, J=8.1 Hz), 6.84-6.97(2H, m), 7.16(1H, d, J=7.3 Hz), 7.41(2H, d, J=8.1 Hz), 7.93(2H, d, J=8.1 Hz), 8.61 (1H, t, J=5.9 Hz), 9.62(1H, br.s)
IR(KBr)cm-1 : 3325(br.), 1630, 1551, 1520, 1507, 1324, 1265, 1154, 740

### Example 25

### N-(2-aminophenyl)-4-[N-(isoxazol-5-yl)carbonylaminomethyl]benzamide (Table 1: Compound 212)

mp: 183-185°C(dec.)
¹ H NMR(270 MHz. DMSO-d6) .delta. ppm: 4.53(2H, d, J=6.6 Hz), 4.89(2H, br.s), 6.60(1H, dd, J=7.3, 7.3 Hz), 6.78(1H, d, J=7.3 Hz), 6.97(1H, dd, J=7.3, 8.1 Hz), 7.12(1H, d, J=2.1 Hz), 7.16(1H, d, J=8.1 Hz), 7.44(2H, d, J=8.1 Hz), 7.95(2H, d, J=8.1 Hz), 8.76(1H, d, J=1.5 Hz), 9.61 (1H, t, J=5.9 Hz), 9.64(1H, br.s)
IR(KBr)cm-1 : 3278(br.), 1636, 1576, 1522, 1458, 1220, 749

### Example 26

### N-(2-aminophenyl)-4-[N-(3-methylisothiazol-5-yl)carbonylaminomethyl]benzamide (Table 1: Compound 213)

mp: 168-169°C
¹ H NMR(270 MHz, DMSO-d6) .delta. ppm: 2.47(3H, s), 4.54(2H, d, J=5.9 Hz), 4.89(2H, br.s), 6.60(1H, dd, J=7.3, 7.3 Hz), 6.78(1H, d, J=7.3 Hz), 6.97(1H, ddd, J=1.0, 7.3, 8.1 Hz), 7.17(1H, d, J=7.3 Hz), 7.44 (2H, d, J=8.1 Hz), 7.73(1H, s), 7.96(2H, d, J=8.1 Hz), 9.44(1H, t, J=5.9 Hz), 9.64(1H, br.s)
IR(KBr)cm-1 : 3310, 1637, 1503, 1294, 751

### Example 27

### N-(2-aminophenyl)-4-[N-(imidazol-4-yl)carbonylaminomethyl]benzamide (Table 1: Compound 214)

mp: (amorphous).
¹ H NMR(270 MHz, DMSO-d6) .delta. ppm: 4.49(2H, d, J=6.4 Hz), 4.87(2H, br.s), 6.59(1H, dd, J=6.9, 6.9 Hz), 6.77(1H, d, J=6.9 Hz), 6.96(1H, dd, J=7.4, 7.4 Hz), 7.16(1H, d, J=6.9 Hz), 7.41 (2H, d, J=6.9 Hz), 7.64(1H, br.s), 7.73(1H, br.s), 7.92(2H, d, J=6.9 Hz), 8.56(1H, br.t, J=6.4 Hz), 9.61 (1H, s), 12.5(1H, br.s)
IR(KBr)cm-1 : 3278(br.), 1636, 1576, 1522, 1458, 1220,749

### Example 28

### N-(2-aminophenyl)-4-[N-(3-aminophenyl)acetylaminomethyl]benzamide (Table 1: Compound 23)

mp: 171-176°C
¹ H NMR(270 MHz, DMSO-d6) .delta. ppm: 4.34(2H, d, J=5.9 Hz), 5.24(4H, br.s), 6.48-6.63(4H,m), 6.78-6.81 (1H, m), 6.94-7.00(2H, m), 7.18(1H, d, J=8.1 Hz), 7.34(2H, d, J=8.1 Hz), 7.92(2H, d, J=8.1 Hz), 8.50(1H, t, J=5.9 Hz), 9.61(1H, s)

### Example 29

### N-(2-aminophenyl)-4-[N-(pyridin-3-yl)acetylaminomethyl]benzamide (Table 1: Compound 74)

mp: 127°C
¹ H NMR(270 MHz, DMSO-d6) .delta. ppm: 3.84(2H, s), 4.40(2H, d, J=5.8 Hz), 7.15-7.29(3H, m), 7.37(1H, d, J=6.6 Hz), 7.43(2H, d, J=8.8 Hz), 7.96(1H, m), 7.98(2H, d, J=8.8 Hz), 8.40(1H, d, J=8.8 Hz), 8.79-8.87(3H, m), 10.20(1H, s)

### Example 30

### N-(2-aminophenyl)-4-[N-[3-(pyridin-3-yl)propionyl]aminomethyl]benzamide (Table 1: Compound 75)

mp: 183-186°C
¹ H NMR(270 MHz, DMSO-d6).delta. ppm: 2.51(2H, t, J=7.3 Hz), 2.88(2H, d, J=7.3 Hz). 4.31(2H, d, J=5.9 Hz), 4.89(2H, br.s), 6.60(1H, dd, J=7.3, 8.1 Hz), 6.78(1H, d, J=8.1 Hz), 6.97(1H, ddd, J=1.5, 7.3, 8.1 Hz), 7.16(1H, d, J=8.1 Hz), 7.23(2H, d, J=8.8 Hz), 7.28-7.33(1H, m), 7.63(1H, d, J=8.1 Hz), 7.89(2H, d, J=8.1 Hz), 8.41-8.45(3H, m), 9.62(1H, br.s)
IR(KBr)cm-1 : 3407, 3313, 1640, 1552, 1522, 1456, 1309, 746, 717

### Example 31

### N-(2-aminophenyl)-4-[N-[4-(pyridin-3-yl)-1,4-dioxobutyl]aminomethyl]benzamide (Table 1: Compound 100)

mp: 145-147°C(dec.
¹ H NMR(270 MHz, DMSO-d6).delta. ppm: 2.37-2.50(2H, m), 2.62-2.68(2H, m), 4.13(2H, s). 4.86(2H, s), 6.56-6.61(1H, m), 6.76-6.79(1H, m), 6.94-6.99(1H, m), 7.10-7.39(4H, m), 7.43-7.46(1H, m), 7.78(2H, d, J=8.1 Hz), 8.60-8.64(1H, m). 9.58(1H, s)
IR(KBr)cm-1 : 3348, 1691, 1655, 1534, 1508, 1458, 1395, 1315, 1083, 746

### Example 32

### N-(2-aminophenyl)-4-[N-(5-chloropyridin-3-yl)oxyacetylaminomethyl]benzamide (Table 1: Compound 158)

mp: 199-201 °C
¹ H NMR(270 MHz, DMSO-d6).delta. ppm: 4.43(2H, d, J=6.6 Hz), 4.75(2H, s), 4.87(2H, br. s), 6.60(1H, dd, J=7.3, 8.1 Hz), 6.78(1H, d, J=8.1 Hz), 6.97(1H, dd, J=7.3, 8.1 Hz), 7.16(1H, d, J=8.1 Hz), 7.37(2H, d, J=8.1 Hz), 7.59(1H, d, J=2.2 Hz), 7.93(2H, d, J=8.1 Hz), 8.25(1H, d, J=1.5 Hz), 8.81(1H, t, J=6.6 Hz), 9.64(1H, s)
IR(KBr)cm-1 : 3288, 3058, 1675, 1633, 1523, 1457, 1314, 912, 755

### Example 33

### N-(2-amino-5-methoxyphenyl)-4-[N-(pyridin-3-yl)oxyacetylaminomethyl]benzami de (Table 1: Compound 175)

mp: 141-144°C
¹ H NMR(270 MHz, DMSO-d6) .delta. ppm: 3.66(3H, s), 4.43(2H, d, J=5.9 Hz), 4.49(2H, br.s), 4.68(2H, s), 6.62(1H, dd, J=2.9, 8.8 Hz), 6.75(1H, d, J=8.8 Hz), 6.91(1H, d, J=2.2 Hz), 7.37(4H, m), 7.92(2H, d, J=8.8 Hz), 8.21(1H, dd, J=1.5, 4.4 Hz), 8.35(1H, d, J=2.7 Hz), 8.81(1H, s), 9.65(1H, s)

### Example 34

### N-(2-aminophenyl)-4-[N-[3-(pyridin-3-yl)-1,3-dioxopropyl]aminomethyl]benzamide (Table 1: Compound 98)

mp: 204-206°C
¹H NMR(270 MHz, DMSO-d6) .delta. ppm: 4.08(4/3H, s), 4.39(4/3H, d, J=5.9 Hz), 4.49(2/3H, d, J=5.9 Hz), 4.90(2H, br.s), 5.93(1/3H, s), 6.60(1H, t, J=7.3 Hz), 6.78(1H, d, J=8.1 Hz), 6.97(1H, t, J=7.3 Hz), 7.16(1H, d, J=7.3 Hz), 7.3-7.7(3H, m), 7.8-8.4(3H, m), 8.6-9.2(3H, m), 9.64(1H, s), 14.74(1/3H, s). (2:1 equilibrium mixture)
IR(KBr)cm-1 : 3282, 1690, 1645, 1527, 1421, 1314, 1217, 1028, 994, 911, 753, 701

### Example 35

### N-(2-aminophenyl)-4-[N-[N-(pyridin-3-yl)aminoacetyl]aminomethyl]benzamide (Table 1: Compound 96)

mp: (amorphous)
¹H NMR(270 MHz, DMSO-d6) .delta. ppm: 3.77(2H, d, J=6.6 Hz), 4.37(2H, d, J=5.9 Hz), 4.87(2H, br.s), 6.27(1H, t, J=5.9 Hz), 6.60(1H, dd, J=7.3, 7.3 Hz), 6.78(1H, d, 7.3 Hz), 6.87(1H, d, J=8.1 Hz), 6.96(1H, dd, J=7.3, 8.1 Hz), 7.09(1H, d, J=4.4 Hz), 7.12(1H, d, J=4.4 Hz), 7.16(1H, d, J=8.1 Hz), 7.33(2H, d, J=8.8 Hz), 7.81 (1H, d, J=4.4 Hz), 7.91 (2H, d, J=7.3 Hz), 7.99(1H, d, J=2.9 Hz), 8.59(1H, br.t, J=5.1 Hz), 9.63(1H, br.s)
IR(KBr)cm-1 : 3350, 1658, 1525, 1502, 1314, 750

### Example 36

### N-(2-aminophenyl)-4-[N-(2-aminothiazol-4-yl)acetylaminomethyl]benzamide (Table 1: Compound 220)

mp: (amorphous).
¹ H NMR(270 MHz, DMSO-d6) .delta. ppm: 3.34(2H, s), 4.35(2H, d, J=5.9 Hz), 4.87(2H, s), 6.25(1H, s), 6.59(1H, dd, J=7.3, 7.3 Hz), 6.78(1H, d, J=7.3 Hz), 6.87(2H, s), 6.96(1H, dd, J=7.3, 7.3 Hz), 7.16(1H, d, J=7.3 Hz), 7.37(2H, d, J=8.1 Hz), 7.93(2H, d, J=8.1 Hz), 8.44(1H, t, J=5.9 Hz), 9.62(1H, s)

### Example 37

### N-(2-aminophenyl)-4-[N-(quinolin-6-yl)carbonylaminomethyl]benzamide (Table 1: Compound 231)

mp: 209-210°C
¹ H NMR(270 MHz, DMSO-d6).delta. ppm: 4.62(2H, d, J=5.9 Hz), 4.88(2H, s), 6.60(1H, t, J=7.7 Hz), 6.78(1H, d, J=7.3 Hz), 6.95(1H, d, J=7.3 Hz), 7.17(1H, d, J=7.3 Hz), 7.49(2H, d, J=8.8 Hz), 7.62(1H, dd, J=4.4, 8.1 Hz), 7.96(2H, d, J=8.8 Hz), 8.10(1H, d, J=8.8 Hz), 8.23(1H, dd, J=2.2, 8.8 Hz), 8.38(1H, m), 8.49(1H, d, J=8.1 Hz), 8.58(1H, s), 8.99(1H, s), 9.64(1H, s)
IR(KBr)cm-1 : 3301, 1640, 1614, 1545, 1496, 1312, 910, 853, 745

### Example 38

### N-(2-aminophenyl)-4-[N-(furo[3,2-b]pyridin-2-yl)carbonylaminomethyl]benzamide (Table 1: Compound 233)

mp: 191 °C(dec.)
¹ H NMR(270 MHz, DMSO-d6).delta. ppm: 4.58(2H, d, J=5.9 Hz), 4.88(2H, s), 6.57-6.62(1H m), 6.76-6.79(1H, m), 6.93-6.99(1H, m), 7.15-7.25(1H, m), 7.45-7.52(3H, m), 7.74(1H, s), 7.95(2H, d, J=8.1 Hz), 8.13(1H, d, J=8.8 Hz), 8.63(1H, d, J=3.7 Hz), 9.54(1H, t, J=5.9 Hz), 9.64(1H, s)
IR(KBr)cm-1 : 3406, 1662, 1529, 1507, 1420, 1313, 1209, 1139, 1170, 1139, 924, 741

### Example 39

### N-(2-aminophenyl)-4-[N-(furo[2,3-c]pyridin-2-yl)carbonylaminomethyl]benzamide (Table 1: Compound 234)

mp: 210°C(dec.)
¹ H NMR(270 MHz, DMSO-d6) .delta. ppm: 4.58(2H, J=6.6 Hz), 4.87(2H, s), 6.57-6.62(1H, m), 6.76-6.79(1H, m), 6.93-6.99(1H, m), 7.14-7.17(1H, m), 7.47(2H, d, J=8.1 Hz), 7.66(1H, s), 7.82(1H, d, J=4.4 Hz), 7.96(2H, d, J=8.1 Hz), 8.48(1H, d, J=5.1 Hz), 9.06(1H, s), 9.60-9.64(2H, m)
IR(KBr)cm-1 : 3320, 1653, 1632, 1598, 1457, 1424, 1308, 1187, 1033, 853, 749

### Example 40

### N-(2-hydroxyphenyl)-4-[N-[3-(pyridin-3-yl)propionyl]aminomethyl]benzamide (Table 1: Compound 125)

mp: (amorphous)
¹ H NMR(270 MHz. CD.sub.3 OD) .delta. ppm: 2.61(2H, t, J=7.3 Hz), 3.00(2H, t, J=7.3 Hz), 4.39(2H, s), 7.04(1H, ddd, J=1.5, 8.1, 8.1 Hz), 7.25(2H, d, J=8.1 Hz), 7.33(1H, dd, J=5.1, 8.1 Hz), 7.69(1H, d, J=8.1 Hz), 7.85(2H, d, J=8.1 Hz), 7.86(1H, d, J=8.1 Hz), 8.41(2H, br.s)
IR(neat)cm-1 : 3276, 1645, 1614, 1536, 1509, 1435, 1415, 1385, 1333, 1280, 1247,1091,737

### Example 41

### N-(2-hydroxyphenyl)-4-[N-(pyridin-3-yl)oxyacetylaminomethyl]benzamide (Table 1: Compound 93)

mp: (amorphous)
¹H NMR(270 MHz, DMSO-d6): 4.43(2H, d, J=6.6 Hz), 4.69(2H, s), 6.83(1H, t, J=6.6 Hz), 6.91(1H, d, J=8.1 Hz), 7.68(1H, d, J=6.6 Hz), 7.82(2H, d, J=8.1 Hz), 8.21(1H, d, J=4.4 Hz), 8.35(1H, d, J=2.2 Hz), 8.81(1H, t, J=6.6 Hz), 9.48(1H, s), 9.75(1H, s)
IR(KBr)cm-1 : 3399, 1664, 1535, 1236, 1064

### Example 42

### N-(2-hydroxyphenyl)-4-[N-(pyridin-3-yl)acetylaminomethyl]benzamide (Table 1: Compound 117)

mp: 201-202°C
¹ H NMR(270 MHz, DMSO-d6) .delta. ppm: 3.56(2H, s), 4.37(2H, d, J=5.9 Hz), 6.83(1H, ddd, J=1.5, 8.1, 8.1 Hz), 6.92(1H, br.d, J=8.1 Hz), 7.03(1H, ddd, J=1.5, 8.1, 8.1 Hz), 7.34(1H, dd, J=3.7, 8.1 Hz), 7.37(2H, d, J=8.1 Hz), 7.70(2H, d, J=8.1 Hz), 7.91(2H, d, J=8.1 Hz), 8.45(1H, br.d, J=3.7 Hz), 8.49(1H, s), 8.73(1H, t, J=5.9 Hz), 9.47(1H, s), 9.73(1H, br.s)
IR(KBr)cm-1 : 3272, 3067, 1661, 1647, 1598, 1536, 1455, 1334, 1288, 1194, 1024, 742

### Example 43

### N-(2-aminophenyl)-4-[N-(pyridin-3-yl)oxyacetyl-N-[3-(pyridin-3-yl-)propyl]a minomethyl]benzamide (Table 1: Compound 91)

mp: (amorphous)
¹ H NMR(270 MHz, DMSO-d6) .delta. ppm: 1.77-1.93(2H, m), 2.50-2.63(2H, m), 3.16-3.30(2H, m), 4.63(1.2H, s), 4.71(0.8H, s), 4.88(1.2H, s), 4.95(0.8H, s), 5.05(2H, s), 6.57-6.63(1H, m), 6.77-6.79(1H, m), 6.94-7.00(1H, m), 7.11-7.42(5H, m), 7.58-7.64(1H, m), 7.92-8.02(2H, m), 8.15-8.43(5H, m), 9.65(0.6H, s), 9.69(0.4H, s)(a mixture of rotational isomers)

### Example 44

### N-(2-aminophenyl)-4-[N-methyl-N-(pyridin-3-yl)oxyacetyl]aminomethylbenzamid e (Table 1: Compound 92)

mp: 117-120°C
¹H NMR(270 MHz, DMSO-d6) .delta. ppm: 2.84 and 2.99(total 3H, s), 4.60 and 4.69(total 2H, s), 4.90(2H, br.s), 4.99 and 5.08(total 2H, s), 6.60(1H, dd, J=7.3, 8.1 Hz), 6.78(1H, d, J=8.1 Hz), 6.97(1H, dd, J=7.3, 7.3 Hz), 7.16(1H, d, J=7.3 Hz), 7.30-7.43(4H, m), 7.95 and 8.01(total 2H, d, J=8.1 Hz), 8.17(1H, d, J=4.4 Hz), 8.31(1H, d, J=2.9 Hz), 9.65 and 9.68(total 1H, br.s) (a mixture of rotational isomers)
IR(KBr)cm-1 : 3298, 1665, 1501, 1425, 1310, 1276, 1254, 1078, 799, 746, 703

### Example 45

### Preparation of N-(2-aminophenyl)-4-[N-(pyridin-3-yl)oxamoylaminomethyl]benzamide (Table 1: Compound 95)

(45-1) Ethyl N-(pyridin-3-yl)oxamate (388 mg, 2 mmol) and 638 mg of the compound from the process (1-4) (2 mmol) were dissolved in ethanol, and the mixture was heated with stirring at 40 to 50°C for 2.5 hours. The precipitated crystals were collected by filtration and washed with 2 ml of ethanol and 3 ml of diethyl ether. The crystals were dried to give 724 mg of N-[2-(N-tertbutoxycarbonyl)aminophenyl]-4-[N-(pyridin-3-yl)oxamoylaminome thyl]benzamide (Yield: 74%).
¹ H NMR(270 MHz, DMSO-d6) .delta. ppm: 1.44(9H, s), 4.49(2H, d, J=5.9 Hz), 7.10-7.30(2H, m), 7.35-7.57(5H, m), 7.93(2H, d, J=8.1 Hz), 8.21(1H, br.d, J=5.1 Hz), 8.35(1H, dd, J=1.5, 5.1 Hz), 8.68(1H, br.s), 9.00(1H, d, J=2.9 Hz), 9.70(1H, t, J=5.9 Hz), 9.82(1H, s), 10.98(1H, br.s)

(45-2) To a suspension of 720 mg of the compound from the process (45-1) in 8 ml of methanol was added 8 ml of 4N hydrochloric acid-dioxane solution. After stirring for 3 hours, the mixture was poured into a diluted sodium hydroxide aq. to be basified, and the precipitated crystals were collected by filtration. The crystals were recrystallized from THF/methanol=1:1, to give 280 mg of the desired product.
mp: 254-258°C(dec.)
¹H NMR(270 MHz, DMSO-d6) .delta. ppm: 4.67(2H, d, J=5.9 Hz), 4.89(2H, br.s), 6.59(1H, dd, J=7.3 Hz), 6.77(1H, d, J=8.1 Hz), 6.97(1H, dd, J=6.6, 7.3 Hz), 7.16(1H, d, J=8.1 Hz), 7.38-7.44(1H, m), 7.43(2H, d, J=8.1 Hz), 7.95(2H, d, J=8.1 Hz), 8.18-8.24(1H, m), 8.34(1H, dd, J=1.5, 4.4 Hz), 9.00(1H, d, J=2.1 Hz), 9.63(1H, s), 9.69(1H, br.t, J=6.6 Hz), 10.97(1H, br.s)
IR(KBr.cm-1): 3312, 3270, 1663, 1636, 1521, 1312, 1296, 1019

### Example 46

### Preparation of N-(2-aminophenyl)-4-[N-(pyridin-3-yl)oxyacetylaminomethyl]benzamide (Table 1: Compound 61)

(46-1) To a suspension of 0.22 g of sodium hydride (60% oil dispersion, 5.5 mmol) in 2 ml of DMF was added dropwise a solution of 0.48 g of 3-hydroxypyridine (5.0 mmol) in 2 ml of DMF at room temperature, and the mixture was stirred for an hour. The resulting brown solution was ice-cooled, 0.81 ml of tert-butyl bromoacetate (5.5 mmol) was added, and the mixture was stirred under ice-cooling for an hour followed by stirring at room temperature for 2 hours. After addition of water, the mixture was extracted with ethyl acetate. The organic layer was washed with saturated brine, dried and evaporated. The residue was purified by column chromatography on silica gel (eluent: chloroform:ethyl acetate=5:1), to give 0.34 g of tert-butyl 3-pyridyloxyacetate (Yield: 32.5%) as a clear oil.
¹ H NMR(270 MHz, CDCl.sub.3) .delta. ppm: 1.49(9H, s), 4.56(2H, s), 7.18-7.24(2H, m), 8.26(1H, dd, J=1.5, 3.6 Hz), 8.32(1H, d, J=2.9 Hz)

(46-2) To a solution of 0.14 g of the compound from the process (46-1) (0.67 mmol) in 2 ml of dichloromethane was added 2 ml of trifluoroacetic acid, and the solution was stirred at room temperature for 3 hours. After evaporation, diisopropyl ether was added, and the precipitated solid was collected by filtration and dried to give 0.15 g of 3-pyridyloxyacetic acid trifluoroacetate (Yield: 83.8%) as a light yellow solid.
¹ H NMR(270 MHz, DMSO-d6) .delta. ppm: 4.86(2H, s), 7.57(1H, dd, J=4.4, 8.1 Hz), 7.67(1H, ddd, J=1.5, 1.5, 8.8 Hz), 8.31(1H, d, J=5.1 Hz), 8.46(1H, d, J=2.1 Hz), 13.00(1H, br.s)

(46-3) To a suspension of 100 mg of the compound from the process (46-2) (0.37 mmol) and 255 mg of the compound from Example 1, the process (1-4) (0.75 mmol) in 5 ml of dichloromethane was added 0.14 ml of triethylamine (1.0 mmol), and the mixture was cooled with ice. Under ice-cooling, to the mixture was added a solution of 140 mg of 2-chloro-1,3-dimethylimidazolinium chloride (0.83 mmol) in 6 ml of dichloromethane, and the mixture was warmed to room temperature with stirring for 7 hours, and left at room temperature overnight. After adding water and saturated brine, the mixture was extracted with chloroform.

The organic layer was washed with saturated brine, dried and evaporated. The residue was purified by column chromatography on silica gel (eluent: ethyl acetate: methanol= 10:1) to give 0.37 g of N-[2-(N-tertbutoxycarbonyl)aminophenyl]-4-[N-(pyridin-3-yl)oxyacetylamino methyl]benzamide (Yield: quantitative) as a clear oil.
¹ H NMR(270 MHz, CDCl.sub.3) .delta. ppm: 1.52(9H, s), 4.62(2H, s), 4.63(2H, d, J=7.3 Hz), 6.76(1H, br.s), 6.90-7.00(1H, br.s), 7.15-7.35(5H, m), 7.40(2H, d, J=8.1 Hz), 7.82(1H, d, J=8.1 Hz), 7.95(2H, d, J=8.1 Hz), 8.32(1H, dd, J=2.1, 4.4 Hz), 8.37(1H, d, J=2.8 Hz), 9.20(1H, br.s)

(46-4) To a solution of 175 mg of the compound from the process (46-3) (0.37 mmol) in 2 ml of dioxane and 2 ml of methanol was added 2 ml of 4N hydrochloric acid-dioxane, and the mixture was stirred at room temperature for 2 hours. After adding saturated sodium bicarbonate aq., the mixture was extracted with ethyl acetate. The organic layer was washed with saturated brine, dried and evaporated. To the residue was added methanol and diisopropyl ether, and the precipitated solid was collected by filtration and dried to give 90 mg of N-(2-aminophenyl)-4-[N-(pyridin-3-yl)oxyacetylaminomethyl]benzamide (Yield: 64.6%) as an opalescent solid.
mp: 154-155°C
¹ H NMR(270 MHz, DMSO-d6) delta. ppm: 4.42(2H, d, J=5.9 Hz), 4.69(2H, s), 4.89(2H, br.s), 6.59(1H, dd, :J=7.3, 8.1 Hz), 6.78(1H, d, J=8.1 Hz), 6.97(1H, dd, J=6.6, 7.3 Hz), 7.16(1H, d, J=7.3 Hz), 7.33-7.39(4H, m), 7.92(2H, d, J=8.1 Hz), 8.21(1H, dd, J=1.5, 4.4 Hz), 8.35(1H, d, J=2.9 Hz), 8.80(1H, br.t, J=5.9 Hz), 9.63(1H, br.s)
IR(KBr)cm-1 : 3307, 1672, 1631, 1523, 1456, 1429, 1269, 1231, 803, 756

### Example 47

### Preparation of N-(2-aminophenyl)-4-[N-[2-(pyridin-3-yl)oxy]propionylaminomethyl]benzamide (Table 4: Compound 3)

(47-1) To a suspension of 1.20 g of sodium hydride (60% oil dispersion; 30.0 mmol) in 10 ml of dry DMF at room temperature were added dropwise 2.85 g of 3-hydroxypyridine (30 mmol) in 10 ml of dry DMF, maintaining the temperature below 40°C, and the mixture was stirred at room temperature for 90 min. Under ice-cooling, 6.28 g of tert-butyl 2-bromopropionate (30 mmol) in 10 ml of dry DMF were slowly added dropwise, maintaining the inner temperature within 5 to 10°C, and then the mixture was warmed to room temperature with stirring for 4 hours. After neutralizing with saturated sodium bicarbonate aq., the mixture was extracted with ethyl acetate. The organic layer was washed with water and then saturated brine, dried and evaporated. The residue was purified by column chromatography on silica gel (eluent: n-hexane:ethyl acetate=2:1) to give 4.15 g of tert-butyl 2-(pyridin-3-yl)oxypropionate (Yield: 62%) as a brown oil.
¹ H NMR(270 MHz, CDCl.sub.3) .delta. ppm: 1.44(9H, s), 1.61 (3H, d, J=7.3 Hz), 4.66(1H, q, J=7.3 Hz), 7.13-7.23(2H, m), 8.24(1H, dd, J=1.5, 4.4 Hz), 8.29(1H, d, J=2.1 Hz)

(47-2) To a solution of 1.65 g of the compound from the process (47-1) (7.4 mmol) in 9 ml of dichloromethane was added 9 ml of trifluoroacetic acid, maintaining the temperature below 30°C, and then the mixture was stirred at room temperature for 8 hours. After evaporation, diisopropyl ether was added and the precipitated solid was collected by filtration and dried to give 1.86 g of 2-(pyridin-3-yl)oxypropionic acid trifluoroacetate (Yield 43.5%) as a light brown solid.
¹ H NMR(270 MHz, DMSO-d6) .delta. ppm: 1.53(3H, d, J=6.6 Hz), 5.12(1H, q, J=6.6 Hz), 7.60-7.75(2H, m), 8.35(1H, d, J=5.1 Hz), 8.47(1H, s), 12.9(1H, br.s) (47-3) To a suspension of 0.98 g of the compound from the process (47-2) (3.5 mmol) and 1.02 g of the compound from Example 1, the process (1-4) (3.0 mmol) in 20 ml of dichloromethane was added 1.3 ml of triethylamine (9.0 mmol) and then the mixture was ice-cooled. Under ice-cooling, 0.59 g of 2-chloro-1,3-dimethylimidazolidinium chloride (3.5 mmol) in 5 ml of dichloromethane was added dropwise, and the mixture was stirred for additional 2 hours. The mixture was neutralized with saturated sodium bicarbonate aq., and then extracted with chloroform. The organic layer was washed with saturated brine, dried and evaporated. The residue was purified by column chromatography on silica gel (eluent: ethyl acetate:methanol=10:1) to give 1.64 g of N-[2-(N-tert-butoxycarbonylamino)phenyl]-4-[N-[2-(pyridin-3-yl)oxypropiony I]aminomethyl]benzamide as a mixture with 1,3-di-methyl-2-imidazolinone.
¹ H NMR(270 MHz, CDCI.sub.3) .delta. ppm: 1.51(9H, s), 1.64(3H, d, J=7.3 Hz), 4.54(2H,m), 4.78(1H, q, J=6.6 Hz), 6.87(2H, br.s), 7.13-7.30(6H, m), 7.81(1H, d, J=7.3 Hz), 7.90(2H, d, J=8.1 Hz), 8.29(1H, dd, J=1.5, 4.4 Hz), 8.33(1H, d, J=2.1 Hz), 9.22(1H, br.s)

(47-4) The compound from the process (47-3) (1.64 g) was dissolved in 10 ml of dioxane and 4 ml of methanol. To the solution was added 10 ml of 4N hydrochloric acid-dioxane solution at room temperature, and the mixture was stirred for 2 hours. The mixture was neutralized with saturated sodium bicarbonate aq. and extracted with ethyl acetate. The organic layer was washed with saturated brine, dried and evaporated. To the residue were added methanol and diisopropyl ether, and the precipitated solid was collected by filtration and dried to give 0.71 g of N-(2-aminophenyl)-4-[N-[2-(pyridin-3-yl)oxy]propionylaminomethyl]benzamide (Yield: 60.5% for the 2 steps) as a white solid.
mp: 171-173°C(dec.)
¹H NMR(270 MHz, DMSO-d6) .delta. ppm: 1.51(3H, d, J=6.6 Hz), 4.36(2H, d, J=5.9 Hz), 4.89(2H, br.s), 4.90(1H, t, J=6.6 Hz), 6.60(1H, dd, J=6.6, 7.3 Hz), 6.78(1H, d, J=8.1 Hz), 6.97(1H, dd, J=6.6, 7.3 Hz), 7.15(1H, d, J=7.3 Hz), 7.27(2H, d, J=8.1 Hz), 7.33-7.37(2H, m), 7.89(2H, d, J=8.1 Hz), 8.21(1H, dd, J=2.9, 2.9 Hz), 8.32(1H, d, J=1.5 Hz), 8.82(1H, t, J=5.9 Hz), 9.63(1H, br.s)

### Example 48

### Preparation of N-(2-aminophenyl)-4-[N-(pyridin-3-yl)methoxycarbonylaminomethyl]benzamide (Table 1: Compound 82)

(48-1) To a solution of 384 mg of 3-pyridinemethanol (3.52 mmol) in 5 ml of dry THF were added 523 mg of N,N'-carbonyldiimidazole (3.22 mmol) at room temperature. After stirring for an hour, to the mixture was added 1.0 g of the compound from Example 1, the process (1-4) (2.93 mmol) in 6 ml of dry THF.

After being left at room temperature overnight, to the mixture was added 100 ml of chloroform, and the mixture was washed with water (3.times.20 ml) and then saturated brine, and dried over anhydrous magnesium sulfate. After evaporating the solvent under reduced pressure, the residue was purified by column chromatography on silica gel (eluent: chloroform:methanol=30:1) to give 1.27 g of N-[2-(N-tert-butoxycarbonyl)aminophenyl]-4-[N-(pyridin-3-yl)methoxycarbony laminomethyl]benzamide (Yield: quantitative) as an amorphous solid.
¹H NMR(270 MHz, CDCI.sub.3) .delta. ppm: 1.51(9H, s), 4.45(2H, d, J=5.9 Hz), 5.16(1H, s), 7.10-7.50(7H, m), 7.70(1H, d, J=8.1 Hz), 7.80(1H, d, J=7.3 Hz), 7.93(1H, d, J=8.1 Hz), 8.57(1H, d, J=4.4 Hz), 8.63(1H, s), 9.17(1H, s).

(48-2) The compound from the process (48-1)(1.2 g, 2.8 mmol) was dissolved in 10 ml of methanol. To the solution was added 20 ml of 4N-hydrochloric acid-dioxane. The mixture was stirred at room temperature for 1.5 hours, and then poured into diluted sodium hydroxide aq. and extracted with chloroform (3.times.60 ml). The combined organic layer was washed twice with saturated brine, dried over anhydrous magnesium sulfate and concentrated to give 0.88 g of crystals, which were then recrystallized from 16 ml of ethanol, to give 668 mg of N-(2-aminophenyl)-4-[N-(pyridin-3-yl)methoxycarbonylaminomethyl]benzamide (Yield: 73%).
mp: 159-160°C
¹ H NMR(270 MHz, DMSO-d6) .delta. ppm: 4.28(2H, d, J=5.9 Hz), 4.86(2H, s), 5.10(2H, s), 6.60(1H, t, J=7.3 Hz), 6.78(1H, d, J=7 Hz), 6.97(1H, t, J=7 Hz), 7.17(1H, d, J=8 Hz), 7.30-7.50(3H, m), 7.78(1H, d, J=8 Hz), 7.93(2H, d, J=8 Hz), 8.53(1H, d, J=3.7 Hz), 8.59(1H, s), 9.61(1H, s).
IR(KBr)cm-1 : 3295, 1648, 1541, 1508, 1457, 1309, 1183, 742

As described in Example 48, the compounds of Examples 49 to 87 were prepared, each of whose melting point (mp), 1 H NMR data and/or IR data are shown below.

### Example 49

### N-(2-aminophenyl)-4-[N-(benzyloxycarbonyl)aminomethyl]benzamide (Table 1: Compound 11)

mp: 174-178°C
¹ H NMR(270 MHz, DMSO-d6) .delta. ppm: 4.28(2H, d, J=5.9 Hz), 4.89(2H, br.s), 5.06(2H, s), 6.59(1H, dd, J=7.3, 8.1 Hz), 6.78(1H, d, J=8.1 Hz), 6.97(1H, dd, J=7.3, 8.1 Hz), 7.16(1H, d, J=7.3 Hz), 7.30-7.40(6H, m), 7.93(3H, m), 9.63(1H, s).
IR(KBr)cm-1 : 3332, 1687, 1652, 1536, 1456, 1279, 747

### Example 50

### N-(2-aminophenyl)-4-[N-(4-(imidazol-1-yl)benzyl)oxycarbonylaminomethyl]benz amide (Table 1: Compound 47)

mp: 195-198°C
¹H NMR(270 MHz, DMSO-d6) .delta. ppm: 4.29(2H, d, J=6.6 Hz), 4.88(2H, s), 5.10(2H, s), 6.60-6.63(1H,m), 6.78(1H, d, J=8.1 Hz), 6.97(1H, t, J=7.3 Hz), 7.11(1H, s), 7.16(1H, d, J=7.3 Hz), 7.37(2H, d, J=8.1 Hz), 7.49(2H, d, J=8.8 Hz), 7.66(2H, d, J=8.1 Hz), 7.74(1H, s), 7.92-7.96(3H, m), 8.25(1H, s), 9.62(1H, s)

### Example 51

### N-(2-aminophenyl)-4-[N-(pyridin-2-yl)methoxycarbonylaminomethyl]benzamide (Table 1: Compound 171)

mp: 166-167°C
¹H NMR(270 MHz, DMSO-d6).delta. ppm: 4.30(2H, d, J=5.9 Hz), 4.88(2H, br.s), 5.12(2H, s), 6.60(1H, dd, J=7.3, 8.1 Hz), 6.78(1H, d, J=8.1 Hz), 6.97(1H, ddd, J=1.5, 7.3, 8.1 Hz), 7.16(1H, d, J=7.3 Hz), 7.33(1H, dd, J=3.7, 7.3 Hz), 7.40(3H, d, J=8.1 Hz), 7.83(1H, ddd, J=1.5, 7.3, 8.1 Hz), 7.94(2H, d, J=8.1 Hz), 8.03(1H, t, J=5.9 Hz), 8.55(1H, d, J=5.1 Hz), 9.62(1H, br.s)
IR(KBr)cm-1 : 3334, 1694, 1632, 1580, 1276, 755

### Example 52

### N-(2-aminophenyl)-4-[N-[2-(pyridin-2-yl)ethoxycarbonyl]aminomethyl]benzamid e (Table 1: Compound 172)

mp: 146-148°C
¹ H NMR(270 MHz, DMSO-d6) .delta. ppm: 3.04(2H, t, J=6.6 Hz), 4.23(2H, d, J=5.9 Hz), 4.36(2H, t, J=6.6 Hz), 4.88(2H, br.s), 6.60(1H, dd, J=7.3, 8.1 Hz), 6.78(1H, d, J=8.1 Hz), 6.97(1H, dd, J=7.3, 8.1 Hz), 7.15-7.30(3H, m), 7.34(2H, d, J=8.1 Hz), 7.69-7.77(2H, m), 7.92(2H, d, J=7.3 Hz), 8.50(1H, d, J=4.4 Hz), 9.62(1H, br.s)
IR(KBr)cm⁻¹ : 3330, 1690, 1633, 1594, 1524, 1277, 760

### Example 53

### N-(2-aminophenyl)-4-[N-(6-methylpyridin-2-yl)methoxycarbonylaminomethyl]ben zamide (Table 1: Compound 179)

mp: 138°C
¹ H NMR(270 MHz, DMSO-d6) .delta. ppm: 2.47(3H, s), 4.30(2H, d, J=5.9 Hz), 5.07(4H, s), 6.63(1H, t, J=8.1 Hz), 6.80(1H, d, J=7.34), 6.98(1H, t, J=8.1 Hz), 7.18(3H, d, J=7.3 Hz), 7.40(2H, d, J=8.1 Hz), 7.71(1H, t, J=8.1 Hz), 7.94(2H, d, J=8.1 Hz), 8.03(1H, t, J=5.9 Hz), 9.66(1H, s)
IR(KBr)cm-1 : 3335, 1693, 1634, 1259

### Example 54

### N-(2-aminophenyl)-4-[N-[2-(pyridin-3-yl)ethoxycarbonyl]aminomethyl]benzamid e (Table 1: Compound 83)

mp: 120-125°C
¹ H NMR(270 MHz, DMSO-d6) .delta. ppm: 2.91(2H, t, J=6.6 Hz), 4.22(4H, t, J=6.6 Hz), 4.89(2H, s), 6.55-6.63(1H, m), 6.78(1H, dd, J=8.1, 1.5 Hz), 6.97(1H, t, J=6.6 Hz), 7.17(1H, d, J=6.6 Hz), 7.33(3H, d, J=8.1 Hz), 7.69(1H, d, J=8.1 Hz), 7.79(1H, t, J=6.6 Hz), 7.93(2H, d, J=8.0 Hz), 8.43-8.49(2H, m), 9.62(1H, s)
IR(KBr)cm-1 : 3234, 1705, 1655, 1260

### Example 55

### N-(2-aminophenyl)-4-[N-[3-(pyridin-3-yl)propyloxycarbonyl]aminomethyl]benza mide (Table 1: Compound 84)

mp: 121-124°C
¹ H NMR(270 MHz, DMSO-d6).delta. ppm: 1.83-1.94(2H, m), 2.67(2H, t, J=7.3 Hz), 3.98(2H, t, J=6.6 Hz), 4.26(2H, d, J=5.9 Hz), 4.89(2H, br.s), 6.60(1H, dd, J=8.1, 8.1 Hz), 6.78(1H, d, J=7.3 Hz), 6.97(1H, ddd, J=1.5, 7.3, 8.1 Hz), 7.16(1H, d, J=8.1 Hz), 7.29-7.33(1H, m), 7.37(1H, d, J=8.1 Hz), 7.64(1H, d, J=8.1 Hz), 7.81(1H, dd, J=5.9, 6.6 Hz), 7.94(2H, d, J=8.1 Hz), 8.40-8.44(2H,m), 9.63(1H, br.s)
IR(KBr)cm-1 : 3348, 1696, 1635, 1523, 1458, 1302, 1272, 1141, 1019, 754, 713

### Example 56

### N-(2-aminophenyl)-4-[N-(2-methylpyridin-3-yl)methoxycarbonylaminomethyl]ben zamide (Table 1: Compound 142)

mp: 164-165°C
¹ H NMR(270 MHz, DMSO-d6) .delta. ppm: 2.49(3H, s), 4.28(2H,d, J=6.6 Hz), 4.89(2H, s), 5.10(2H, s), 6.60(1H, t, J=6.6 Hz), 6.78(1H, d, J=8.1 Hz), 6.90(1H, t, J=7.3 Hz), 7.17(1H, d, J=7.3 Hz), 7.21-7.26(1H, m), 7.37(2H, d, J=8.1 Hz), 7.68(1H, d, J=6.6 Hz), 7.92-8.00(3H, m), 8.39(1H, d, J=4.4 Hz), 9.62(1H, s)
IR(KBr)cm-1 : 3332, 1719, 1630, 1260

### Example 57

### N-(2-aminophenyl)-4-[N-(6-methylpyridin-3-yl)methoxycarbonylaminomethyl]ben zamide (Table 1: Compound 144)

mp: 164-165°C
¹ H NMR(270 MHz, DMSO-d6) .delta. ppm: 2.46(3H, s), 4.27(2H, d, J=6.6 Hz), 4.88(2H, s), 5.05(2H, s), 6.59(1H, dt, J=1.5, 8.1 Hz), 6.78(1H, dd, J=1.5, 8.1 Hz), 6.97(1H, dt, J=1.5, 7.3 Hz), 7.17(1H, d, J=7.3 Hz), 7.26(1H, d, J=8.1 Hz), 7.36(2H, d, J=8.1 Hz), 7.67(1H, dd, J=2.2, 8.1 Hz), 7.93(3H, d, J=8.1 Hz), 8.45(1H, d, J=1.5 Hz), 9.62(1H, s)
IR(KBr)cm-1 : 3293, 1701, 1632, 1260

### Example 58

### N-(2-aminophenyl)-4-[N-(2-chloropyridin-3-yl)methoxycarbonylaminomethyl]ben zamide (Table 1: Compound 155)

mp: (amorphous)
¹ H NMR(270 MHz, DMSO-d6) .delta. ppm: 4.30(2H, d, J=5.9 Hz), 5.00(2H, s), 5.13(2H, s), 6.61 (1H, t, J=7.3 Hz), 6.79(1H, dd, J=1.5, 8.1 Hz), 6.98(1H, dt, J=1.5, 7.3 Hz), 7.17(1H, d, J=6.6 Hz), 7.39(2H, d, J=8.8 Hz), 7.47-7.52(1H, m), 7.91-7.96(3H, m), 8.08(1H, t, J=5.9 Hz), 8.40(1H, dd, J=4.4, 1.5 Hz), 9.64(1H, s)
IR(KBr)cm-1 : 3340, 1702, 1632, 1273

### Example 59

### N-(2-aminophenyl)-4-[N-(6-chloropyridin-3-yl)methoxycarbonylaminomethyl]ben zamide (Table 1: Compound 157)

mp: 180-185°C
¹ H NMR(270 MHz, DMSO-d6) .delta. ppm: 4.24(2H, d, J=5.9 Hz), 4.89(2H, br.s), 5.10(2H, s), 6.60(1H, t, J=7.3 Hz), 6.78(1H, d, J=8.1 Hz), 6.97(1H, dt, J=1.5, 8.1 Hz), 7.16(1H, d, J=6.6 Hz), 7.37(2H, d, J=8.1 Hz). 7.56(1H, d, J=8.1 Hz), 7.85-8.02(4H, m), 8.44(1H, d, J=2.2 Hz), 9.62(1H, s)
IR(KBr)cm-1 : 3346, 3282, 1696, 1533, 1271

### Example 60

### N-(2-aminophenyl)-4-[N-(pyridin-4-yl)methoxycarbonylaminomethyl)benzamide (Table 1: Compound 181)

mp: 180-183°C
¹ H NMR(270 MHz, DMSO-d6) .delta. ppm: 4.30(2H, d, J=6.6 Hz), 4.89 (2H, s), 5.12(2H, s), 6.60(1H, dd, J=7.3, 7.3 Hz), 6.78(1H, dd, J=1.5, 7.3 Hz), 6.97(1H, ddd, J=1.5, 7.3, 8.1 Hz), 7.16(1H, d, J=7.3 Hz), 7.34(2H, d, J=5.9 Hz), 7.39(2H, d, J=8.1 Hz), 7.94(2H, d, J=8.1 Hz), 8.09(1H, t, J=5.9 Hz), 8.57(1H, d), 9.64(1H, br.s)
IR(KBr)cm-1 : 3394, 3290, 1711, 1645, 1624, 1535, 1504, 1321, 1251, 1138, 1049, 763

### Example 61

### N-(2-aminophenyl)-4-[N-[2-(thiophen-3-yl)ethoxycarbonyl]aminomethyl]benzami de (Table 1: Compound 203)

mp: (amorphous)
¹ H NMR(270 MHz, DMSO-d6) .delta. ppm: 2.90(2H, t, J=7.3 Hz), 4.17-4.26(4H, m), 4.89(2H, s), 6.60(1H, t, J=8.1 Hz), 6.78(1H, d, J=6.6 Hz), 6.97(1H, t, J=7.3 Hz), 7.06(1H, d, J=5.1 Hz), 7.17(1H, d, J=7.3 Hz), 7.26(1H, s), 7.36(2H, d, J=8.1 Hz), 7.47(1H, t, J=2.2 Hz), 7.81 (1H, t, J=5.9 Hz), 7.93(2H, d, J=8.1 Hz), 9.63(1H, s).
IR(KBr)cm-1 : 3314, 1716, 1638, 1252

### Example 62

### N-(2-aminophenyl)-4-[N-(3-phenyloxazol-5-yl)methoxycarbonylaminomethyl]benz amide (Table 1: Compound 211)

mp: 192-195°C
¹ H NMR(270 MHz, DMSO-d6).delta. ppm: 4.30(2H, d, J=5.9 Hz), 4.89(2H, s), 5.25(2H, s), 6.60(1H, t, J=6.6 Hz), 6.68(1H, d, J=8.1 Hz), 6.94(1H, t, J=7.3 Hz), 7.09(1H, s), 7.16(1H, d, J=7.3 Hz), 7.39(2H, d, J=8.1 Hz), 7.51(4H, d, J=2.2 Hz), 7.87-7.96(5H, m), 8.12(1H, t, J=5.9 Hz), 9.63(1H, s)
IR(KBr)cm-1 : 3292, 1718, 1630, 1262

### Example 63

### N-(2-aminophenyl)-4-[N-(thiazol-5-yl)methoxycarbonylaminomethyl]benzamide (Table 1: Compound 216)

mp: 168-175°C
¹ H NMR(270 MHz, DMSO-d6) .delta. ppm: 4.28(2H, d, J=5.9 Hz), 4.91 (2H, br.s), 5.30(2H, s), 6.60(1H, dd, J=7.3, 7.3 Hz), 6.78(1H, d, J=8.1. Hz), 6.97(1H, dd, J=7.3, 8.1 Hz), 7.16(1H, d, J=7.3 Hz), 7.36(2H, d, J=8.1 Hz), 7.91-8.00(4H, m), 9.09(1H, s), 9.63(1H, s)
IR(KBr)cm-1 : 3346(br.), 1697, 1636, 1525, 1456, 1271, 873, 753

### Example 64

### N-(2-aminophenyl)-4-[N-[2-(4-methylthiazol-5-yl)ethoxycarbonyl]aminomethyl] benzamide (Table 1: Compound 217)

mp: 130-133°C
¹H NMR(270 MHz, DMSO-d6).delta. ppm: 2.32(3H, s), 3.07(2H, t, J=5.9 Hz), 4.15(2H, t, J=5.9 Hz), 4.25(2H, d, J=6.6 Hz), 4.89(2H, s), 6.60(1H, t, J=5.9 Hz), 6.78(1H, dd, J=7.3, 1.5 Hz), 6.97(1H, dt, J=1.5, 7.3 Hz), 7.16(1H, d, J=8.1 Hz), 7.35(2H, d, J=8.1 Hz), 7.83(1H, t, J=5.9 Hz), 7.94(2H, d, J=8.1 Hz), 8.85(1H, s), 9.62(1H, s)
IR(KBr)cm-1 : 3350, 1691, 1635, 1270

### Example 65

### N-(2-aminophenyl)-4-[N-(1-methylpiperidin-3-yl)methoxycarbonylaminomethyl]b enzamide (Table 1: Compound 225)

mp: 130-135°C
¹H NMR(270 MHz, DMSO-d6) .delta. ppm: 1.49-1.78(3H, m), 1.83-2.01(3H, m), 2.30(3H, s), 2.85(2H, t), 3.74-3.94(2H, m), 4.25(2H, d, J=5.8 Hz), 6.55-6.62(3H, m), 6.78(1H, d, J=8.1 Hz), 6.97(1H, t, J=7.3 Hz), 7.16(1H, d, J=8.1 Hz), 7.37(2H, d, J=8.1 Hz), 7.79(1H, t, J=6.6 Hz), 7.93(2H, d, J=8.0 Hz), 9.66(1H, s)
IR(KBr)cm-1 : 3323, 2722, 1702, 1648, 1263

### Example 66

### N-(2-aminophenyl)-4-[N-(4-methylpiperazin-1-yl)methoxycarbonylaminomethyl]b enzamide (Table 1: Compound 227)

mp: (amorphous)
¹H NMR(270 MHz, DMSO-d6).delta. ppm: 1.73(2H, t, J=6.6 Hz), 2.36-2.63(13H, m), 4.00(2H, t, J=6.6 Hz), 4.30(2H, d, J=5.8 Hz), 6.55-6.63(4H, m), 6.78(1H, d, J=6.6 Hz), 6.97(1H, t, J=7.3 Hz), 7.16(1H, d, J=7.3 Hz), 7.37(2H, d, J=8.7 Hz), 7.73(1H, t, J=5.9 Hz), 7.94(2H, d, J=8.0 Hz), 9.66(1H, s)
IR(KBr)cm-1 : 3341, 2706, 1701, 1262

### Example 67

### N-(2-aminophenyl)-4-[N-(tetrahydrofuran-3-yl)methoxycarbonylaminomethyl]ben zamide (Table 1: Compound 221)

mp: (amorphous)
¹H NMR(270 MHz, DMSO-d6) .delta. ppm: 1.50-1.60(1H, m), 1.88-2.00(1H, m), 2.44-2.54(1H, m), 3.41-3.47(1H, m), 3.56-3.77(3H, m), 3.85-4.04(2H, m), 4.25(2H, d, J=5.9 Hz), 4.89(2H, s), 6.60(1H, dd, J=7.3, 7.3 Hz), 6.78(1H, d, J=8.1 Hz), 6.97(1H, dd, J=7.3, 8.1 Hz), 7.17(1H, d, J=8.1 Hz), 7.37(2H, d, J=8.1 Hz), 7.81 (1H, t, J=5.9 Hz), 7.94(2H, d, J=8.1 Hz), 9.62(1H, br.s)
IR(KBr)cm-1 : 3349, 1695, 1635, 1523, 1457, 1259, 754

### Example 68

### N-(2-aminophenyl)-4-[N-(phenoxycarbonyl)aminomethyl]benzamide (Table 1: Compound 12)

mp: 174-175°C
¹ H NMR(270 MHz, DMSO-d6) .delta. ppm: 4.36(2H, d, J=5.9 Hz), 4.90(2H, br.s), 6.60(1H, dd, J=7.3, 7.3 Hz), 6.77(1H, dd, J=7.3, 7.3 Hz), 6.98(1H, ddd, J=1.5, 7.3, 7.3 Hz), 7.05-7.24(4H, m), 7.39-7.46(4H, m), 7.97(2H, d, J=8.1 Hz), 8.41(1H, t, J=5.9 Hz), 9.65(1H, br.s)
IR(KBr)cm-1 : 3443, 3362, 3313, 1732, 1706, 1636, 1527, 1493, 1458, 1305, 1217,748

### Example 69

### N-(2-aminophenyl)-4-[N-(pyridin-3-yl)oxycarbonylaminomethyl]benzamide (Table 1: Compound 81)

mp: 209°C(dec.)
¹H NMR(270 MHz, DMSO-d6).delta. ppm: 4.38(2H, d, J=6.6 Hz), 4.90(2H, br.s), 6.55-6.63(1H, m), 6.78(1H, d, J=8.1 Hz), 7.00(1H, dd, J=7.3, 7.3 Hz), 7.17(1H, d, J=8.8 Hz), 7.37-7.47(3H, m), 7.64(1H, d, J=8.8 Hz), 7.97(2H, d, J=8.1 Hz), 8.43(2H, d, J=3.1 Hz), 8.59(1H, t, J=5.9 Hz), 9.66(1H, br.s)

### Example 70

### N-(2-amino-5-fluorophenyl)-4-[N-(pyridin-3-yl)methoxycarbonylaminomethyl]be nzamide (Table 1: Compound 110)

mp: 160-162°C
¹H NMR(270 MHz, DMSO-d6) .delta. ppm: 4.28(2H, d, J=6.6 Hz), 4.81(2H, s), 5.10(2H, s), 6.70-6.90(2H, m), 7.10-8.00(8H, m), 8.53(1H, d, J=3.6 Hz), 8.59(1H, s), 9.61(1H, s)
IR(KBr)cm-1 : 3269, 1716, 1638, 1488, 1436, 1247, 1141, 1043, 744

### Example 71

### N-(2-aminophenyl)-4-[N-(2-aminophenyl)methoxycarbonylaminomethyl]benzamide (Table 1: Compound 51)

mp: 149-151 °C(dec.)
¹ H NMR(270 MHz, DMSO-d6).delta. ppm: 4.28(2H, d, J=5.9 Hz), 4.88(2H, s), 4.96(2H, s), 5.06(2H, s), 6.53(1H, dd, J=7.3, 7.3 Hz), 6.56-6.67(2H, m), 6.78(1H, dd, J=1.5, 8.1 Hz), 6.93-7.12(3H, m), 7.16(1H, d, J=6.6 Hz), 7.38(2H, d, J=8.1 Hz), 7.86(1H, t-like, J=5.9 Hz), 7.93(2H, d, J=8.1 Hz), 9.61(1H, s)
IR(KBr)cm-1 : 3336, 1685, 1632, 1527, 1276, 748

### Example 72

### N-(2-aminophenyl)-4-[N-(quinuclidin-3-yl)oxycarbonylaminomethyl]benzamide (Table 1: Compound 228)

mp: (amorphous)
¹ H NMR(270 MHz, DMSO-d6).delta. ppm: 1.30-1.90(4H, m), 1.90(1H, br.s), 2.45-2.80(6H, m), 3.04-3.13(1H, m), 4.15(2H, d, J=5.9 Hz), 4.55-4.60(1H, m), 4.88(2H, br.s), 6.60(1H, ddd, J=1.5, 7.3, 7.3 Hz), 6.78(1H, d, J=8.1 Hz), 6.97(1H, ddd, J=1.5, 7.3, 7.3 Hz), 7.17(1H, d, J=6.6 Hz), 7.37(2H, d, J=8.1 Hz), 7.78(1H, t, J=5.9 Hz), 7.94(1H, d, J=7.3 Hz), 9.62(1H, s)
IR(KBr)cm-1 : 3328, 2942, 1700, 1648, 1504, 1259, 749

### Example 73

### N-(2-aminophenyl)-4-[N-(3-aminophenyl)methoxycarbonylaminomethyl]benzamide (Table 1: Compound 52)

mp: 149-153°C(dec.)
¹ H NMR(270 MHz, DMSO-d6).delta. ppm: 4.27(2H, d, J=5.9 Hz), 4.88 and 4.89(total 4H, each br.s), 5.08(2H, s), 6.47-6.63(3H, m), 6.78(1H, d, J=8.1 Hz), 6.94-7.02(2H, m), 7.15(1H, dd, J=7.3, 8.8 Hz), 7.37(2H, d, J=8.1 Hz), 7.84(1H, t, J=5.9 Hz), 7.93(2H, d, J=8.8 Hz), 9.61(1H, br.s)
IR.(KBr)cm-1 : 3367, 1682, 1632, 1523, 1457, 1261, 754

### Example 74

### N-(2-aminophenyl)-4-[N-(1-methylimidazol-5-yl)methoxycarbonylaminomethyl]benzamide (Table 1: Compound 218)

mp: 162-165°C(dec.)
¹ H NMR(270 MHz, DMSO-d6) .delta. ppm: 3.62(3H, s), 4.27(2H, d, J=5.9 Hz), 4.91(2H, br.s), 5.05(2H, s), 6.60(1H, dd, J=7.3, 7.3 Hz), 6.78(1H, d, J=8.1 Hz), 6.95-7.00(2H, m), 7.16(1H, d, J=7.3 Hz), 7.36(2H, d, J=8.1 Hz), 7.63(1H, s), 7.87-7.95(3H, m), 9.64(1H, br.s)
IR(KBr)cm-1 : 3293, 1688, 1651, 1534, 1506, 1259, 1121, 1043, 748

### Example 75

### N-(2-amino-4-chlorophenyl)-4-[N-(pyridin-3-yl)methoxycarbonylaminomethyl]be nzamide (Table 1: Compound 113)

mp: 167-170°C
¹ H NMR(270 MHz, DMSO-d6) .delta. ppm: 4.28(2H, d, J=5.9 Hz), 5.10(2H, s), 5.21(2H, s), 6.72(1H, dd, J=2.2, 8.1 Hz), 6.81(1H, d, J=2.2 Hz), 7.16(1H, d, J=8.1 Hz), 7.37(2H, d, J=8.1 Hz), 7.78(1H, d, J=8.1 Hz), 7.92(2H, d, J=8.1 Hz), 8.53(1H, d, J=4.4 Hz), 8.59(1H, s), 9.60(1H, s)
IR(KBr)cm-1 : 3347, 3062, 2931, 1653, 1576, 1505, 1456, 1428, 1301, 1232, 1114, 1070, 1019

### Example 76

### N-(2-aminophenyl)-4-[N-(5-methoxypyridin-3-yl)methoxycarbonylaminomethyl]be nzamide (Table 1: Compound 161)

mp: 169-170°C
¹ H NMR(270 MHz, DMSO-d6) .delta. ppm: 3.83(3H, s), 4.29(2H, d, J=6.6 Hz), 4.87(2H, s), 5.09(2H, s), 6.57-6.62(1H, m), 6.76-6.79(1H, m), 6.94-6.99(1H, m), 7.14-7.18(1H, m), 7.36-7.39(3H, m), 7.91-7.99(3H, m), 8.19-8.30(2H, m), 9.63(1H, s)
IR(KBr)cm-1 : 3330, 1694, 1633, 1524, 1457, 1298, 1269, 1045, 760

### Example 77

### N-(2-aminophenyl)-4-[N-(pyrazin-2-yl)methoxycarbonylaminomethyl]benzamide (Table 1: Compound 192)

mp: 182°C
¹ H NMR(270 MHz, DMSO-d6).delta. ppm: 4.30(2H, d, J=6.6 Hz), 4.88(2H, br.s), 5.20(2H, s), 6.60(1H, dd, J=7.3, 8.1 Hz), 6.78(1H, d, J=8.1 Hz), 6.97(1H, dd, J=6.6, 8.1 Hz), 7.16(1H, d, J=7.3 Hz), 7.39(2H, d, J=8.8 Hz), 7.94(2H, d, J=8.8 Hz), 8.08(1H, t-like, J=6.6 Hz), 8.61(1H, s), 8.65(1H, s), 8.68(1H, s), 9.63(1H, s)
IR(KBr)cm-1 : 3266, 1709, 1632, 1535, 1508, 1284, 1055, 1022, 744

### Example 78

### N-(2-amino-5-methoxyphenyl)-4-[N-(pyridin-3-yl)methoxycarbonylaminomethyl]b enzamide (Table 1: Compound 121)

mp: 141-143°C
¹H NMR(270 MHz, DMSO-d6) .delta. ppm: 3.66(3H, s), 4.29(2H, d, J=5.9 Hz), 4.51(2H, br.s), 5.10(2H, s), 6.63(1H, dd, J=2.9, 8.8 Hz), 6.74(1H, d, J=8.8 Hz), 6.91(1H, d, J=2.2 Hz), 7.38(2H, d, J=8.8 Hz), 7.41(1H, s), 7.79(1H, d, J=8.1 Hz), 7.92(2H, d, J=8.1 Hz), 7.98(1H, t, J=5.9 Hz), 8.54(1H, d, J=3.7 Hz), 8.60(1H, s), 9.65(1H, s)

### Example 79

### N-(2-aminophenyl)-4-[N-(pyridin-3-yl)methyl-N-(pyridin-3-yl)methoxycarbonyl aminomethyl]benzamide (Table 1: Compound 109)

mp: (amorphous)
¹ H NMR(270 MHz, DMSO-d6) .delta. ppm: 4.50(2H, s), 4.56(2H, s), 4.87(2H, s), 5.21(2H, s), 6.60(1H, t, J=7.7 Hz), 6.78(1H, d, J=7.3 Hz), 6.97(1H, d, J=7.3 Hz), 7.17(1H, d, J=7.3 Hz), 7.20-7.50(4H, m), 7.60-8.00(4H, m), 8.40-8.60(4H, m), 9.65(1H, s)
IR(KBr)cm-1 : 3268, 1700, 1504, 1246, 1120, 940, 714

### Example 80

### N-(2-aminophenyl)-4-[N-(3-(pyridin-3-yl)propyl]-N-(pyridin-3-yl)methoxycarbonyl aminomethyl]benzamide (Table 1: Compound 120)

mp: (amorphous)
¹ H NMR(270 MHz, DMSO-d6) .delta. ppm: 1.75-1.90(2H, m), 2.48-2.62(2H, m), 3.20-3.36(2H, m), 4.55(2H, s), 4.89(2H, s), 5.16(2H, s), 6.57-6.63(1H, m), 6.76-6.80(1H, m), 6.94-6.99(1H, m), 7.14-7.17(1H, m), 7.32-7.74(6H, m), 7.94(2H, d, J=8.1 Hz), 8.30-8.65(4H, m), 9.64(1H, s)

### Example 81

### N-(2-hydroxyphenyl)-4-[N-(pyridin-3-yl)methyl-N-(pyridin-3-yl)methoxycarbon ylaminomethyl]benzamide (Table 1: Compound 115)

mp: (amorphous)
¹ H NMR(270 MHz, DMSO-d6) delta. ppm: 4.52(2H, s), 4.57(2H, s), 5.20(2H, s), 6.84(1H, t, J=6.6 Hz), 6.93(1H, d, J=6.6 Hz), 7.03(1H, d, J=7.3 Hz), 7.37(4H, m), 7.68(2H, dd, J=1.5, 8.1 Hz), 7.92(2H, br.s), 8.53(4H, m), 9.49(1H, s), 9.77(1H, br.s)
IR(KBr)cm-1 : 3035, 1698, 1243, 1118, 754, 640

### Example 82

### N-(2-hydroxyphenyl)-4-[N-(pyridin-3-yl) methoxycarbonylaminomethyl]benzamide (Table 1: Compound 111)

mp: 162-164°C
¹H NMR(270 MHz, DMSO-d6) .delta. ppm: 4.29(1H, d, J=5.9 Hz), 5.10(2H, s), 6.83(1H, t, J=8.1 Hz), 6.92(1H, d, J=6.6 Hz), 7.07(1H, t, J=6.6 Hz), 7.39(2H, d, J=8.8 Hz), 7.43(1H, d, J=5.1 Hz), 7.68(2H, d, J=8.1 Hz), 7.80(1H, d, J=8.1 Hz), 7.92(2H, d, J=8.1Hz), 7.99(1H, t, J=5.9 Hz), 8.54(1H, d, J=4.4 Hz), 8.60(1H, s), 9.49(1H, s), 9.76(1H, br.s)
IR(KBr)cm-1 : 3333, 3259, 1694, 1645, 1529, 1267, 720

### Example 83

### N-(2,4-dihydroxyphenyl)-4-[N-(pyridin-3-yl) methoxycarbonylaminomethyl]benzamide (Table 1: Compound 116)

mp: (amorphous)
¹ H NMR(270 MHz, DMSO-d6) .delta. ppm: 4.27(2H, d, J=6.6 Hz), 5.10(2H, s), 6.20(2H, dd, J=2.2, 8.1 Hz), 6.39(2H, d, J=2.9 Hz), 6.88(2H, d, J=8.8 Hz), 7.33(1H, d, J=8.1 Hz), 7.41(1H, dd, J=5.1, 7.1 Hz), 7.89(1H, d, J=8.8 Hz), 7.98(1H, t, J=6.6 Hz), 8.05(2H, s), 8.52(1H, m), 8.59(1H, s), 9.30(2H, br.s)
IR(KBr)cm-1 : 3387, 1702, 1612, 1311, 1169, 845

### Example 84

### N-(2-hydroxy-5-methylphenyl)-4-[N-(pyridin-3-yl) methoxycarbonylaminomethyl]benzamide (Table 1: Compound 118)

mp: 155-155.5°C
¹ H NMR(270 MHz, DMSO-d6) delta. ppm: 2.22(3H, s), 4.29(2H, d, J=5.8 Hz), 5.11(2H, s), 6.82(2H, m), 7.39(2H, d, J=8.8 Hz), 7.42(2H, m), 7.51(1H, s), 7.79(1H, d, J=8.1 Hz), 7.92(1H, d, J=8.1 Hz), 7.98(1H, t, J=5.9 Hz), 8.54(1H, d, J=4.4 Hz), 8.60(1H, s), 9.48(2H, d, J=8.1 Hz)
IR(KBr)cm-1 : 3306, 1723, 1655, 1525, 801, 639

### Example 85

### N-(2-hydroxy-5-methoxyphenyl)-4-[N-(pyridin-3-yl)methoxycarbonylaminomethyl ]benzamide (Table 1: Compound 119)

mp: 175-176°C
¹ H NMR(270 MHz, DMSO-d6).delta. ppm: 3.69(3H, s), 4.29(2H, d, J=5.9 Hz), 5.10(2H, s), 6.63(1H, dd, J=2.9, 8.7 Hz), 6.84(1H, d, J=8.8 Hz), 7.41(4H, m), 7.79(1H, d, J=8.1 Hz), 7.91(1H, d, J=8.1 Hz), 7.99(1H, t, J=5.9 Hz), 8.54(1H, d, J=5.1 Hz), 8.60(1H, s), 9.31(1H, s), 9.45(1H, s)
IR(KBr)cm-1 : 3305, 1687, 1573, 1262, 1039, 868

### Example 86

### N-(2-aminophenyl)-4-[N-[2-(pyridin-3-yl)ethoxycarbonyl]amino]benzamide (Table 1: Compound 124)

mp: (amorphous)
¹ H NMR(270 MHz, DMSO-d6) .delta. ppm: 3.00(2H, t, J=6.6 Hz), 4.37(2H, t, J=6.6 Hz), 4.87(2H, br.s), 6.60(1H, t, J=7.3 Hz), 6.97(1H, t, J=7.3 Hz), 7.15(1H, d, J=7.3 Hz), 7.36(1H, dd, J=4.4, 8.1 Hz), 7.56(2H, d, J=8.8 Hz), 7.92(2H, d, J=8.8 Hz), 8.46(1H, d, J=4.4 Hz), 8.54(1H, d, J=2.2 Hz), 9.95(1H, s)
IR(KBr)cm-1 : 3285, 1695, 1519, 1315, 1233, 1079

### Example 87

### N-(2-aminophenyl)-5-[(pyridin-3-yl)methoxycarbonyl]aminobenzofuran-2-carbox yamide (Table 3: Compound 2)

mp: 173-174°C
¹ H NMR(270 MHz, DMSO-d6) .delta. ppm: 5.22(2H, s), 6.60(1H, dd, J=8.1, 8 Hz), 6.79(1H, dd, J=1.5, 8.1 Hz), 7.00(1H, dd, J=8.1, 8 Hz), 7.20(1H, dd, J=1.5, 8.1 Hz), 7.44(1H, m), 7.48(1H, dd, J=1.5, 8.8 Hz), 7.61(1H, d, J=8.8 Hz), 7.67(1H, s), 7.88(1H, dd, J=1.5, 8 Hz), 7.96(1H, d, J=1.5 Hz), 8.56(1H, dd, J=1.5, 4.8 Hz), 8.68(1H, d, J=1.5 Hz), 9.83(1H, s), 9.91(1H, s)
IR(KBr)cm-1 : 3308, 1707, 1667, 1584, 1536, 1452, 1316, 1248, 1157, 1128, 1070,955,879,795,748,710

### Example 88

### Preparation of N-(2-aminophenyl)-4-[N-(pyridin-3-yl)methoxythiocarbonylaminomethyl]benzam ide (Table 1: Compound 86)

(88-1) To a solution of 20 mg of 3-pyridinemethanol (0.18 mmol) in 5 ml of dry THF were added 30 mg of N,N'-thiocarbonyldiimidazole (0.16 mmol) at room temperature. After stirring overnight, to the mixture were added 50 mg of the compound from Example 1, the process (1-4) (0.14 mmol).

After leaving at room temperature overnight, to the solution was added 100 ml of chloroform, and the solution was washed with water (3.times.20 ml) and then saturated brine, and dried over anhydrous magnesium sulfate. After evaporation, the residue was purified by column chromatography on silica gel(eluent:chloroform:methanol=30:1) to give 70 mg of N-[2-(N-tertbutoxycarbonyl)aminophenyl]-4-[N-(pyridin-3-yl)methoxythiocar bonylaminomethyl]benzamide (Yield: 88%) as amorphous.
¹ H NMR(270 MHz, DMSO-d6).delta. ppm: 1.45(9H, s), 4.73(2H, d, J=5.9 Hz), 5.52(2H, s), 6.73-7.33(3H, m), 7.35-7.43(2H, m), 7.58-7.95(5H, m), 8.14-8.65(3H, m), 9.80(1H, s), 9.91(1H, t)

(88-2) To a solution of 50 mg of the compound from the process (88-1) (0.10 mmol) in 3 ml of methanol was added 3 ml of 4N hydrochloric acid-dioxane, and the mixture was stirred at room temperature for 1.5 hours. The mixture was poured into diluted sodium hydroxide aq. to neutralize the residual hydrochloric acid, and then was extracted with chloroform (3.times.10 ml). The organic layer was washed twice with saturated brine, dried over anhydrous magnesium sulfate and concentrated to give 34 mg of N-(2-aminophenyl)-4-[N-(pyridin-3-yl)methoxythiocarbonylaminomethyl]benzam ide (Yield: 87%).
mp: 154-156°C(dec.)
¹ H NMR(270 MHz, DMSO-d6) .delta. ppm: 4.73(2H, d, J=5.9 Hz), 4.88(2H, s), 5.52(2H, s), 6.60(1H, t, J=7.3 Hz), 6.77(1H, d, J=8.1 Hz), 6.96(1H, t, J=8.1 Hz), 7.16(1H, d, J=7.3 Hz), 7.29-7.41(3H, m), 7.83-7.95(3H, m), 8.50-8.56(1H, m), 8.65(1H, s), 9.62(1H, s), 9.93(1H, s)
IR(KBr)cm-1 : 3204, 3035, 1631, 1523, 1456, 1289, 1191, 920, 753

### Example 89

### Preparation of N-(2-aminophenyl)-4-[N'-(pyridin-3-ylmethyl)ureidomethyl]benzamide (Table 1: Compound 88)

(89-1) To a solution of 0.28 g of 3-picolylamine (2.6 mmol) in 10 ml of THF was added 0.42 g of N,N'-carbonyldiimidazole (2.4 mmol) at room temperature, and the mixture was stirred for an hour. To the solution was added 0.58 g of the compound from Example 1, the process (1-4) (1.8 mmol) at room temperature, and the solution was stirred for 3 hours and then left overnight.

After diluting with water, the mixture was extracted with ethyl acetate. The organic layer was washed with saturated brine, dried and evaporated. The residue was purified by column chromatography on silica gel (eluent:ethyl acetate:methanol=10:1) to give 0.77 g of N-[2-(N-tertbutoxycarbonyl)amino]phenyl-4-[N'-(pyridin-3-ylmethyl)ureidom ethyl]benzamide (Yield: 90%) as a white amorphous solid.
¹ H NMR(270 MHz, CDCl.sub.3) .delta. ppm: 1.46(9H, s), 4.20(2H, d, J=5.1 Hz), 4.28(2H, d, J=4.3 Hz), 6.10-6.30(2H, m), 7.00-7.25(4H, m), 7.33(1H, d, J=7.3 Hz), 7.49-7.54(2H, m), 7.58-7.64(3H, m), 7.75(1H, s), 8.28(1H, br.s), 8.39(1H, d, J=5.1 Hz), 9.65(1H, br.s)

(89-2) To a solution of 0.63 g of the compound from the process (89-1) (1.32 mmol) in 4 ml of dioxane and 2 ml of methanol was added 4 ml of 4N hydrochloride-dioxane, and the mixture was stirred at room temperature for 2 hours. After adding saturated sodium bicarbonate aq., the mixture was extracted with ethyl acetate-methyl ethyl ketone. The organic layer was washed with saturated brine, dried and evaporated. The residue was washed with diisopropyl ether to give 0.37 g of N-(2-aminophenyl)-4-[N'-(pyridin-3-ylmethyl)ureidomethyl]benzamide (Yield: 74.7%) as a brown solid.
mp: 167-175°C
¹ H NMR(270 MHz, DMSO-d6) .delta. ppm: 4.27(2H, d, J=5.9 Hz), 4.31(2H, d, J=5.9 Hz), 4.89(2H, br.s), 6.57-6.63(3H, m), 6.78(1H, d, J=8.1 Hz), 6.97(1H, dd, J=7.3, 8.1 Hz), 7.17(1H, d, J=7.3 Hz), 7.32-7.38(3H, m), 7.66(1H, d, J=8.1 Hz), 7.93(2H, d, J=8.1 Hz), 8.44(1H, d, J=5.1 Hz), 8.49(1H, d, J=2.1 Hz), 9.63(1H, br.s)
IR(KBr)cm-1 : 3344, 3241, 1645, 1560, 1527, 1505, 1283, 751, 708

As described in Example 89, the compounds of Examples 90 to 95 were prepared, each of whose melting point (mp), 1 H NMR data and/or IR data are shown below.

### Example 90

### N-(2-aminophenyl)-4-[N'-(3-aminophenyl)ureidomethyl]benzamide (Table 1: Compound 24)

mp: 206-208°C(dec.)
¹ H NMR(270 MHz, DMSO-d6) .delta. ppm: 4.35(2H, d,J=5.9 Hz), 4.93(4H, br.s), 6.13(1H, d, J=7.3 Hz), 6.51-6.62(3H, m), 6.74-6.98(3H, m), 7.12-7.18(1H, m), 7.41(2H,d, J=8.1 Hz), 7.94(2H, d, J=8.1 Hz), 8.28(1H, s), 9.61(1H, s)
IR(KBr)cm-1 : 3356, 3269, 1640, 1555, 1495, 1458, 1308, 1236, 753

### Example 91

### N-(2-aminophenyl)-4-[N'-(pyridin-3-yl)ureidomethyl]benzamide (Table 1: Compound 87)

mp: 187-190°C
¹H NMR(270 MHz, DMSO-d6) .delta. ppm: 4.39(2H, d, J=5.9 Hz), 4.89(2H, br.s), 6.59(1H, d, J=7.3, 7.3 Hz), 6.77(1H, d, J=6.6 Hz), 6.88(1H, t, J=5.9 Hz), 6.97(1H, ddd, J=1.5, 6.6, 7.3 Hz), 7.16(1H, d, J=8.1 Hz), 7.26(1H, dd, J=4.4, 8.1 Hz), 7.42(2H, d, J=8.8 Hz), 7.95(2H, d, J=8.1 Hz), 7.89-7.96(1H, m), 8.12(1H, dd, J=1.5, 4.4 Hz), 8.56(1H, d, J=3.0 Hz), 8.85(1H, s), 9.62(1H, s)
IR(KBr)cm-1 : 3248, 1663, 1541, 1423, 1280, 1054

### Example 92

### N-(2-aminophenyl)-4-[N'-(3-aminophenyl)thioureidomethyl]benzamide (Table 1: Compound 25)

mp: 123°C(dec.)
¹ H NMR(270 MHz, DMSO-d6) .delta. ppm: 4.80(2H, d, J=5.1 Hz), 4.87(2H, s), 5.12(2H, s), 6.36(1H, dd, J=1.5, 8.1 Hz). 6.48-6.63(3H, m), 6.78(1H, d, J=6.6 Hz), 6.94-7.00(2H, m), 7.17(1H, d, J=8.1 Hz), 7.42(2H, d, J=8.1 Hz), 7.92-8.01(3H, m), 9.46(1H, s), 9.61(1H, s)
IR(KBr)cm-1 : 3335, 1616, 1528, 1503, 1456, 1311, 864, 751

### Example 93

### N-(2-aminophenyl)-4-[N'-(3-nitrophenyl)thioureidomethyl]benzamide (Table 1: Compound 20)

mp: 160°C(dec.)
¹ H NMR(270 MHz, DMSO-d6) .delta. ppm: 4.87(2H, d, J=5.1 Hz), 7.27-7.33(3H, m), 7.46-7.63(5H, m), 7.89-7.95(2H, m), 8.05(2H, d, J=8.1 Hz), 8.70(1H, s), 8.84(1H, t, J=8.9 Hz), 10.37(1H, s)

### Example 94

### N-(2-amino-5-fluorophenyl)-4-[N'-(pyridin-3-yl)methylureidomethyl]benzamide (Table 1: Compound 112)

mp: (amorphous)
¹H NMR(270 MHz, DMSO-d6) .delta. ppm: 4.77(4H, d, J=5.1 Hz), 4.85(2H, s), 6.81 (2H, m), 7.16(1H, dd, J=2.9, 10.3 Hz), 7.39(1H, dd, J=5.1, 8.1 Hz), 7.53(2H, d, J=8.1 Hz), 7.81(1H, d, J=8.1 Hz), 7.93(2H, d, J=8.1 Hz), 8.51(1H, dd, J=1.5, 5.1 Hz), 8.62(1H, d, J=1.5 Hz), 9.66(1H, s)
IR(KBr)cm-1 : 3399, 1730, 1638, 1508, 1444, 1411

### Example 95

### N-(2-hydroxyphenyl)-4-[N'-(pyridin-3-yl)methylureidomethyl]benzamide (Table 1: Compound 114)

mp: (amorphous)
¹ H NMR(270 MHz, DMSO-d6) .delta. ppm: 4.43(2H, d, J=6.6 Hz), 4.69(2H, s), 6.83(1H, t, J=6.6 Hz), 6.91(1H, d, J=8.1 Hz), 7.68(1H, d, J=6.6 Hz), 7.82(2H, d, J=8.1 Hz), 8.21(1H, d, J=4.4 Hz), 8.35(1H, d, J=2.2 Hz), 8.81(1H, t, J=6.6 Hz), 9.48(1H, s), 9.75(1H, s)
IR(KBr)cm-1 : 3399, 1664, 1535, 1236, 1064

### Example 96

### Preparation of N-(2-aminophenyl)-4-[2-[N-(pyridin-3-yl)acetylamino]ethyl]benzamide (Table 1: Compound 77)

(96-1) To a suspension of 3.40 g of terephthalaldehydic acid (22.6 mmol) in 25 ml of toluene was added 4 ml of thionyl chloride, and the mixture was heated with stirring at 80°C for 2 hours. After cooling and evaporation, the residue was dissolved in 50 ml of THF to give a solution of the acid chloride. To a solution of 4.16 g of the compound from Example 1, the process (1-2) (20.0 mmol) in 10 ml of THF was added 6 ml of triethylamine (42.8 mmol) and then the above solution of the acid chloride was added dropwise under ice-cooling over 30 min.

After stirring for 5 hours, to the mixture was added saturated sodium bicarbonate aq., and the mixture was extracted with ethyl acetate. The organic layer was washed with saturated brine, dried and evaporated. The residue was purified by column chromatography on silica gel (gradient elution with chloroform to chloroform:ethyl acetate=10:1) to give 3.42 g of N-[2-(N-tertbutoxycarbonyl)aminophenyl]-4-formylbenzamide (Yield: 50.2%) as a light brown solid.
¹ H NMR(270 MHz, CDCl.sub.3) .delta. ppm: 1.52(9H, s), 6.77(1H, br.s), 7.16-7.18(2H, m), 7.23-7.26(1H, m), 7.88(1H, d, J=8.8 Hz), 7.98(2H, d, J=8.8 Hz), 8.13(2H, d, J=8.8 Hz), 9.57(1H, br.s), 10.11(1H, br.s)
IR(KBr)cm-1 : 3326, 3251, 1707, 1696, 1659, 1603, 1165

(96-2) A suspension of 3.0 g of the compound from the process (96-1) (8.82 mmol) and 4.5 g of ethoxycarbonylmethyl triphenylphosphine (12.9 mmol) in 10 ml of toluene was stirred in a stream of nitrogen at 80°C for 5.5 hours. After cooling, the mixture was diluted with ethyl acetate; washed with saturated sodium bicarbonate, water and saturated brine; dried; and evaporated. The residue was purified by column chromatography on silica gel (eluent:chloroform:ethyl acetate=20:1) to give 3.3 g of ethyl 4-[N-[2-(N-tertbutoxycarbonyl)aminophenyl]aminocarbonyl]cinnamate (Yield: 91.1%) as a yellow amorphous solid.
¹H NMR(270 MHz, CDCI. sub. 3) .delta. ppm: 1.35(3H, t, J=7.3 Hz), 1.52(9H, s), 4.28(2H, q, J=7.3 Hz), 6.52(1H, d, J=15.1 Hz), 6.80(1H, br.s), 7.16-7.25(3H, m), 7.61 (2H, d, J=8.1 Hz), 7.71(1H, d, J=15.1 Hz), 7.82(1H, d, 7.3 Hz), 7.98(2H, d, J=8.1 Hz), 9.34(1H, br.s)

(96-3) To a solution of 2.50 g of the compound from the process (96-2) (6.09 mmol) in 30 ml of THF and 40 ml of methanol was added 10% Pd/C (wet, 0.5 g) in a stream of nitrogen, and then stirred in a stream of hydrogen for 30 min. After filling with nitrogen, the mixture was filtered to remove the catalyst, and the filtrate was evaporated. To the residue was added diisopropyl ether, and the precipitated solid was collected by filtration and dried to give 2.23. g of N-[2-(N-tert-butoxycarbonyl)aminophenyl]-4-(2-ethoxycarbonylethyl)benzamide (Yield: 88.8%) as a white solid.
¹ H NMR(270 MHz, CDCl.sub.3) .delta. ppm: 1.25(3H, t, J=7.3 Hz), 1.52(9H, s), 2.65(2H, t, J=7.3 Hz), 3.02(2H, t, J=7.3 Hz), 4.13(2H, q, J=7.3 Hz), 6.77(1H, br.s), 7.16-7.33(5H, m), 7.78(1H, d, J=8.1 Hz), 7.89(2H, d, J=8.8 Hz), 9.06(1H, br.s)

(96-4) To a suspension of 2.21 g of the compound from the process (96-3) (5.36 mmol) in 10 ml of methanol and 15 ml of water was added 0.37 g of lithium hydroxide monohydrate (8.82 mmol), and the mixture was stirred at 40°C for 3 hours. After cooling, to the mixture was added 10% hydrochloric acid and the mixture was extracted with ethyl acetate. The organic layer was washed with saturated brine, dried and evaporated. To the residue was added diisopropyl ether, and the precipitated solid was filtered and dried to give 1.87 g of N-[2-(N-tert-butoxycarbonyl)aminophenyl]-4-(2-carboxyethyl)benzamide (Yield: 90.8%) as a white solid.
¹ H NMR(270 MHz, DMSO-d6) .delta. ppm: 1.45(9H, s), 2.59(2H, t, J=7.3 Hz), 2.91(2H, t, J=7.3 Hz), 7.13-7.20(2H, m), 7.40(2H, d, J=8.1 Hz), 7.54(2H, dd, J=7.3, 2.1 Hz), 7.88(2H, d, J=8.1 Hz), 8.66(1H, br.s), 9.79(1H, br.s)

(96-5) To a suspension of 0.12 g of the compound from the process (96-4) (0.3 mmol) in 5 ml of benzene were added 0.1 ml of triethylamine (0.7 mmol) and 0.3 g of molecular sieves 4A, and the mixture was stirred in a stream of nitrogen for 0.5 hours. To the mixture was added 0.15 ml of diphenylphosphoryl azide (0.7 mmol), and the mixture was refluxed with heating for 2 hours. After cooling, to the mixture was added 0.4 ml of benzyl alcohol (3.8 mmol), and the mixture was refluxed with heating for additional 2.5 hours. After diluting with ethyl acetate, the reaction mixture was washed with water and saturated brine.

The organic layer was dried and evaporated. The residue was purified by column chromatography on silica gel (eluent:chloroform:ethyl acetate=4:1) to give 129 mg of N-[2-(N-tert-butoxycarbonyl)aminophenyl]-4-[2-(N-benzyloxycarbonylamino)et hyl]benzamide (Yield: 88%) as a clear oil.
¹H NMR(270 MHz, CDCl.sub.3) .delta. ppm: 1.51(9H, s), 2.89(2H, t, J=7.3 Hz), 3.45-3.54(2H, m), 4.80(1H, m), 5.10(2H, s), 6.76(1H, br.s), 7.20-7.38(10H, m), 7.79(1H, d, J=8.8 Hz), 7.89(2H, d, J=8.1 Hz), 9.10(1H, br.s)

(96-6) To a solution of 129 mg of the compound from the process (96-5) (0.26 mmol) in 10 ml of methanol was added 10% Pd/C (wet, 0.05 g) in a stream of nitrogen, and then stirred in a hydrogen stream for 2 hours. After removing the catalyst, the filtrate was evaporated and dried. The residue was dissolved in 5 ml of dichloromethane. To the solution were added 0.18 g of 3-pyridineacetic acid hydrochloride (1.04 mmol) and then 0.28 g of triethylamine (2.0 mmol), and the mixture was ice-cooled. Under ice-cooling, to the mixture was added 0.17 g of 2-chloro-1,3-dimethylimidazolinium chloride (1.0 mmol), and the mixture was stirred for 2 hours. To the mixture was added saturated sodium bicarbonate aq., and the mixture was extracted with chloroform. The organic layer was washed with saturated brine, dried and evaporated. The residue was purified by column chromatography on silica gel (eluent:ethyl acetate:methanol=10:1) to give 50 mg of N-[2-(N-tert-butoxycarbonyl)aminophenyl]-4-[2-[N-(pyridin-3-yl)acetylamino]ethyl]benzamide (Yield: 40%) as a colorless oil.
¹ H NMR(270 MHz, CDCI.sub.3) .delta. ppm: 1.48(9H, s), 2.80(2H, t, J=6.6 Hz), 3.42(2H, m), 3.52(2H, s), 6.33(1H, t-like, J=5.9 Hz), 7.09(2H, d, J=8.1 Hz), 7.14-7.20(2H, m), 7.24(1H, dd, J=4.4, 7.3 Hz), 7.41(1H, dd, J=3.7, 5.9 Hz), 7.50(1H, s), 7.58(1H, dd, J=1.5, 5.9 Hz), 7.69(1H, dd, J=3.7, 5.9 Hz), 7.75(2H, d, J=8.1 Hz), 8.22(1H, d, J=2.1 Hz), 8.44(1H, dd, J=1.5, 4.4 Hz), 9.49(1H, br.s)

(96-7) To a solution of 50 mg of the compound from the process (96-6) (0.10 mmol) in 2 ml of dioxane and 1 ml of methanol was added 2 ml of 4N hydrochloric acid-dioxane, and the mixture was stirred at room temperature for 2.5 hours. To the mixture was added saturated sodium bicarbonate, and the mixture was extracted with ethyl acetate. The organic layer was washed with saturated brine, dried and evaporated. The residue was dried to give 22 mg of N-(2-aminophenyl)-4-[2-[N-(pyridin-3-yl)acetylamino]ethyl]benzamide (Yield: 59%) as an amorphous solid.
mp: (amorphous)
¹ H NMR(270 MHz, DMSO-d6) .delta. ppm: 2.70-2.90(4H, m) 3.42(2H, s), 4.89(2H, br.s), 6.60(1H, dd, J=7.3, 7.3 Hz), 6.78(1H, d, J=7.3 Hz), 6.97(1H, dd, J=7.3, 7.3 Hz), 7.16(1H, d, J=7.3 Hz.), 7.29-7.32(3H, m), 7.59(1H, d, J=8.1 Hz), 7.89(1H, d, J=8.1 Hz), 8.22(1H, t-like), 8.41-8.43(2H, m), 9.62(1H, br.s)

### Example 97

### Preparation of N-(2-aminophenyl)-4-[2-[N-(3-picolyl)aminocarbonyl]ethyl]benzamide (Table 1: Compound 80)

(97-1) To a suspension of 0.58 g of the compound from Example 96, the process (96-4) (1.5 mmol) in 5 ml of dichloromethane were added 0.22 g of 3-picolylamine (2.0 mmol) and 0.56 ml of triethylamine (4.0 mmol). Under ice-cooling, to the mixture was added 0.39 g of 2-chloro-1,3-dimethylimidazolinium chloride (2.0 mmol) in 5 ml of dichloromethane, and the mixture was stirred for 1.5 hours. To the mixture was added saturated sodium bicarbonate aq., and the mixture was extracted with chloroform.

The organic layer was washed with water and saturated brine, dried and evaporated. The residue was purified by column chromatography on silica gel (eluent:chloroform:methanol:NH.sub.3 aq.=100:10:1) to give 0.71 g of N-[2-(N-tert-butoxycarbonyl)aminophenyl]-4-[2-[N-3-picolyl)aminocarbonyl]e thyl]benzamide (Yield: 94%) as a light brown oil.
¹ H NMR(270 MHz, CDCl.sub.3) .delta. ppm: 1.45(9H, s), 2.42(2H, t, J=7.3 Hz), 2.98(2H, t, J=7.3 Hz), 4.32(2H, d, J=6.6 Hz), 6.44(1H, t, J=6.6 Hz), 7.14-7.27(5H, m), 7.48-7.57 (3H, m), 7.63-7.68(3H, m), 7.90(1H, d, J=2.1 Hz), 8.43(1H, dd, J=1.4, 4.4 Hz), 9.86(1H, br.s)

(97-2) To a solution of 0.70 g of the compound from the process (97-1) (1.47 mmol) in 5 ml of dioxane was added 5 ml of 4N hydrochloride-dioxane and then 2 ml of methanol, and the mixture was stirred at room temperature for 2 hours. To the mixture was added saturated sodium bicarbonate aq., and the mixture was extracted with ethyl acetate. The organic layer was washed with saturated brine, dried and evaporated. To the residue was added diisopropyl ether, and the precipitated solid was collected by filtration and dried to give 0.42 g of N-(2-aminophenyl)-4-[2-[N-(3-picolyl)aminocarbonyl]ethyl]benzamide (Yield: 76.3%) as an opalescent solid.
mp: 168-170°C
¹ H NMR(270 MHz, DMSO-d6) .delta. ppm: 2.47-2.53(2H, m), 2.93(2H, t, J=7.3 Hz), 4.27(2H, d, J=5.9 Hz), 4.90(2H, br.s), 6.60(1H, dd, J=7.3, 7.3 Hz), 6.78(1H, d, J=8.1 Hz), 6.97(1H, dd, J=6.6, 7.3 Hz), 7.16(1H, d, J=6.6 Hz), 7.28-7.35(1H, m), 7.33(2H, d, J=8.1 Hz), 7.49(1H, dd, J=2.1, 5.9 Hz), 7.89(2H, d, J=8.1 Hz), 8.39-8.44(3H, m), 9.62(1H, br.s)
IR(KBr)cm-1 : 3313, 1641, 1523, 1457, 1300, 748, 713

### Example 98

### Preparation of N-(2-aminophenyl)-4-[(pyridin-3-yl)methylaminocarbonyloxymethyl]benzamide (Table 1: Compound 85)

(98-1) To a solution of 1.99 g of methyl 4-hydroxymethylbenzoate (12.0 mmol) in 20 ml of THF were added 1.78 g of N,N'-carbonyldiimidazole (11.0 mmol) at room temperature, and the solution was stirred for an hour. To the solution were added 1.08 g of 3-picolylamine (10.0 mmol) at room temperature, and the mixture was stirred for 3.5 hours and left overnight. Water was added to the solution, and the mixture was extracted with ethyl acetate.

The organic layer was washed with saturated brine, dried and evaporated. The residue was purified by column chromatography on silica gel (eluent: ethyl acetate) to give 2.76 g of N-(4-methoxycarbonyl)benzyloxycarbonyl-3-picolylamine (Yield: 91.9%) as a white waxy solid.
¹H NMR(270 MHz, CDCl.sub.3) .delta. ppm: 3.91(3H, s), 4.40(2H, d, J=5.9 Hz), 5.18(2H, s), 5.50(1H, br.s), 7.24-7.28(1H, m), 7.40(2H, d, J=8.1 Hz), 7.65(1H, d, J=7.3 Hz), 8.02(2H, d, J=8.8 Hz), 8.50-8.53(2H, m)

(98-2) To a suspension of 2.40 g of the compound from the process (98-1) (8.0 mmol) in 10 ml of methanol and 20 ml of water was added 0.42 g of lithium hydroxide monohydrate (10.0 mmol), and the mixture was stirred at room temperature for 5 hours. To the reaction mixture was added 10% hydrochloric acid to acidified to pH 2to 4, and the precipitated solid was collected by filtration and dried to give 1.83 g of N-(4-carboxy)benzyloxycarbonyl-3-picolylamine (79.9%) as a white solid.
¹H NMR(270 MHz, DMSO-d6) .delta. ppm: 4.24(2H, d, J=5.9 Hz), 5.13(2H, s), 7.33-7.38(1H, m), 7.46(2H, d, J=8.1 Hz), 7.94(2H, d, J=8.1 Hz), 7.95-8.01 (1H, m), 8.46(1H, d, J=5.1 Hz), 8.49(1H, d, J=1.5 Hz), 13.0(1H, br.s)

(98-3) To a suspension of 1.26 g of the compound from the process (98-2) (4.4 mmol) in 20 ml of dichloromethane were slowly added 1.0 ml of oxalyl chloride (11.4 mmol) and then several drops of DMF. The reaction mixture was stirred at room temperature for 10 min. and at 40°C for additional 30 min. After cooling, the mixture was evaporated and the excess oxalyl chloride was removed by evaporation with toluene. To the residue was added 10 ml of dichloromethane. Under ice-cooling, to the mixture was added dropwise a solution of 0.83 g of the compound from Example 1, the process (1-2) (4.0 mmol) in 8 ml of dichloromethane and 8 ml of pyridine, and the solution was warmed to room temperature with stirring for 7 hours and left overnight.

To the mixture was added saturated sodium bicarbonate, and the mixture was extracted with chloroform. The organic layer was washed with saturated brine, dried and evaporated. Toluene was added to the residue to azeotropically remove the excess pyridine. The residue was purified by column chromatography on silica gel (eluent: ethyl acetate) to give 1.40 g of N-[2-(N-tert-butoxycarbonyl)aminophenyl]-4-[(pyridin-3-yl)methylaminocarbo nyloxymethyl]benzamide (Yield: 73.4%) as a light brown solid.
¹H NMR(270 MHz, CDCI.sub.3) .delta. ppm: 1.51(9H, H), 4.40(2H, d, J=5.9 Hz), 5.19(2H, s), 5.56(1H, m), 7.07(1H, br.s), 7.14-7.31(4H, m), 7.43(2H, d, J=8.1 Hz), 7.65(1H, d, J=8.1 Hz), 7.76(1H, d, J=7.3 Hz), 7.95(2H, d, J=8.1 Hz), 8.52(2H, d, J=4.1 Hz), 9.32(1H,br.s)

(98-4) To a solution of 1.00 g of the compound from the process (98-3) (2.10 mmol) in 10 ml of dioxane and 2 ml of methanol was added 9 ml of 4N hydrochloric acid-dioxane at room temperature, and the mixture was stirred for 2 hours. To the mixture was added saturated sodium bicarbonate and the mixture was extracted with ethyl acetate-methyl ethyl ketone (1:1). The organic layer was washed with saturated brine, dried and evaporated. To the residue was added methanol-diisopropyl ether, and the precipitated solid was collected by filtration and dried to give 0.79 g of N-(2-aminophenyl)-4-[(pyridin-3-yl)methylaminocarbonyloxymethyl]benzamide (Yield: quantitative) as a white solid.
mp: 139-141°C
¹ H NMR(270 MHz, DMSO-d6) .delta. ppm: 4.25(2H, d, J=5.9 Hz), 4.90(2H, s), 5.13(2H, s), 6.60(1H, dd, J=6.6, 7.3 Hz), 6.78(1H, d, J=7.3 Hz), 6.97(1H, dd, J=6.6, 7.3 Hz), 7.17(1H, d, J=7.3 Hz), 7.36(1H, dd, J=4.4, 8.1 Hz), 7.47(2H, d, J=8.1 Hz), 7.67(1H, d, J=8.1 Hz), 7.97(2H, d, J=7.3 Hz), 7.90-8.00(1H, m), 8.46(1H, dd, J=1.5, 5.1 Hz), 8.49(1H, d, J=2.1 Hz), 9.65(1H, br.s)
IR(KBr)cm-1 : 3326(br.), 1694, 1637, 1526, 1458, 1147, 750, 712

### Example 99

### Preparation of N-(2-aminophenyl)-4-[3-(imidazol-1-yl)propylaminocarbonyloxymethyl]benzami de (Table 1: Compound 215)

The title compound was prepared as described in Example 98.
mp: (amorphous)
¹H NMR(270 MHz, DMSO-d6) .delta. ppm: 1.80-1.89(2H, m), 2.94-3.02(2H, m), 3.98(2H, t, J=7.3 Hz), 4.88(2H, s), 5.11(2H, s), 6.55-6.63(1H, m), 6.76-6.97(3H, m), 7.10-7.18(2H, m), 7.43-7.48(3H, m), 7.61(1H, s), 7.98(2H, d, J=8.1 Hz), 9.66(1H, s)

### Example 100

### Preparation of N-(2-aminophenyl)-4-(phenylacetylamino)benzamide (Table 1: Compound 2)

(100-1) To a solution of 16.6 g of the compound from Example 1, the process (1-2) (80 mmol) in 120 ml of dichloromethane was added 16.8 ml of triethylamine (120 mmol) and then, was slowly added a solution of 16.0 g of 4-nitrobenzoyl chloride (86.4 mmol) in 40 ml of dichloromethane, and the solution was stirred for 7 hours. To the solution was added saturated sodium bicarbonate aq., and the mixture was extracted with chloroform.

The organic layer was washed with 1N hydrochloric acid, saturated sodium bicarbonate and saturated brine; dried; and evaporated. The residue was washed with diisopropyl ether to give 28.0 g of N-[2-(N-tert-butoxycarbonylamino)phenyl]-4-nitrobenzamide (Yield: 98%) as a light yellow solid.
¹ H NMR(270 MHz, CDCl.sub.3) .delta. ppm: 1.53(9H, s), 7.17-7.29(4H, m), 7.85(1H, br.d, J=7.3 Hz). 8.17(2H, d, J=8.8 Hz), 8.32(2H, d, J=8.8 Hz), 9.88(1H, br.s)

(100-2) To a solution of 24.0 g of the compound from the process (100-1) (67.2 mmol) in 80 ml of THF and 80 ml of methanol was added 2.4 g of 10% Pd/C (wet) in a stream of nitrogen, and the mixture was stirred in a stream of hydrogen for 1.5 hours. After cease of absorption of hydrogen, the catalyst was removed by filtration and the filtrate was evaporated. To the residue were added diisopropyl ether and ethyl acetate, and the precipitated solid was collected by filtration and dried to give 18.96 g of N-[2-(N-tert-butoxycarbonylamino)phenyl]-4-aminobenzamide (Yield: 86%) as a white solid.
¹ H NMR(270 MHz, DMSO-d6).delta. ppm: 1.46(9H, s), 5.84(2H, s), 6.61(2H, d, J=8.8 Hz), 7.10-7.18(2H, m), 7.46-7.55(2H, m), 7.68(2H, d, J=8.8 Hz), 8.67(1H, s), 9.49(1H, s)

(100-3) To a solution of 1.6 g of the compound from the process (100-2) (4.88 mmol) in 15 ml of dichloromethane were added 0.8 ml of pyridine (9.9 mmol) and 0.96 ml of phenylacetyl chloride (7.26 mmol), and the solution was stirred for one day. After completion of the reaction, water was added and the precipitated crystals were collected by filtration to give 1.66 g of N-[2-(N-tert-butoxycarbonylamino)phenyl]-4-(phenylacetylamino)benzamide (Yield: 76%).

(100-4) To a solution of 1 g of the compound from the process (100-3) (2.24 mmol) in 25 ml of acetonitrile was added 0.88 ml of iodotrimethylsilane (6.18 mmol) at room temperature, and the solution was stirred for 3 hours. After completion of the reaction, the solution was concentrated. The residue was recrystallized from methanol to give 0.29 g of N-(2-aminophenyl)-4-(phenylacetylamino)benzamide (Yield: 38%) as white crystals.
mp: 232-237°C
¹H NMR(270 MHz, DMSO-d6) .delta. ppm: 3.69(2H, s), 4.90(2H, s). 6.60(1H, t, J=7.3 Hz), 6.77(1H, d, J=7.3 Hz), 6.96(1H, t, J=7.3 Hz), 7.15(1H, d, J=7.4 Hz), 7.22-7.35(5H, m), 7.72(2H, d, J=8.8 Hz), 7.95(2H, d, J=8.8 Hz), 9.57(1H, s), 10.43(1H, s)
IR(KBr)cm-1 : 2937, 2764, 1660, 1598, 1506, 1459

As described in Example 100, the compounds of Examples 101 to 128 were prepared, each of whose melting point (mp), 1 H NMR data and/or IR data are shown below.

### Example 101

### N-(2-aminophenyl)-4-[(4-phenylbutanoyl)amino]benzamide (Table 1: Compound 4)

mp: (amorphous)
¹ H NMR(270 MHz, DMSO-d6).delta. ppm: 1.91(2H, hep, J=7.3 Hz), 2.37(2H, t, J=7.3 Hz), 2.64(2H, t, J=7.3 Hz), 5.0(2H, br.s), 6.61(1H, t, 7.0 Hz), 6.79(1H, dd, J=1.5, 8.1 Hz), 6.97(1H, t, J=7.0 Hz), 7.10-7.40(6H, m), 7.71(2H, d, J=8.8 Hz), 7.94(2H, d, J=8.8 Hz), 9.57(1H, s), 10.15(1H, s)
IR(KBr)cm-1 ; 3344, 1687, 1603, 1542, 1460, 1315, 1033, 842, 737

### Example 102

### N-(2-aminophenyl)-4-[(4-chlorophenylacetyl)amino]benzamide (Table 1: Compound 15)

mp: (amorphous)
¹ H NMR(270 MHz, DMSO-d6) .delta. ppm: 3.72(2H, s), 7.29-7.43(8H, m), 7.77(2H, d, J=8.8 Hz), 8.00(2H, d, J=8.8 Hz), 10.29(1H, s), 10.52(1H, s)
IR(KBr)cm-1 : 3300, 2868, 1664, 1638, 1520

### Example 103

### N-(2-aminophenyl)-4-[(2-nitrophenylacetyl)amino]benzamide hydrochloride (Table 1: hydrochloride of Compound 19)

mp: (amorphous)
¹ H NMR(270 MHz, DMSO-d6) .delta. ppm: 4.20(2H, s), 7.20-7.30(3H, m), 7.40-7.45(1H, m), 7.60(2H, d), 7.71-7.77(3H, m), 8.02-8.10(4H, m), 10.27(1H, br.s), 10.64(1H,br.s)
IR(KBr)cm-1 : 3263, 1676, 1647, 1518, 1184, 759

### Example 104

### N-(2-aminophenyl)-4-[(4-nitrophenylacetyl)amino]benzamide (Table 1: Compound 21)

mp: 222-226°C
¹ H NMR(270 MHz, DMSO-d6) .delta. ppm: 3.90(2H, s), 4.96(2H, br.s), 6.60(1H, dt, J=1.5, 6.6 Hz), 6.78(1H, dd, J=1.5, 6.6 Hz), 6.97(1H, dt, J=1.5, 6.6 Hz), 7.15(1H, dd, J=1.5, 6.6 Hz), 7.63(2H, d, J=8.8 Hz), 7.71(2H, d, J=8.8 Hz), 7.95(2H, d, J=8.8 Hz), 8.22(2H, d, J=8.8 Hz), 9.59(1H, s), 10.54(1H, s).
IR(KBr)cm-1 : 3395, 3334, 1671, 1630, 1519, 1346

### Example 105

### N-(2-aminophenyl)-4-[(2-aminophenylacetyl)amino]benzamide (Table 1: Compound 22)

mp: 177-182°C(dec.)
¹ H NMR(270 MHz, DMSO-d6) .delta. ppm: 3.54(2H, s), 4.88(2H, br.s), 5.09(2H, br.s), 6.55(1H, dd, J=6.6, 7.3 Hz), 6.59(1H, dd, J=7.3, 7.3 Hz), 6.68(1H, d, J=7.3 Hz), 6.78(1H, d, J=7.3 Hz), 6.96(2H, dd, J=7.3, 7.3 Hz), 7.06(1H, d, J=6.6 Hz), 7.15(1H, d, J=7.3 Hz), 7.71(2H, d, J=8.8 Hz), 7.95(2H, d, J=8.8 Hz), 9.57(1H, br.s), 10.39(1H, br.s)
IR(KBr)cm-1 : 3374, 3256(br.), 1683, 1597, 1503, 1317, 1262, 1180, 1153, 747

### Example 106

### N-(2-aminophenyl)-4-[(4-aminophenylacetyl)amino]benzamide (Table 1: Compound 26)

mp: 219-226°C(dec.)
¹ H NMR(270 MHz, DMSO-d6) .delta. ppm: 3.46(2H, s), 4.93(4H, br.s), 6.52(2H, d, J=8.1 Hz), 6.59(1H, dt, J=1.5, 7.3 Hz), 6.77(1H, dd, J=1.4, 7.3 Hz), 6.97(1H, dt, J=1.4, 7.3 Hz), 6.99(2H, d, J=8.1 Hz), 7.15(1H, dd, J=1.5, 7.3 Hz), 7.70(2H, d, J=8.8 Hz), 7.93(2H, d, J=8.8 Hz)
IR(KBr)cm-1 : 3278, 3032, 1675, 1628, 1516

### Example 107

### N-(2-aminophenyl)-4-[(4-methoxyphenylacetyl)amino]benzamide (Table 1: Compound 32)

mp: (amouphous)
¹ H NMR(270 MHz, DMSO-d6) .delta. ppm: 3.62(2H, s), 3.74(3H, s), 6.90(2H, d, J=8.8 Hz), 7.26(2H, d, J=8.8 Hz), 7.30(3H, m), 7.39(1H, m), 7.77(2H, d, J=8.8 Hz), 7.99(2H, d, J=8.8 Hz), 10.26(1H, s), 10.44(1H, s)
IR(KBr)cm-1 : 3300, 2759, 1670, 1638, 1514, 1250

### Example 108

### N-(2-aminophenyl)-4-[[4-(N,N-dimethylamino)phenylacetyl]amino]benzamide (Table 1: Compound 53)

mp: 140°C
¹ H NMR(270 MHz, DMSO-d6) .delta. ppm: 3.04(6H, s), 3.67(2H, s), 7.16(2H, d, J=8.0 Hz), 7.29-7.40(6H, m), 7.76(2H, d, J=8.8 Hz), 7.99(2H, d, J=8.8 Hz), 10.29(1H, s), 10.47(1H, s)
IR(KBr)cm-1 : 3244, 2951, 2639, 1647, 1599, 1507

### Example 109

### N-(2-aminophenyl)-4-[(4-trifluoromethylphenylacetyl)amino]benzamide (Table 1: Compound 43)

mp: (amorphous)
¹H NMR(270 MHz, DMSO-d6) .delta. ppm: 3.84(2H, s), 6.89(1H, t, J=7.4 Hz), 7.00(1H, d, J=7.4 Hz), 7.11(1H, t, J=7.4 Hz), 7.25(1H, d, J=7.4 Hz), 7.57(2H, d, J=8.8 Hz), 7.71(2H, d, J=8.8 Hz), 7.73(2H, d, J=8.8 Hz), 7.97(2H, d, J=8.8 Hz), 9.87(1H, s), 10.54(1H, s)
IR(KBr)cm-1 : 3260, 1664, 1605, 1521, 1327, 1119

### Example 110

### N-(2-aminophenyl)-4-[(pyridin-2-yl)acetylamino]benzamide dihydrochloride(Table 1: hydrochloride of Compound 174)

mp: (amorphous)
¹ H NMR(270 MHz, DMSO-d6) .delta. ppm: 4.60(2H, s), 7.30-7.46(3H, m), 7.56(1H, d, J=7.4 Hz), 7.79(2H, d, J=8.8 Hz), 7.95(1H, t, J=6.6 Hz), 8.01(1H, d, J=7.4 Hz), 8.11(2H, d, J=8.8 Hz), 8.49(1H, t, J=7.4 Hz), 8.87(1H, d, J=5.1 Hz), 10.46(1H, s)

### Example 111

### N-(2-aminophenyl)-4-[(pyridin-3-yl)acetylamino]benzamide dihydrochloride(Table 1: hydrochloride of Compound 68)

mp: 182-189°C(dec.)
¹ H NMR(270 MHz, DMSO-d6) .delta. ppm: 4.12(2H, s), 7.29-7.59(4H, m), 7.80(2H, d, J=8.8 Hz), 8.05(1H, m), 8.11(2H, d, J=8.8 Hz), 8.57(1H, d, J=8.1 Hz), 8.85(1H, d, J=5.2 Hz), 8.95(1H, s), 10.25(1H, s), 10.48(1H, s)

### Example 112

### N-(2-aminophenyl)-4-[[3-(pyridin-3-yl)propanoyl]amino]benzamide (Table 1: Compound 69)

mp: 184-186°C
¹ H NMR(270 MHz, DMSO-d6) .delta. ppm: 2.80(2H, t, J=7.3 Hz), 3.08(2H, t, J=7.3 Hz), 6.87(1H, t, J=8.0 Hz), 6.99(1H, dd, J=1.4, 8.0 Hz), 7.11(1H, dt, J=1.4, 8.0 Hz), 7.25(1H, d, J=8.0 Hz), 7.70(2H, d, J=8.8 Hz), 7.77(1H, dd, J=5.8, 8.0 Hz), 7.96(2H, d, J=8.8 Hz), 8.22(1H, d, J=8.0 Hz), 8.75(1H, d, J=1.4 Hz), 9.83(1H, s), 10.25(1H, s)

### Example 113

### N-(2-aminophenyl)-2-chloro-4-[3-(pyridin-3-yl)propanoylamino]benzamide (Table 1: Compound 123)

mp: (amorphous)
¹ H NMR(270 MHz, DMSO-d6) .delta. ppm: 2.70(2H, t, J=8.1 Hz), 2.96(2H, t, J=7.3 Hz), 4.74(2H, br.s), 6.60(1H, t, J=6.6 Hz), 6.78(1H, d, J=6.6 Hz), 6.95(1H, t, J=6.6 Hz), 7.19(1H, dd, J=1.5, 7.3 Hz), 7.29(1H, dd, J=5.1, 7.3 Hz), 7.66(2H, d, J=8.8 Hz), 7.92(2H, d, J=8.8 Hz), 8.48(1H, d, J=2.2 Hz), 9.37(1H, s), 10.00(1H, s)
IR(KBr)cm-1 : 3273, 1675, 1519, 1315, 1181, 852, 747

### Example 114

### N-(2-aminophenyl)-4-[[N-(pyridin-3-yl)methyl-N-trifluoroacetylamino]acetyla mino]benzamide (Table 1: Compound 107)

mp: 145°C(dec.)
¹ H NMR(270 MHz, DMSO-d6) .delta. ppm: 4.18 and 4.42(total 2H, s), 4.73 and 4.83(total 2H, s), 4.87(2H, br.s), 6.60(1H, dd, J=7.3, 8.1 Hz), 6.78(1H, d, J=8.1 Hz), 6.96(1H, dd, J=7.3, 7.3 Hz), 7.16(1H, d, J=8.1 Hz), 7.35-7.45(1H, m), 7.66(2H, d, J=5.9 Hz), 7.70-7.80(1H, m), 7.90-8.00(2H, m), 8.51-8.55(1H, m), 8.58(1H, s), 9.60(1H, br.s), 10.36 and 10.43(total 1H, br.s)

### Example 115

### N-(2-aminophenyl)-4-[[N-(pyridin-3-yl)methylamino]acetylamino]benzamide (Table 1: Compound 105)

mp: 160°C(dec.)
¹ H NMR(270 MHz, DMSO-d6) .delta. ppm: 3.30(2H, s), 3.79(2H, s), 4.88(2H, s), 6.60(1H, dd, J=7.3, 7.3 Hz), 6.78(1H, d, J=8.1 Hz), 6.97(1H, dd, J=7.3, 8.1 Hz), 7.16(1H, d, J=8.1 Hz), 7.74(2H, d, J=8.8 Hz), 7.80(1H, d, J=7.3 Hz), 7.95(2H, d, J=8.1 Hz), 8.46(1H, d, J=3.7 Hz), 8.57(1H, s), 9.57(1H, s), 10.08(1H, br.s)
IR(KBr)cm-1 : 3298, 1693, 1637, 1602, 1544, 1454, 1262, 848, 762

### Example 116

### N-(2-aminophenyl)-4-[N-(pyridin-3-yl)methyloxamoylamino]benzamide (Table 1: Compound 104)

mp: (amorphous)
¹ H NMR(270 MHz, DMSO-d6) .delta. ppm: 4.43(2H, d, J=6.6 Hz), 4.90(2H, br.s), 6.60(1H, dd, J=6.6, 7.3 Hz), 6.78(1H, d, J=7.3 Hz), 6.97(1H, ddd, J=1.5, 6.6, 7.3 Hz), 7.16(1H, d, J=7.3 Hz), 7.37(1H, dd, J=4.4, 8.1 Hz), 7.73(1H, d, J=8.1 Hz), 7.96 and 7.96(4H, AA'BB', J=9.4 Hz), 8.47(1H, dd, J=1.5, 5.1 Hz), 8.56(1H, d, J=1.5 Hz), 9.59(1H, s), 9.67(1H, t, J=6.6 Hz), 10.92(1H, br.s)
IR(KBr)cm-1 : 3299, 1644, 1518, 1320, 1119, 748

### Example 117

### N-(2-aminophenyl)-4-[[N-(pyridin-3-yl)methyl-N-nicotinoylamino]acetylamino] benzamide (Table 1: Compound 106)

mp: (amorphous)
¹ H NMR(270 MHz, DMSO-d6) .delta. ppm: 4.11(major 2H, s), 4.26(minor 2H, s), 4.75(major 2H, s), 4.65(minor 2H, s), 4.88(total 2H, br.s), 6.60(total 1H, dd, J=7.3, 8.1 Hz), 6.78(total 1H, d, J=7.3 Hz), 6.97(total 1H, dd, J=7.3, 8.1 Hz), 7.15(total 1H, d, J=8.1 Hz), 7.41-7.95(total 8H, m), 8.46-8.52(total 1H, m), 8.63-8.70(total 2H, m), 9.59(total 1H, s), 10.22(major 1H, br.s), 10.37(minor 1H, br.s)
IR(KBr)cm-1 : 3269, 1701, 1637, 1603, 1534, 1506, 1312, 1254, 752

### Example 118

### N-(2-aminophenyl)-4-[[4-(pyridin-3-yl)butanoyl]amino]benzamide (Table 1: Compound 70)

mp: 165-167°C(dec.)
¹ H NMR(270 MHz, DMSO-d6) .delta. ppm: 1.88-1.99(2H, m), 2.68(2H, t, J=7.3 Hz), 2.39(2H, t, J=7.3 Hz), 6.78-6.81(1H, m), 6.94-6.99(1H, m), 7.15-7.18(1H, m), 7.34-7.39(1H, m), 7.69-7.72(3H, m), 7.94(2H, d, J=8.8 Hz), 8.43-8.48(2H, m)
IR(KBr)cm-1 : 3291, 1660, 1626, 1308, 1261, 1182, 1027, 825, 747

### Example 119

### N-(2-aminophenyl)-4-[[N-(pyridin-3-yl)methyl-N-methylamino]acetylamino]benz amide (Table 1: Compound 108)

mp: 154-155°C
¹ H NMR(270 MHz, DMSO-d6) .delta. ppm: 2.28(3H, s), 3.27(2H, s), 3.71(2H, s), 4.88(2H, br.s), 6.60(1H, dd, J=6.6, 7.3 Hz), 6.78(1H, d, J=8.1 Hz), 6.97(1H, dd, J=7.3, 8.1 Hz), 7.16(1H, d, J=8.1 Hz), 7.38(1H, dd, J=2.9, 8.1 Hz), 7.77(2H, d, J=8.8 Hz), 7.75-7.85(1H, m), 7.95(2H, d, J=8.8 Hz), 8.47(1H, d, J=1.5 Hz), 8.49(1H, s), 9.56(1H, s), 10.62(1H, br.s)

### Example 120

### N-(2-aminophenyl)-4-[N-(pyridin-3-yl)oxyacetylamino]benzamide (Table 1: Compound 65)

mp: 175-179°C
¹ H NMR(270 MHz, DMSO-d6) .delta. ppm: 4.86(2H, s), 4.90(2H, br.s), 6.60(1H, d, J=7.3, 7.3 Hz), 6.78(1H, d, J=7.3 Hz), 6.97(1H, dd, J=6.6, 7.3 Hz), 7.16(1H, d, J=8.1 Hz), 7.34-7.47(2H, m), 7.76(2H, d, J=8.8 Hz), 7.98(2H, d, J=8.8 Hz), 8.22(1H, d, J=3.6 Hz), 8.39(1H, d, J=2.9 Hz), 9.60(1H, br.s), 10.40(1H, br.s)
IR(KBr)cm-1 : 3321, 1655, 1530, 1276, 1231, 1068,

### Example 121

### N-(2-aminophenyl)-4-[4-(pyridin-3-yl)-1,4-dioxobutylamino]benzamide (Table 1: Compound 99)

mp: 190-194°C
¹ H NMR(270 MHz, DMSO-d6) .delta. ppm: 2.08(2H, t, J=6.4 Hz), 3.41(2H, t, J=6.4 Hz), 4.86(2H, s), 6.59(1H, t, J=5.6 Hz), 6.78(1H, d, J=7.9 Hz), 6.96(1H, t, J=7.4 Hz), 7.15(1H, d, J=7 Hz), 7.58(1H, dd, J=4.9, 7.9 Hz), 7.70(2H, d, J=8.9 Hz), 7.94(2H, d, J=8.9 Hz), 8.35(1H, d, J=7.9 Hz), 8.81(1H, d, J=4 Hz), 9.18(1H, s), 9.56(1H, s), 10.32(1H, s)
IR(KBr)cm-1 : 3317, 1691, 1652, 1601, 1522, 1312, 982, 847, 764, 701

### Example 122

### N-(2-aminophenyl)-4-[3-[N-(pyridin-3-yl)amino]-1,3-dioxopropylamino]benzami de (Table 1: Compound 94)

mp: 196°C(dec.)
¹ H NMR(270 MHz, DMSO-d6) .delta. ppm: 3.57(2H, s), 4.87(2H, s), 6.57-6.62(1H, m), 6.76-6.79(1H, m), 6.94-6.99(1H, m), 7.14-7.17(1H, m), 7.33-7.38(1H, m), 7.73(2H, d, J=8.8 Hz), 7.97(2H, d, J=8.8 Hz), 8.05-8.08(1H, m), 8.27-8.30(1H, m), 8.75-8.76(1H, m), 9.59(1H, s), 10.44(1H, s), 10.47(1H, s)
IR(KBr)cm-1 : 3410, 3315, 1685, 1655, 1625, 1536, 1428, 1362, 1263, 1201, 744

### Example 123

### N-(2-aminbphenyl)-4-[N-(pyridin-3-yl)methoxyacetylamino]-3-methylbenzamide (Table 1: Compound 102)

mp: 178-181 °C(dec.)
¹ H NMR(270 MHz, DMSO-d6).delta. ppm: 2.28(3H, s), 4.22(2H, s), 4.71(2H, s), 4.89(2H, br.s), 6.60(1H, dd, J=7.3, 7.3 Hz), 6.78(1H, d, J=8.1 Hz), 6.97(1H, dd, J=7.3, 8.1 Hz), 7.16(H, d, J=7.3 Hz), 7.43(1H, dd, J=4.4, 8.1 Hz), 7.71(1H, d, J=8.1 Hz), 7.79-7.89(3H, m), 8.54(1H, dd, J=1.5, 4.4 Hz), 8.66(1H, d, J=1.5 Hz), 9.36(1H, br.s), 9.60(1H, br.s)
IR(KBr)cm-1 : 3394, 3269, 1683, 1630, 1593, 1521, 1460, 1131, 750, 716

### Example 124

### N-(2-aminophenyl)-4-[N-(thiophen-3-yl)methoxyacetylamino]benzamide (Table 1: Compound 204)

mp: 186-189°C
¹ H NMR(270 MHz, DMSO-d6) .delta. ppm: 4.11(2H, s), 4.63(2H, s), 4.89(2H, br.s), 6.60(1H, dd, J=7.3, 7.3 Hz), 6.78(1H, d, J=8.1 Hz), 6.97(1H, dd, J=7.3, 7.3 Hz), 7.12-7.19(2H, m), 7.53-7.57(2H, m), 7.78(2H, d, J=8.8 Hz), 7.95(2H, d, J=8.8 Hz), 9.58(1H, br.s), 10.04(1H, br.s)
IR(KBr)cm-1 : 3341, 3248, 1694, 1631, 1611, 1506, 1314, 1126

### Example 125

### N-(2-aminophenyl)-4-[N-methyl-N-(pyridin-3-yl)methoxyacetylamino]benzamide (Table 1: Compound 103)

mp: 180-183°C(dec.)
¹ H NMR(270 MHz, DMSO-d6) .delta. ppm: 3.24(3H, s), 4.08(2H, br.s), 4.50(2H, s), 4.94(2H, br.s), 6.60(1H, dd, J=7.3, 7.3 Hz), 6.79(1H, d, J=8.1 Hz), 6.98(1H, dd, J=7.3, 8.1 Hz), 8.03(1H, d, J=8.1 Hz), 8.48-8.50(2H, m), 9.72(1H, br.s)
IR(KBr)cm-1 : 3395, 3283, 1683, 1639, 1604, 1506, 1459, 1307, 1124

### Example 126

### N-(2-aminophenyl)-4-[N-(pyridin-2-yl)methoxyacetylamino]benzamide (Table 1: Compound 176)

mp: 171-173°C
¹H NMR(270 MHz, DMSO-d6) .delta. ppm: 4.26(2H, s), 4.74(2H, s), 4.89(2H, br.s), 6.60(1H, dd, J=6.6, 8.1 Hz), 6.78(1H, d, J=7.3 Hz), 6.97(1H, ddd,J=1.5, 7.3, 8.1 Hz), 7.16(1H, d, J=7.3 Hz), 7.35(1H, dd, J=5.1, 6.6 Hz), 7.80(2H, d, J=8.1 Hz), 7.80-7.89(1H, m), 7.97(2H, d, J=8.1 Hz), 8.59(1H, d, J=4.4 Hz), 9.59(1H, br.s), 10.30(1H, br.s)
IR(KBr)cm-1 : 3391, 3258, 1678, 1629, 1593, 1517, 1128, 767, 742

### Example 127

### N-(2-aminophenyl)-4-[N-(N-nicotinoylamino)acetylamino]benzamide (Table 1: Compound 97)

mp: 218-220°C(dec.)
¹ H NMR(270 MHz, DMSO-d6) .delta. ppm: 4.13(2H, d, J=5.9 Hz), 4.89(2H, s), 6.59(1H, dd, J=7.3, 7.3 Hz), 6.77(1H, d, J=8.1 Hz), 6.96(1H, dd, J=7.3, 8.1 Hz), 7.15(1H, d, J=7.3 Hz), 7.55(1H, dd, J=5.1, 8.1 Hz), 7.73(2H, d, J=8.8 Hz), 7.96(2H, d, J=8.8 Hz), 8.25(1H, d, J=8.1 Hz), 8.74(1H, d, J=5.1 Hz), 9.07(1H, d, J=1.5 Hz), 9.13(1H, t-like, J=5.9 Hz), 9.58(1H, s), 10.36(1H, s)

### Example 128

### N-(2-aminophenyl)-5-[3-(pyridin-3-yl)propionamide]benzofuran-2-carboxyamide (Table 3: Compound 1)

mp: 267-272°C
¹ H NMR(270 MHz, DMSO-d6) .delta. ppm: 2.51 (2H, t, J=7.3 Hz), 2.97(2H, t, J=7.3 Hz), 6.61(1H, dd, J=8.1, 8.8 Hz), 6.80(1H, dd, J=1.5, 8.1 Hz), 6.99(1H, dd, J=8.1, 8.8 Hz), 7.20(1H, dd, J=1.5, 8.1 Hz), 7.32(1H, dd, J=5.2, 8.1 Hz), 7.49(1H, dd, J=1.5, 8.8 Hz), 7.61(1H, d, J=8.8 Hz), 7.67(1H, s), 7.70(1H, m), 8.15(1H, d, J=1.5 Hz), 8.40(1H, dd, J=1.5, 5.2 Hz), 8.51(1H, d, J=1.5 Hz), 9.84(1H, s), 10.1(1H, s)
IR(KBr)cm-1 : 3333, 3272, 1666, 1583, 1561, 1458, 1314, 1247, 1143, 807, 746, 713

### Example 129

### Preparation of N-(2-aminophenyl)-4-[N-[2-(pyridin-3-yl)oxypropionyl]amino]benzamide (Table 4: Compound 2)

(129-1) In 10 ml of dichloromethane were dissolved 0.34 g of the compound from Example 47, the process (47-2) (1.2 mmol) and 0.34 g of the compound from Example 100, the process (100-2) (1.0 mmol), and then 0.5 ml of triethylamine (3.6 mmol). Under ice-cooling, to the solution was added 0.21 g of 2-chloro-1,3-dimethylimidazolidinium chloride (1.24 mmol) in 5 ml of dichloromethane, and the solution was stirred under ice-cooling for 2 hours. After neutralizing with saturated sodium bicarbonate aq., the mixture was diluted with water and extracted with chloroform. The organic layer was washed with saturated brine, dried and evaporated. The residue was purified by column chromatography on silica gel (eluent: ethyl acetate:methanol=10:1) to give 0.68 g of N-[2-(N-tert-butoxycarbonylamino)phenyl]-4-[N-[2-(pyridin-3-yl)oxypropiony I]amino]benzamide as a mixture with 1,3-dimethyl-2-imidazolinone.
¹ H NMR(270 MHz, CDCl.sub.3) .delta. ppm: 1.52(9H,s), 1.70(3H, d, J=6.6 Hz), 4.84(1H, q, J=6.6 Hz), 6.89(1H, br.s), 7.12-7.31(6H, m), 7.68(2H, d, J=8.8 Hz), 7.79(1H, d, J=8.1 Hz), 7.96(2H, d, J=8.8 Hz), 8.34(1H, d, J=2.9, 2.9 Hz), 8.43(1H, d, J=1.5 Hz), 9.25(1H, br.s)

(129-2) To a solution of 0.68 g of the compound from the process (129-1) in 5 ml of dichloromethane was added 10 ml of 15% (vol/vol) trifluoroacetic acid/dichloromethane, and the solution was stirred at room temperature for 4.5 hours. After neutralizing the solution with saturated sodium bicarbonate aq., dichloromethane was removed by evaporation. The solution was extracted with ethyl acetate. The organic layer was washed with saturated brine, dried and evaporated. To the residue were added methanol and diisopropyl ether, and the precipitated solid was collected by filtration and dried to give 0.22 g of N-(2-aminophenyl)-4-[N-[2-(pyridin-3-yl)oxypropionyl]amino]benzamide (Yield: 58% for the 2 steps) as an opalescent solid.
mp: 193-196°C
¹H NMR(270 MHz, DMSO-d6) .delta. ppm: 1.60(3H, d, J=6.6 Hz), 4.88(2H, br.s), 5.04(1H, q, J=6.6 Hz), 6.60(1H, dd, J=6.6, 7.3 Hz), 6.78(1H, d, J=8.1 Hz), 6.97(1H, dd, J=7.3, 8.1 Hz), 7.15(1H, d, J=7.3 Hz), 7.32-7.39(2H, m), 7.75(2H, d, J=8.8 Hz), 7.96(2H, d, J=8.1 Hz), 8.20(1H, dd, J=1.5, 3.7 Hz), 8.35(1H, d, J=2.1 Hz), 9.59(1H, br. s), 10.44(1H, br. s)

### Example 130

### Preparation of N-(2-aminophenyl)-4-[(pyridin-3-yl)methoxyacetylamino]benzamide (Table 1: Compound 101)

(130-1) To a suspension of 4.4 g of sodium hydride (60% oil dispersion; 110 mmol) in 300 ml of THF were added dropwise 10.91 g of 3-pyridinemethanol (100 mmol) in 20 ml of THF at room temperature, and the mixture was stirred at room temperature for 2 hours. The resulting white suspension was ice-cooled, and 19.51 g of tert-butyl bromoacetate (100 mmol) in 20 ml of THF was added dropwise, maintaining the inner temperature within 10 to 12°C The suspension was warmed to room temperature with stirring for 3 hours, and then left overnight. After adding water and saturated sodium bicarbonate aq., the mixture was extracted with ethyl acetate. The organic layer was washed with saturated brine, dried and evaporated. The residue was purified by column chromatography on silica gel (gradient elution with n-hexane:ethyl acetate=1:1 to ethyl acetate) to give 7.56 g of tert-butyl (pyridin-3-yl)methoxyacetate (33.8%) as a light brown oil.
¹ H NMR(270 MHz, CDCl.sub.3) .delta. ppm: 1.49(9H,9s), 4.03(2H, s), 4.64(2H, s), 7.30(1H, dd, J=4.9, 7.3 Hz), 7.76(1H, d, J=7.3 Hz), 8.56(1H, d, J=4.9 Hz), 8.60(1H, s)

(130-2) Under ice-cooling, 12 ml of trifluoroacetic acid was added to 3.5 g of the compound from the process (130-1) (15.7 mmol), and the solution was stirred at room temperature for 6 hours. Part of trifluoroacetic acid was removed by evaporation to give a mixture of (pyridin-3-yl)methoxyacetic acid and trifluoroacetic acid (6.5 g). The mixture was dissolved in 70 ml of dichloromethane. To the solution was added 25 ml of pyridine and then, was slowly added dropwise under ice-cooling, 2.37 g of 2-chloro-1,3-dimethylimidazolinium chloride (14.0 mmol) in 20 ml of dichloromethane over 30 min, and the solution was stirred under ice-cooling for additional 5 hours. To the solution was added saturated sodium bicarbonate aq., and stirring was continued until foaming ceased. The mixture was extracted with chloroform. The organic layer was washed with saturated brine, dried and evaporated. The residue was purified by column chromatography on silica gel (gradient elution with ethyl acetate to ethyl acetate:methanol=10:1) to give 4.78 g of N-[2-(N-tertbutoxycarbonyl)aminophenyl]-4-[N-(pyridin-3-yl)methoxyacetyla mino]benzamide (Yield: 62%) as a 1:1 (molar ratio) mixture with DMI (1,3-dimethyl-2-imidazolinone).
¹ H NMR(270 MHz, CDCl.sub.3) .delta. ppm: 1.51(9H, s), 4.15(2H, s), 4.70(2H, s), 6.92(1H, br.s), 7.15-7.29(3H, m), 7.37(1H, dd, J=7.3, 5.1 Hz), 7.67(2H, d, J=8.8 Hz), 7.71-7.79(2H, m), 7.96(2H, d, J=8.8 Hz), 8.41(1H, s), 8.62-8.66(2H, m), 9.23(1H, br.s)

(130-3) To a solution of 2.39 g of the compound from the process (130-2) (4.0 mmol) in 28 ml of dichloromethane was added 55 ml of 15% (vol/vol) trifluoroacetic acid/dichloromethane, and the solution was stirred at room temperature for 7 hours. The solution was neutralized with saturated sodium bicarbonate, and then water was added. The reaction mixture was stirred at room temperature and extracted with a 2:1 mixture of ethyl acetate-methyl ethyl ketone, a 2:1 mixture of ethyl acetate-THF, and ethyl acetate, in sequence. The combined organic layer was washed with saturated brine and dried over anhydrous sodium sulfate. After removing the dehydrating reagent by filtering, the filtrate was concentrated. To the residue thus obtained were added methanol and diisopropyl ether, and the precipitated solid was collected by filtration and dried to give 1.29 g of N-(2-aminophenyl)-4-[N-(pyridin-3-yl)methoxyacetylamino]benzamide (Yield: 85.6%) as a dark brown solid.
¹ H NMR(270 MHz, DMSO-d6) .delta. ppm: 4.19(2H, s), 4.68(2H, s), 4.90(2H, br.s), 6.60(1H, ddd, J=1.5, 7.3, 8.1 Hz), 6.78(1H, dd, J=1.5, 8.1 Hz), 6.97(1H, dd, J=7.3, 7.3 Hz), 7.15(1H, d, J=7.3 Hz), 7.42(1H, dd, J=4.4, 8.1 Hz), 7.77(2H, d, J=8.8 Hz), 7.85(1H, d, J=7.3 Hz), 7.96(2H, d, J=8.8 Hz), 8.54(1H, dd, J=1.5, 5.1 Hz), 8.63(1H, s), 9.58(1H, s), 10.09(1H, s)
IR(KBr)cm-1 : 3403, 3341, 3250, 1694, 1630, 1610, 1506, 1314, 1259, 1118, 764

### Example 131

### Preparation of N-(2-aminophenyl)-4-[N-[2-(pyridin-3-yl)methoxypropionyl]amino]benzamide (Table 4: Compound 1)

(131-1) To a suspension of 1.24 g of sodium hydride (60% oil dispersion; 31 mmol) in 90 ml of THF were added dropwise 3.27 g of 3-pyridinemethanol (30 mmol) in 10 ml of dry THF at room temperature over 5 min. The resulting white suspension was stirred at room temperature for an hour, to which was then added dropwise 6.27 g of tert-butyl 2-bromopropionate (30 mmol) in 10 ml of dry THF at room temperature over 5 min. The mixture was stirred at room temperature for 11.5 hours. After adding water, the mixture was extracted with ethyl acetate. The organic layer was washed with saturated brine, dried and evaporated. The residue was purified by column chromatography on silica gel (eluent: n-hexane:ethyl acetate=1:1) to give 4.01 g of tert-butyl (pyridin-3-yl)methoxyacetate (Yield: 56.3%) as a dark brown oil.
¹ H NMR(270 MHz, CDCl.sub.3) .delta. ppm: 1.42(3H, d, J=7.3 Hz), 1.50(9H, s), 3.96(1H, q, J=6.6 Hz), 4.47, 4.69 (2H, ABq, J=11.0 Hz), 7.29(1H, dd, J=5.1, 8.1 Hz), 7.75(1H, d, J=8.1 Hz), 8.5(1H, d, J=4.4 Hz), 8.60(1H, s)

(131-2) To a solution of 1.09 g of the compound from the process (131-1) (4.59 mmol) in 5 ml of dichloromethane was added 8 ml of trifluoroacetic acid, and the solution was stirred at room temperature for 9.5 hours. After evaporation, to the residue was added 25 ml of dichloromethane and 3 ml of pyridine. Under ice-cooling, to the solution was added dropwise 0.70 g of 2-chloro-1,3-dimethylimidaolidinium chloride (4.1 mmol) in 8 ml of dichloromethane, and then the mixture was stirred for 30 min. To the solution was slowly added dropwise 0.98 g of the compound from Example 100, the process (100-2) (3.0 mmol) in 20 ml of dichloromethane and 10 ml of pyridine under ice-cooling over 15 min, and the solution was warmed to room temperature with stirring for 8 hours. After adding saturated sodium bicarbonate aq., the mixture was diluted with water and extracted with chloroform. The organic layer was washed with saturated brine, dried and evaporated. The residue was purified by column chromatography on silica gel (eluent: ethyl acetate:methanol=8:1) to give 1.19 g of N-[2-(N-tert-butoxycabonylamino)phenyl]-4-[N-[2-(pyridin-3-yl)methoxypropi onyl]amino]benzamide as a 2:3 (molar ratio) mixture with 1,3-dimethyl-2-imidazolinone.
¹H NMR(270 MHz, CDCl.sub.3) .delta. ppm: 1.51(9H, s), 1.54(3H, d, J=6.6 Hz), 4.13(4H, q, J=6.6 Hz), 4.65, 4.71(2H, ABq, J=11.7 Hz), 7.12-7.18(2H,m), 7.28-7.37(3H, m), 7.65(2H, d, J=8.1 Hz), 7.73(2H, br.d, J=5.9 Hz), 7.96(2H, d, J=8.8 Hz), 8.59-8.64(3H, m), 9.39(1H, br.s)

(131-3) To a solution of 1.19 g of the compound from the process (131-2) (1.8 mmol) in 10 ml of dichloromethane was added 20 ml of 15% (vol/vol) trifluoroacetic acid in dichloromethane, and the solution was stirred at room temperature for 4.5 hours. The solution was poured into saturated sodium bicarbonate, and dichloromethane was removed by evaporation. The resulting aqueous layer was extracted with ethyl acetate. The organic layer was washed with saturated brine, dried and evaporated. To the residue were added methanol and diisopropyl ether, and the precipitated solid was collected by filtration and dried to give 585 mg of N-(2-aminophenyl)-4-[N-[2-(pyridin-3-yl)methoxypropionyl]amino]benzamide as a light brown solid.
mp: 144-148°C
¹ H NMR(270 MHz, DMSO-d6) .delta. ppm: 1.40(3H, d, J=6.6 Hz), 4.14(1H, q, J=6.6 Hz), 4.56 and 4.65(2H, ABq, J=11.8 Hz), 4.89(2H, br.s), 6.60(1H, dd, J=7.3, 7.3 Hz), 6.78(1H, d, J=8.1 Hz), 6.97(1H, dd, J=6.6, 7.3 Hz), 7.16(1H, d, J=7.3 Hz), 7.40(1H, dd, J=4.4 Hz, 7.3 Hz), 7.78-7.85(3H, m), 7.97(2H, d, J=8.8 Hz), 8.52(1H, dd, J=1.5, 5.1 Hz), 8.61(1H, d, J=2.1 Hz), 9.60(1H, s), 10.15(1H, s)

### Example 132

### Preparation of N-(2-aminophenyl)-4-(N-benzylamino)carbonylbenzamide (Table 1: Compound 8)

(132-1) To a suspension of 13.0 g of monomethyl terephthalate (72.2 mmol) in 100 ml of toluene was added dropwise 10 ml of thionyl chloride at room temperature. After stirring at 80°C for 3 hours, the solvent and an excess amount of thionyl chloride were removed by evaporation. The residue was suspended in 100 ml of dioxane, and 9.98 g of 2-nitroaniline (72.2 mmol) were added to the suspension, followed by refluxing with heating for 4 hours.

After cooling and evaporation, the residue was washed with methanol to give 20.3 g of N-(2-nitrophenyl)-4-methoxycarbonylbenzamide (Yield: 93.7%) as a yellow solid.
¹ H NMR(270 MHz, DMSO-d6) .delta. ppm: 3.91 (3H, s), 7.43-7.49(1H, m), 7.76-7.78(2H, m), 8.03(1H, d, J=8.1 Hz), 8.08(2H, d, J=8.8 Hz), 8.14(2H, d, J=8.8 Hz), 10.94(1H, s)

(132-2) To a solution of 4.24 g of the compound from the process (132-1) in 50 ml of THF and 50 ml of methanol was added 0.4 g of 10% Pd/C in a stream of nitrogen, and the mixture was stirred in a stream of hydrogen for 1.5 hours. The catalyst was removed by filtration, and the filtrate was evaporated. The residue was washed with methanol to give 3.4 g of N(2-aminophenyl)-4-methoxycarbonylbenzamide (Yield: 87.5%) as a light yellow solid.
¹H NMR(270 MHz, DMSO-d6) .delta. ppm: 3.90(3H, s), 4.95(2H, s), 6.60(1H, dd, J=7.3, 8.1 Hz), 6.78(1H, d, J=7.3 Hz), 6.99(1H, dd, J=77.3, 7.3 Hz), 7.17(1H, d, J=7.3 Hz), 8.08(2H, d, J=8.1 Hz), 8.11(2H, d, J=8.1 Hz), 9.85(1H, s)

(132-3) To a solution of 2.71 g of the compound from the process (132-2) (10.0 mmol) in 100 ml of dioxane and 50 ml of water was added 5% sodium hydroxide aq. under ice-cooling, and then were added dropwise 2.62 g of di-tert-butyl dicarbonate (12.0 mmol) in 40 ml of dioxane. The mixture was stirred at room temperature for 4 hours and left overnight. To the mixture were added saturated brine and ethyl acetate, and the two layers were separated. The aqueous layer was extracted with ethyl acetate. The organic layer was washed with saturated brine, dried and evaporated. The residue was washed with methanol to give 3.54 g of N-[2-(N-tert-butoxycarbonyl)aminohenyl]-4-methoxycarbonylbenzamide (Yield: 95.7%) as a light brown solid.
¹ H NMR(270 MHz, DMSO-d6) .delta. ppm: 1.44(9H, s), 3.90(3H, s), 7.12-7.24(2H, m), 7.55-7.58(2H, m), 8.09(2H, d, J=8.8 Hz), 8.10(2H, d, J=8.8 Hz), 8.72(1H, s), 10.00(1H, s)

(132-4) A suspension of 3.00 g of the compound from the process (132-3) (8.10 mmol) in 50 ml of methanol and 25 ml of 0.5N lithium hydroxide aq. was heated with stirring at 40°C for 5 hours. After removing methanol by evaporation, to the residue was added 1N hydrochloric acid, and the mixture was extracted with ethyl acetate. The organic layer was washed with a small amount of water and saturated brine, dried and evaporated. The residue was washed with methanol to give 2.24 g of terephthalic mono-2-(N-tert-butoxycarbonyl)aminoanilide (Yield: 77.6%) as a light brown solid.
¹ H NMR(270 MHz, DMSO-d6) .delta. ppm: 1.45(9H, s), 7.12-7.21(2H, m), 7.53-7.58(2H, m), 8.06(2H, d, J=8.8 Hz), 8.10(2H, d, J=8.8 Hz), 8.71(1H, s), 9.97(1H, s)

(132-5) To a suspension of 0.20 g of the compound from the process (132-4) (0.56 mmol) in 4 ml of dichloromethane were added 0.14 g of benzylamine (1.3 mmol) and then 0.21 ml of triethylamine (1.5 mmol). To the solution was added 0.25 g of 2-chloro-1,3-dimethylimidazolium chloride (1.48 mmol) under ice-cooling, and then the mixture was stirred under ice-cooling for an hour and at room temperature for an hour. After diluting with chloroform and adding water, the aqueous layer was extracted with chloroform.

The combined organic layer was washed with saturated brine, dried and evaporated. The residue was purified by column chromatography on silica gel (eluent: chloroform:methanol=10:1). The solid obtained was washed with diethyl ether to give 279 mg of N-(2-tert-butoxycarbonylaminophenyl)-4-(N-benzylamino)carbonylbenzamide (Yield: 62.6%) as a white solid.
¹ H NMR(270 MHz, DMSO-d6) .delta. ppm: 1.45(9H, s), 4.52(2H, d, J=5.8 Hz), 7.13-7.28(4H, m), 7.34-7.35(3H, m), 7.56(2H, d, J=8.1 Hz), 8.05(4H, s), 8.71(1H, br.s), 9.23(1H, t), 9.94(1H, s)

(132-6) To 151 mg of the compound from the process (132-5) (0.339 mmol) was added 5 ml of 4N hydrochloric acid-dioxane at room temperature, and the mixture was stirred for 4 hours. After evaporation, the mixture was partitioned between ethyl acetate and saturated sodium bicarbonate aq. After removing the precipitate, the aqueous layer was extracted with ethyl acetate. The combined organic layer was washed with saturated brine, dried and evaporated. To the residue was added diethyl ether, and the precipitate was collected by filtration and dried to give 78 mg of N-(2-aminophenyl)-4-(N-benzylamino)carbonylbenzamide (Yield: 67%) as a white solid.
mp: 239-241 °C(dec.)
¹ H NMR(270 MHz, DMSO-d6) .delta. ppm: 4.51(2H, s), 4.93(2H, br.d), 6.60(1H, dd, J=7.3, 7.3 Hz), 6.78(1H, d, J=8.1 Hz), 6.95(1H, dd, J=7.3, 8.3 Hz), 7.18(1H, d), 7.23-7.35(5H, m), 8.01(2H, d, J=8.8 Hz), 8.07(2H, d, J=8.8 Hz), 9.22(1H, br.t), 9.81(1H, br.s)

As described in Example 132, the compound of Example 133 was prepared, whose melting point (mp), 1 H NMR data and IR data are shown below.

### Example 133

### N-(2-aminophenyl)-4-[N-(2-phenylethyl)amino]carbonylbenzamide (Table 1: Compound 9)

mp: 237-240°C(dec.)
¹H NMR(270 MHz, DMSO-d6).delta. ppm: 2.87(2H, t, J=7.3 Hz), 3.51(2H, dt, J=5.9, 7.3 Hz), 4.94(2H, br.s), 6.60(1H, dd, J=7.3, 7.3 Hz), 6.78(1H, d, J=7.3 Hz), 6.98(1H, dd, J=7.3, 7.3 Hz), 7.15-7.34(6H, m), 7.93(2H, d, J=8.1 Hz), 8.04(2H, d, J=8.1 Hz), 8.73(1H, t, J=5.1 Hz), 9.76(1H, br.s)
IR(KBr)cm-1 : 3396, 3320, 1625, 1602, 1539, 1458, 1313, 699

### Example 134

### Preparation of N-(2-aminophenyl)-4-[N-(4-nitrophenoxyacetyl)amino]benzamide (Table 1: Compound 54)

(134-1) To a solution of 3 g of the compound from Example 100, the process (100-2) (9.2 mmol) and 2.16 g of 4-nitrophenoxyacetic acid (11.0 mmol) in 7 ml of DMF were added 2.82 g of dicyclohexylcarbodiimide (13.8 mmol) in 5 ml of DMF and a catalytic amount of N,N-dimethylaminopyridine, and the mixture was stirred for one day. After completion of the reaction, ethyl acetate was added to the mixture, insolubles were filtered off through celite, and the solvent was removed by evaporation.

The residue was recrystallized from chloroform to give 2.34 g of N-[2-(tert-butoxycarbonylamino)phenyl]-4-[(4-nitrophenoxyacetyl)amino]benz amide (Yield: 50%).
¹ H NMR(270 MHz, DMSO-d6) .delta. ppm: 1.45(9H, s), 4.97(2H, s), 7.12-7.26(3H, m), 7.23(2H, d, J=8.8 Hz), 7.53(1H, dt, J=2.2, 7.3 Hz), 7.79(2H, d, J=8.8 Hz), 7.95(2H, d, J=8.8 Hz), 8.25(2H, d, J=8.8 Hz), 8.71(1H, s), 9.79(1H, s), 10.52(1H, s)

(134-2) To a solution of 0.7 g of the compound from the process (134-1) (1.38 mmol) in 10 ml of acetonitrile was added 1.26 ml of iodotrimethylsilane (8.85 mmol) at room temperature, and the solution was stirred for 2 hours. After completion of the reaction, the solution was concentrated. Ethyl acetate was added to the residue, the solution was stirred for 20 min, and the precipitated crystals were collected by filtration. The crystals were dissolved in methyl ethyl ketone. The solution was washed with saturated sodium thiosulfate aq. and saturated brine in sequence, dried over anhydrous magnesium sulfate, and evaporated. The residue was washed with ethyl acetate to give 0.22 g of N-(2-aminophenyl)-4-[N-(4-nitrophenoxyacetyl)amino]benzamide (Yield: 39%) as white crystals.
mp: 212-215°C(dec.)
¹ H NMR(270 MHz, DMSO-d6) .delta. ppm: 4.97(2H, s), 6.88(1H, t, J=7.3 Hz), 6.99(1H, d, J=7.3 Hz), 7.11(1H, t, J=7.3 Hz), 7.23(2H, d, J=8.8 Hz), 7.24(1H, m), 7.77(2H, d, J=8.8 Hz), 8.00(2H, d, J=8.8 Hz), 8.25(2H, d, J=8.8 Hz), 9.89(1H, s), 10.52(1H, s)
IR(KBr)cm-1 : 3382, 3109, 1650, 1591, 1508, 1341

### Example 135

### Preparation of N-(2-aminophenyl)-4-[(4-aminophenoxyacetyl)amino]benzamide (Table 1: Compound 55)

To a solution of 1.41 g of the compound from Example 134, the process (134-1) (2.78 mmol) in 15 ml of methanol and 25 ml of THF was added 10% Pd-C, and the mixture was stirred in an atmosphere of hydrogen, at room temperature for an hour. After completion of the reaction, the catalyst was filtered off and the filtrate was concentrated. The residue was triturated with diisopropyl ether to give 1.1 g of N-[2-(tert-butoxycarbonylamino)phenyl]-4-[(4-aminophenoxyacetyl)amino]benz amide.

The product was dissolved in 15 ml of acetonitrile. To the solution was added 0.74 ml of iodotrimethylsilane (5.20 mmol), and the mixture was stirred at room temperature for 3 hours. After completion of the reaction, the mixture was evaporated. The residue was washed with methyl ethyl ketone to give 0.86 g of N-(2-aminophenyl)-4-[(4-aminophenoxyacetyl)amino]benzamide (Yield: 83%).
mp: (amorphous)
¹ H NMR(270 MHz, DMSO-d6) .delta. ppm: 4.82(2H, s), 7.13(2H, d, J=8.8 Hz), 7.30-7.48(6H, m), 7.82(2H, d, J=8.8 Hz), 8.03(2H, d, J=8.8 Hz), 10.34(1H, s), 10.46(1H, s)
IR(KBr)cm-1 : 2873, 2590, 1680, 1602, 1505, 1243

### Example 136

### Preparation of N-(2-aminophenyl)-4-(5-phenoxymethyl-1.3-oxazolin-2-on-3-yl)benzamide (Table 2: Compound 1)

(136-1) To 0.7 g of tert-butyl 4-(N-benzyloxycarbonylamino)benzoate (2.14 mmol) in 10 ml of THF at -78°C was added dropwise 1.33 ml of n-butyl lithium (2.25 mmol) over 5 min. The mixture was stirred at the same temperature for 1.5 hours. To the mixture was then added 0.31 ml of phenylglycidol (2.29 mmol), and the reaction mixture was then stirred at the same temperature for an hour and left overnight at room temperature. After adding saturated ammonium chloride aq., the mixture was extracted twice with ethyl acetate. The organic layer was dried over anhydrous magnesium sulfate and evaporated. The residue was recrystallized from diethyl ether to give 0.31 g of N-[4-(tertbutoxycarbonyl)phenyl]-5-phenoxymethyl-1,3-oxazolin-2-one (Yield: 39%).
1 H NMR(270 MHz, DMSO-d6) .delta. ppm: 1.53(9H, s), 3.97(1H, dd, J=6.0, 8.8 Hz), 4.23-4.34(3H, m), 5.11(1H, m), 6.94-7.00(3H, m), 7.31(2H, m), 7.71(2H, d, J=8.8 Hz), 7.93(2H, d, J=8.8 Hz)

(136-2) To a solution of 0.26 g of the compound from the process (136-1) (0.704 mmol) in 4 ml of acetonitrile was added 0.15 ml of trimethylsilyl iodide (1.05 mmol), and the solution was stirred at room temperature for 2 hours. After completion of the reaction, the solution was concentrated. The concentrate was triturated with ethyl acetate-methyl ethyl ketone to give 0.2 g of N-(4-carboxyphenyl)-5-phenoxymethyl-1,3-oxazolin-2-one (Yield: 91 %).
¹H NMR(270 MHz, DMSO-d6) .delta. ppm: 3.98(1H, dd, J=6.6, 9.6 Hz), 4.23-4.34(3H, m), 5.10(1H, m), 6.94-6.99(3H, m), 7.30(2H, t, J=8.1 Hz), 7.72(2H, d, J=8.8 Hz), 7.98(2H, d, J=8.8 Hz), 12.85(1H, s)

(136-3) To a solution of 0.15 g of the compound from the process (136-2) (0.479 mmol) in 7 ml of dichloromethane were added a catalytic amount of DMF and 0.12 ml of oxalyl chloride (1.40 mmol), and the solution was stirred at room temperature for 2 hours. The solution was concentrated and the residual solvent was azeotropically removed twice with toluene. To a solution of the residue in 4 ml of dichloromethane were added a solution of 0.105 g of the compound from Example 1, the process (1-2) (0.504 mmol) and 0.12 g of pyridine (1.52 mmol) in 1 ml of dichloromethane under ice-cooling, and the solution was warmed to room temperature and stirred for an hour. After completion of the reaction, water was added. The mixture was extracted twice with chloroform. The organic layer was washed with saturated brine, dried over anhydrous magnesium sulfate and evaporated. The residue was triturated with diisopropyl ether to give 0.25 g of N-[2-(N-tert-butoxycarbonylamino)phenyl]-4-(5-phenoxymethyl-1,3-oxazolin-2 - on-3-yl)benzamide (Yield: quantitative).
¹ H NMR(270 MHz, DMSO-d6) .delta. ppm: 1.52(9H, s), 4.11(1H, dd, J=5.9, 6.6 Hz), 4.21-4.27(3H, m), 5.01(1H, m), 6.84(1H, br.s), 6.91 (2H, d, J=8.8 Hz), 7.01(1H, t, J=7.4 Hz), 7.12-7.34(5H, m), 7.68(2H, d, J=8.8 Hz)

(136-4) To a solution of 0.22 g of the compound from the process (136-3) (0.437 mmol) in 4 ml of acetonitrile was added 0.1 ml of trimethylsilyl iodide (0.703 mmol) at room temperature, and the solution was stirred for 2 hours. After adding saturated sodium thiosulfate aq., the mixture was extracted twice with ethyl acetate. The organic layer was dried over anhydrous magnesium sulfate and evaporated. The residue was recrystallized from methanol to give 0.13 g of N-(2-aminophenyl)-4-(5-phenoxymethyl-1,3-oxazolin-2-on-3yl)benzamide (Yield: 74%) as white crystals.
mp: 165-170°C(dec.)
¹ H NMR(270 MHz, DMSO-d6) .delta. ppm: 4.01(1H, dd, J=6.6, 9.6 Hz), 4.28-4.34(3H, m), 5.12(1H, m), 5.23(2H, br.s), 6.64(1H, t, J=7.4 Hz), 6.81(1H, d, J=8.1 Hz), 6.95-7.00(3H, m), 7.18(1H, d, J=6.6 Hz), 7.31(2H, t, J=8.1 Hz), 7.72(2H, d, J=8.8 Hz), 8.05(2H, d, J=8.8 Hz), 9.69(1H, s)
IR(KBr)cm-1 : 3393, 1740, 1610, 1508, 1253

As described in Example 136, the compounds of Examples 137 to 143 were prepared, each of whose melting point (mp), 1 H NMR data and/or IR data are shown below.

### Example 137

### N-(2-aminophenyl)-4-[5-(4-nitrophenoxy)methyl-1,3-oxazolin-2-on-3-yl]ben zamide (Table 2: Compound 2)

mp: 162-164°C
¹H NMR(270 MHz, DMSO-d6) .delta. ppm: 3.97(1H, dd, J=6.6, 9.5 Hz), 4.10(1H, dd, J=5.1, 11.0 Hz), 4.17(1H, dd, J=3.7, 11.0 Hz), 4.27(1H, t, J=8.8 Hz), 6.53-6.80(6H, m), 6.97(1H, t, J=8.1 Hz), 7.16(1H, d, J=6.6 Hz), 7.72(2H, d, J=8.8 Hz), 8.04(2H, d, J=8.8 Hz), 9.65(1H, s)
IR(KBr)cm-1 : 3356, 2365, 1741, 1609, 1510, 1247

### Example 138

### N-(2-aminophenyl)-4-(5-benzyloxymethyl-1,3-oxazolin-2-on-3-yl)benzamide hydrochloride (Table 2: hydrochloride of Compound 3)

mp: 181-183°C
¹ H NMR(270 MHz, DMSO-d6) .delta. ppm: 3.69(1H, dd, J=5.2, 11.0 Hz), 3.76(1H, dd, J=3.7, 11.0 Hz), 3.91(1H, dd, J=5.9, 8.8 Hz), 4.59(2H, s), 4.93(1H, m), 7.26-7.41(8H, m), 7.51(1H, m), 7.74(2H, d, J=8.8 Hz), 8.15(2H, d, J=8.8 Hz), 10.42(1H, s)

### Example 139

### N-(2-aminophenyl)-4-[5-(pyridin-3-yl)oxymethyl-1,3-oxazolin-2-on-3-yl]benza mide (Table 2: Compound 4)

mp: 199-201 °C
¹ H NMR(270 MHz, DMSO-d6) .delta. ppm: 4.01(1H, dd, J=6.6, 8.8 Hz), 4.28-4.46(3H, m), 4.96(2H, br.s), 5.14(1H, m), 6.61(1H, t, J=7.4 Hz), 6.79(1H, d, J=7.4 Hz) 6.98(1H, t, J=7.4 Hz), 7.16(1H, d, J=7.4 Hz), 7.36(1H, dd, J=4.4, 8.1 Hz), 7.44(1H, dd, J=1.5, 8.1 Hz)
IR(KBr)cm-1 : 2815, 2631, 2365, 1752, 1610, 1520, 1225

### Example 140

### N-(2-aminophenyl)-4-[5-(pyridin-3-yl)methyloxymethyl-1,3-oxazolin-2-on-3-yl ]benzamide (Table 2: Compound 5)

mp: 160-164°C(dec.)
¹H NMR(270 MHz, DMSO-d6) .delta. ppm: 3.73(1H, dd, J=5.2, 11.7 Hz), 3.79(1H, dd, J=2.9, 11.7 Hz), 3.91(1H. dd, J=5.9, 8.8 Hz), 4.21(1H, t, J=8.8 Hz), 4.62(2H, s), 4.91(3H, br.s), 6.60(1H, t, J=7.4 Hz), 6.78(1H, d, J=7.4 Hz), 6.98(1H, t, J=7.4 Hz), 7.16(1H, d, J=7.4 Hz), 7.38(1H, dd, J=4.4, 7.4 Hz), 7.69(2H, d, J=8.8 Hz), 7.71(1H, m), 8.03(2H, d, J=8.8 Hz), 8.51(1H, dd, J=1.5, 4.4 Hz), 8.54(1H, d, J=1.5 Hz), 9.65(1H, s)
IR(KBr)cm-1 : 3368, 1742, 1648, 1608, 1492, 1226

### Example 141

### N-(2-aminophenyl)-4-[5-(3-nitrophenoxy)methyl-1,3-oxazolin-2-on-3-yl]benzam ide (Table 2: Compound 6)

mp: 230°C(dec.)
¹ H NMR(270 MHz, DMSO-d6) .delta. ppm: 4.04(1H, t, J=8.8 Hz), 4.32(1H, t, J=8.8 Hz), 4.41-4.53(2H, m), 4.91(2H, s), 5.15(1H, m), 6.61(1H, t, J=7.4 Hz), 6.79(1H, d, J=7.4 Hz), 6.98(1H, t, J=7.4 Hz), 7.16(1H, d, J=7.4 Hz), 7.46(1H, dd, J=1.5, 8.1 Hz), 7.61(1H, t, J=8.1 Hz), 7.71-7.79(3H, m), 7.87(1H, d, J=8.1 Hz), 8.06(2H, d, J=8.8 Hz), 9.66(1H, s)
IR(KBr)cm-1 : 3363, 3095, 2365, 1741, 1608, 1529

### Example 142

### N-(2-aminophenyl)-4-[5-(pyridin-2-yl)methyloxymethyl-1,3-oxazolin-2-on-3-yl ]benzamide (Table 2: Compound 7)

mp: 172-174°C
¹ H NMR(270 MHz, DMSO-d6) .delta. ppm: 3.79(1H, dd, J=5.2, 11.0 Hz), 3.85(1H, dd, J=2.9, 11.0 Hz), 3.95(1H, dd, J=6.6, 9.6 Hz), 4.23(1H, t, J=9.6 Hz), 4.67(2H, s), 4.90(2H, s), 4.95(1H, m), 6.60(1H, t, J=7.4 Hz), 6.78(1H, d, J=7.4 Hz), 6.97(1H, t, J=7.4 Hz), 7.16(1H, d, J=7.4 Hz), 7.29(1H, dd, J=5.2, 6.6 Hz), 7.40(1H, d, J=6.6 Hz), 7.70(2H, d, J=8.8 Hz), 7.78(1H, dt, J=2.2, 7.4 Hz), 8.03(2H, d, J=8.8 Hz), 8.51(1H, d, J=4.4 Hz), 9.64(1H, s)
IR(KBr)cm-1 : 3369, 1743, 1651, 1608, 1492, 1283

### Example 143

### N-(2-aminophenyl)-4-[5-(pyridin-2-yl)oxymethyl-1,3-oxazolin-2-on-3-yl]benza mide (Table 2: Compound 8)

mp: (amorphous)
¹ H NMR(270 MHz, DMSO-d6) .delta. ppm: 3.96(1H, dd, J=5.9, 9.6 Hz), 4.21-4.40(3H, m), 4.90(2H, s), 5.03(1H, m), 6.28(1H, t, J=6.6 Hz), 6.43(1H, d, J=9.6 Hz), 6.60(1H, t, J=6.6 Hz), 6.78(1H, d, J=6.6 Hz), 6.97(1H, t, J=7.4 Hz), 7.15(1H, d, J=6.6 Hz), 7.46(1H, dt, J=7.4, 1.5 Hz), 7.67(2H, d, J=8.8 Hz), 7.69(1H, m), 8.03(2H, d, J=8.8 Hz), 9.64(1H, s)

### Example 144

### N-(2-aminophenyl)-4-[N-[3-[(pyridin-3-yl)methylamino]cyclobuten-1,2-dion-4-yl]aminomethyl]benzamide (Table 2: Compound 9)

(144-1) To a solution of 0.073 g of 3,4-di-n-butoxy-3-cyclobuten-1,2-dione (0.323 mmol) in 2 ml of THF was added 0.1 g of the compound from Example 1, the process (1-4) (0.293 mmol), and the solution was stirred for 4 hours. After adding 0.033 ml of 3-aminomethylpyridine (0.327 mmol), the solution was reacted for a day. After completion of the reaction, water was added to the solution, and the mixture was extracted twice with methyl ethyl ketone. The organic layer was dried over anhydrous magnesium sulfate and evaporated. The residue was triturated with methanol to give 0.12 g of N-[2-(N-tert-butoxycarbonylamino)phenyl]-4-[N-[3-[(pyridin-3-yl)methylamino]cyclobuten-1,2-dion-4-yl]aminomethyl]benzamide (Yield: 78%)
¹H NMR(270 MHz, DMSO-d6) .delta. ppm: 1.44(9H, s), 4.75-4.81(4H, m), 7.15(1H, dt, J=2.2, 7.4 Hz), 7.20(1H, dt, J=2.2, 7.4 Hz), 7.40(1H, dd, J=2.2, 7.4 Hz), 7.47(2H, d, J=8.1 Hz), 7.54(2H, dd, J=2.2, 7.4 Hz), 7.73(1H, m), 7.94(2H, d, J=8.1 Hz), 8.50(1H, m), 8.55(1H, d, J=1.5 Hz), 8.67(1H, s), 9.82(1H, s)

(144-2) To a solution of 0.1 g of the compound from the process (144-1) (0.19 mmol) in 4 ml of dioxane and 1 ml of methanol was added 4 ml of 4N hydrochloric acid-dioxane, and the mixture was reacted for 2 hours. After completion of the reaction, the mixture was concentrated and neutralized with saturated sodium bicarbonate aq. Methyl ethyl ketone was added to the mixture, and the precipitated crystals were collected by filtration to give 0.04 g of N-(2-aminophenyl)-4-[N-[3-[(pyridin-3-yl)methylamino]cyclobuten-1,2-dion-4 - yl]aminomethyl]benzamide (Yield: 49%).
mp: 230°C
¹H NMR(270 MHz, DMSO-d6) .delta. ppm: 4.76(2H, s), 4.79(2H, s), 4.90(2H, s), 6.60(1H, t, J=7.4 Hz), 6.78(1H, d, J=7.4 Hz), 6.97(1H, t, J=7.4 Hz), 7.16(1H, d, J=7.4 Hz), 7.39(1H, m), 7.43(2H, d, J=8.1 Hz), 7.73(1H, d, J=8.1 Hz), 7.97(2H, d, J=8.1 Hz), 7.99(1H, br.s), 8.51(1H, d, J=8.1 Hz), 8.55(1H, s), 9.64(1H, s)

### Example 145

### N-(2-aminophenyl)-4-[3-(pyridin-3-yl)methylimidazolin-2-on-1-yl]methylbenza mide (Table 2: Compound 10)

(145-1) Potassium carbonate (7.88 g; 57 mmol) was added to a solution of 4.92 g of ethylene urea (57 mmol), 5.73 g of methyl 4-bromomethylbenzoate (25 mmol) and 1.85 g of tetra-n-butylammonium iodide (5.0 mmol) in 30 ml of DMF, and the mixture was heated with stirring at 80°C for 5 hours. After cooling, the solid was collected by filtration and washed with ethyl acetate. The filtrate was concentrated. The residue was purified by column chromatography on silica gel (eluent: ethyl acetate:methanol=10:1). To the light yellow oil obtained was added diisopropyl ether, and the precipitated solid was collected by filtration and dried to give 3.36 g of N-(4-methoxycarbonylphenylmethyl)imidazolin-2-one (Yield: 57.4%) as a light brown solid.
¹H NMR(270 MHz, CDCl.sub.3) .delta. ppm: 3.28-3.35(2H, m), 3.41-3.47(2H, m), 3.92(3H, s), 4.42(2H, s), 4.61(1H, br.s), 7.35(2H, d, J=8.1 Hz), 8.01(2H, d, J=8.1 Hz)

(145-2) Saturated sodium bicarbonate aq. was added to 2.05 g of 3-chloromethylpyridine hydrochloride (12.5 mmol), and the mixture was extracted with ethyl acetate. The organic layer was washed with saturated brine, dried and evaporated. The residual solvent was azeotropically removed from the residue with toluene. To the residue was added 5 ml of DMF and then 0.37 g of tetra-n-butylammonium iodide (1.0 mmol) to prepare a solution of a benzyl halide in DMF. To a suspension of 0.30 g of sodium hydride (60% oil dispersion) (7.5 mmol) in 5 ml of DMF was slowly added dropwise a solution of 1.17 g of the compound from the process (145-1) (5.0 mmol) in 10 ml of DMF, and the solution was stirred at room temperature for 30 min. After adding the above solution of the benzyl halide, the resulting solution was heated with stirring at 80°C for 7 hours, and then left at room temperature overnight. After removing DMF, the residue was partitioned between ethyl acetate and water. The aqueous layer was extracted with ethyl acetate-methyl ethyl ketone (2:1). The combined organic layer was washed with saturated brine, dried and evaporated. The residue was purified by column chromatography on silica gel (eluent:ethyl acetate:methanol=10:1) to give 1.17 g of N-(4-methoxycarbonylphenylmethyl)-N'-(pyridin-3-yl)methylimidazolin-2-one (Yield: 72.3%) as a brown oil.
¹H NMR(270 MHz, CDCI.sub.3) .delta. ppm: 3.20(4H, s), 3.92(3H, s), 4.44(2H, S), 4.46(2H, S), 7.27-7.36(3H, m), 7.64-7.69(1H, m), 8.01(2H, d, J=8.1 Hz), 8.53-8.56(2H, m)

(145-3) To a solution of 0.55 g of the compound from the process (145-2) (1.7 mmol) in 8 ml of methanol and 8 ml of water were added 110 mg of lithium hydroxide monohydrate (1.7 mmol) at room temperature, and the solution was heated with stirring at 50°C for 1.5 hours. Additional lithium hydroxide monohydrate (0.05 g; 1.2 mmol) was added, and the solution was stirred at 50°C for additional 1.5 hours. The solution was acidified to pH 3-4) with 10% hydrochloric acid. Saturated brine was added, and the mixture was extracted twice with ethyl acetate and once with ethyl acetate-methyl ethyl ketone (1:1). The organic layer was dried over anhydrous sodium sulfate and evaporated. The residue was dried to give 0.32 g of 4-[3-(pyridin-3-yl)methylimidazolin-2-on -1-yl]methylbenzoic acid (Yield: 61%) as a brown oil.
¹ H NMR(270 MHz, DMSO-d6) .delta. ppm: 3.17(2H, s), 3.20(2H, s), 4.36(2H, s), 4.38(2H, s), 7.35-7.42(3H, m), 7.68(1H, dd, J=6.6 Hz), 7.92(2H, d, J=8.1 Hz), 8.51(2H, m)

(145-4) To a solution of 0.31 g of the compound from the process (145-3) (1.0 mmol) in 12 ml of dichloromethane was added dropwise 0.3 ml of oxalyl chloride (3.5 mmol) at room temperature, and the solution was stirred at room temperature for 30 min and then at 40°C for 1.5 hours. After evaporation, the residual solvent was azeotropically removed with toluene, and the residue was suspended in 10 ml of dichloromethane. To the suspension under ice-cooling was added dropwise 0.21 g of the compound from Example 1, the process (1-2) (1.0 mmol) in 2 ml of dichloromethane and 2 ml of pyridine. The mixture was warmed with stirring to room temperature and left at room temperature overnight. After adding saturated sodium bicarbonate aq., the mixture was extracted with chloroform. The organic layer was washed with saturated brine, dried and evaporated. The residue was purified by column chromatography on silica gel (eluent:ethyl acetate:methanol=20:1) to give 0.10 g of N-(2-tert-butoxycarbonylaminophenyl)-4-[3-(pyridin-3-ylmethyl)imidazolin-2 -on-1-yl]methylbenzamide (Yield: 20%) as a brown oil.
¹ H NMR(270 MHz, CDCl.sub.3) .delta. ppm: 1.52(9H, s), 3.20(4H, s), 4.45(2H, s), 4.48(2H, s), 6.75(1H, br.s), 7.15-7.40(5H, m), 7.65-7.70(2H, m), 7.83(1H, d, J=7.3 Hz), 7.94(2H, d, J=8.1 Hz), 8.50-8.60(3H, br.m)

(145-5) To a solution of 100 mg of the compound from the process (145-4) (0.20 mmol) in 2 ml of dioxane was added 2 ml of 4N hydrochloric acid-dioxane and then 0.5 ml of methanol to make the mixture homogenous. After stirring for 2 hours, the solution was neutralized with saturated sodium bicarbonate and extracted with ethyl acetate. The organic layer was washed with saturated brine, dried and evaporated. The residue was dried under reduced pressure to give 47 mg of N-(2-aminophenyl)-4-[3-(pyridin-3-yl)methylimidazolin-2-on-1-yl]methylbenz amide (Yield: 58%) as a brown oil.
mp: (amorphous)
¹H NMR(270 MHz, DMSO-d6) .delta. ppm: 3.20(4H, s), 4.37(2H, s), 4.39(2H, s), 4.87(2H, br.s), 6.60(1H, dd, J=7.3, 7.3 Hz), 6.78(1H, d, J=8.1 Hz), 6.97(1H, dd, J=6.6, 7.3 Hz), 7.16(1H, d, J=7.3 Hz), 7.35-7.41 (3H, m), 7.68(1H, d, J=8.1 Hz), 7.90-8.00(2H, m), 8.50(2H, br.s), 9.63(1H, br.s)

### Example 146

### Preparation of N-(2-aminophenyl)-4-[N-(pyridin-3-yl)methoxycarbonylaminomethyl]benzamide 0.5 fumarate (Table 1: fumarate of Compound 82)

To 10 ml of methanol were added 310 mg of the compound from Example 48, and the mixture was heated to dissolve the solid. To the solution was added 96 mg of fumaric acid in methanol, and the solution was cooled. The precipitated crystals were collected by filtration and recrystallized from 5 ml of methanol to give 200 mg of the desired product (Yield: 56%).
mp: 166-167°C
¹ H NMR(270 MHz, DMSO-d6) .delta. ppm: 4.28(2H, d, J=6.6 Hz), 5.10(2H, s), 6.60(1H, t, J=8.0 Hz), 6.63(1H, s), 6.78(1H, d, J=8.0 Hz), 6.90-7.50(5H, m), 7.70-8.00(4H, m), 8.53(1H, d, J=3.6 Hz), 8.60(1H, s), 9.63(1H, s)
IR(KBr)cm-1 : 3332, 1715, 1665, 1505, 1283, 1136, 1044, 983, 760, 712 Elementary analysis for C.sub.21 H.sub.20 N.sub.4 O.sub.3 +1/2C.sub.4 H.sub.4 O.sub.4

| | C | H | N |
|---|---|---|---|
| Calculated: | 63.59 | 5.10 | 12.90 |
| Observed: | 63.56 | 5.22 | 12.97 |

As described in Example 146, the compounds of Examples 147 to 149 are prepared, each of whose melting point (mp), 1 H NMR data, IR data and/or elementary analysis data are shown below.

### Example 147

### N-(2-aminophenyl)-4-[N-(pyridin-3-yl)methoxycarbonylaminomethyl]benzamide maleate (Table 1: maleate of Compound 82)

mp: 123-124°C
¹ H NMR(270 MHz, DMSO-d6) .delta. ppm: 4.28(2H, d, J=6.6 Hz), 5.11(2H, s), 6.24(2H, s), 6.66(1H, t, J=8.0 Hz), 6.83(1H, d, J=8.0 Hz), 6.90-8.00(9H, m), 8.56(1H, d, J=3.6 Hz), 8.62(1H, s), 9.69(1H, s)
IR(KBr)cm-1 : 3298, 1719, 1546, 1365, 1313, 1250, 1194, 1149, 1044, 993, 862, 751 Elementary analysis for C.sub.21 H.sub.20 N.sub.4 O.sub.3 +C.sub.4 H.sub.4 O.sub.4 +0.3 H.sub.2 O

| | C | H | N |
|---|---|---|---|
| Calculated: | 60.31 | 4.98 | 11.25 |
| Observed: | 60.52 | 5.12 | 11.03 |

### Example 148

### N-(2-aminophenyl)-4-[N-(pyridin-3-yl)methoxycarbonylaminomethyl]benzamide hydrochloride (Table 1: hydrochloride of Compound 82)

mp: 140(dec.) °C
¹ H NMR(270 MHz, DMSO-d6) .delta. ppm: 4.31(2H, d, J=5.8 Hz), 5.24(2H, s), 7.10-7.60(6H, m), 7.90-8.50(5H, m), 8.70-8.90(2H, m), 10.46(1H, s)
IR(KBr)cm-1 : 2553, 1715, 1628, 1556, 1486, 1254, 1049, 778, 687

### Example 149

### N-(2-aminophenyl)-4-[N-(pyridin-3-yl)oxyacetylaminomethyl]benzamide 0.7 fumarate (Table 1: fumarate of Compound 61)

As described in Example 146, the title compound was prepared from the compound of Example 46.
mp: 154-155°C
¹ H NMR(270 MHz, DMSO-d6) .delta. ppm: 4.42(2H, d, J=5.9 Hz), 4.69(1H, s), 6.60(1H, t, J=8.0 Hz), 6.63(0.7H, s) 6.78(1H, d, J=8.0 Hz), 6.90-7.50(6H, m), 7.93(2H, d, J=8.0 Hz), 8.20-8.40(2H, m), 8.82(1H, br.s), 9.63(1H, s)
IR(KBr)cm-1 : 3324, 1709, 1631, 1521, 1457, 1428, 1260, 1064, 806, 698 Elementary analysis for C.sub.21 H.sub.20 N.sub.4 O.sub.3 +0.7 C.sub.4 H.sub.4 O.sub.4 +0.7 H.sub.2 O

| | C | H | N |
|---|---|---|---|
| Calculated: | 60.79 | 5.19 | 11.91 |
| Observed: | 60.95 | 5.20 | 11.75 |

Compounds described in EP 0847992 (US 6,174,905) (compounds of formula II) are incorporated by reference.

However, compounds described in EP 0847992 (US 6,174,905) show only minor effects on diseases if applied alone.

However, there is high demand for a medicament, or medicaments, which show a clear effect when applied to cancer or tumors, or diseases as discussed supra. Thus, there is a high demand for a medicament, such as a formulation or combination, respectively, which can overcome these problems.

It has now been found that a combination comprising
a) 9cis-retine acid (CRA), 13cis-retine acid, or a derivative thereof and
   and
b) at least one compound from the group of compounds of formula II) 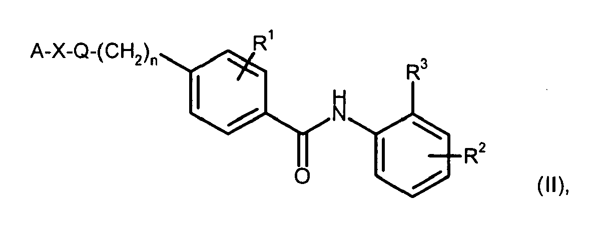 wherein
   A is an optionally substituted phenyl group or an optionally substituted heterocyclic group wherein the substituent(s) for the phenyl group or the heterocyclic group is (are) 1 to 4 substituents selected from the group consisting of a halogen atom, a hydroxyl group, an amino group, a nitro group, a cyano group, an alkyl group having 1 to 4 carbons, an alkoxy group having 1 to 4 carbons, an aminoalkyl group having 1 to 4 carbons, an alkylamino group having 1 to 4 carbons, an acyl group having 1 to 4 carbons, an acylamino group having 1 to 4 carbons, an alkylthio group having 1 to 4 carbons, a perfluoroalkyl group having 1 to 4 carbons, a perfluoroalkyloxy group having 1 to 4 carbons, a carboxyl group, an alkoxycarbonyl group having 1 to 4 carbons, a phenyl group and a heterocyclic group;
   X is a bond or a moiety having the following structure 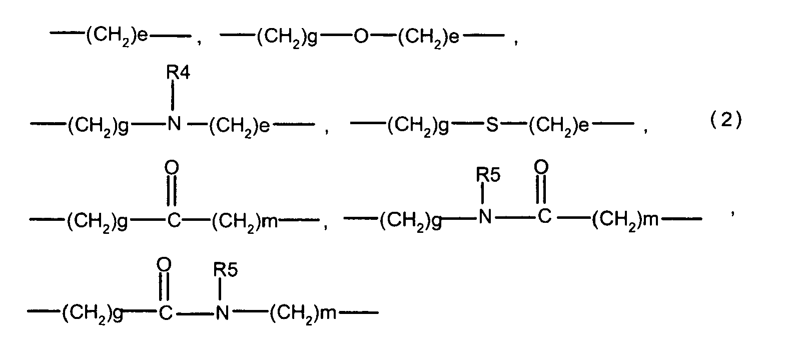 wherein e is an integer of 1 to 4; g and m are independently an integer of 0 to 4; R⁴ is a hydrogen atom or an optionally substituted alkyl group having 1 to 4 carbons, or the acyl group represented by formula (3) 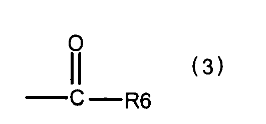 wherein R⁶ is an optionally substituted alkyl group having 1 to 4 carbons, a perfluoroalkyl group having 1 to 4 carbons, a phenyl group or a heterocyclic group; R⁵ is a hydrogen atom or an optionally substituted alkyl group having 1 to 4 carbons;
   n is an integer of 0 to 4, provided that when X is a bond, n is not zero;
   Q is a moiety having a structure selected from those illustrated in formula (4) 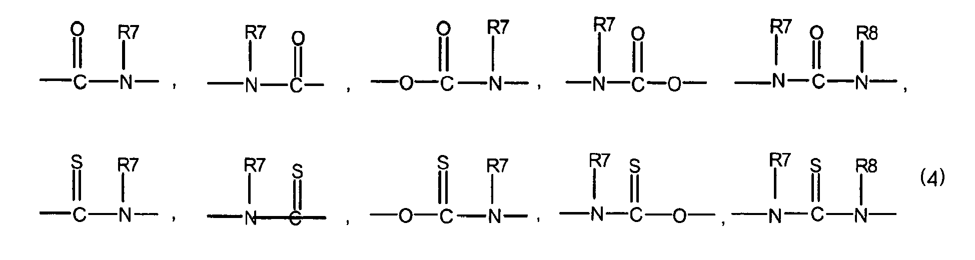 wherein R⁷ and R⁸ are independently hydrogen atom or an optionally substituted alkyl group having 1 to 4 carbons;
   R¹ and R² are independently a hydrogen atom, a halogen atom, a hydroxyl group, an amino group, an alkyl group having 1 to 4 carbons, an alkoxy group having 1 to 4 carbons, an aminoalkyl group having 1 to 4 carbons, an alkylamino group having 1 to 4 carbons, an acyl group having 1 to 4 carbons, an acylamino group having 1 to 4 carbons, an alkylthio group having 1 to 4 carbons, a perfluoroalkyl group having 1 to 4 carbons, a perfluoroalkyloxy group having 1 to 4 carbons, a carboxyl group or an alkoxycarbonyl group having 1 to 4 carbons;
   R³ is a hydroxyl or amino group or a pharmaceutically acceptable salt thereof,
   and
c) at least one anti-hormonal compound taken from the groups comprising anti-oestrogen, anti-progesterone and anti-androgen compounds, overcome the disadvantages of the known single compounds,
will overcome the disadvantages of the known single compounds.

For example, the combination of 9cis-retine acid (CRA) and compounds of general formula II) together with the anti-hormonal compound tamoxifen or derivatives thereof show a rapidly reduction of the tumor mass (s. examples). Beside tamoxifen other anti-hormonal compounds can also be used in combination, such as for example onapristone or derivatives thereof, and dihydrospirorenone or derivatives thereof.

Further, of selected interest as anti-hormonal compounds are the following compounds:
i) at least one anti-hormonal compound taken from the group of anti-oestrogen compounds:
   (Z)-2-[p-(1,2-diphenyl-1-butenyl)phenoxy]-N,N-dimethylethylamine (tamoxifen),
   1-[2-[4-(6-methoxy-2-phenyl-3,4-dihydro-1-naphthyl)phenoxy]ethyl]-pyrrolidine hydrochloride (nafoxidine),
   1-[p-(2-diethylaminoethoxy)phenyl]2-(p-methoxyphenyl)-1-phenylethanol (Mer 25),
   11α-methoxy-17α-ethynyl-1,3,5(10)-estratriene-3,17β-diol, 16β-ethylestradiol,
   11-(3,17β-dihydroxy-1,3,5(10)-estratrien-7α-yl)undecanoic-acid(N-butyl-N-methyl)amide,
   [6-Hydroxy-2-(4-hydroxyphenyl)benzo[b]thien-3-yl-[2-(piperidinyl)-ethoxy]phenyl]methanone,
ii) at least one anti-hormonal compound taken from the group of anti progesterone compounds
   11β-[(4-N,N-dimethylamino)phenyl]-17β-hydroxy-17α-propynyl-4,9(10) estradien-3-one,
   11β-[(4-N,N-dimethylamino)phenyl]-17β-hydroxy-18-methyl-17α-propynyl-4,9(10)-estradien-3-one,
   11β-[(4-N,N-dimethylamino)phenyl]-17α-hydroxy-17aα-propynyl-D-homo-4,9(10)-16-estratrien-3-one,
   11β-p-methoxyphenyl-17β-hydroxy-17β-ethynyl-4,9(10)-estradien-3-one,
   11β-(4-dimethylaminophenyl)-17α-hydroxy-17β-(3-hydroxypropyl)-13-methyl-4,9-gonadien-3-one,
   11β-(4-dimethylaminophenyl)-17α-hydroxy-17-(3-hydroxypropyl)-13α-estra-4,9-dien-3-one (onapristone),
   and
iii) at least one anti-hormonal compound taken from the group of anti-androgen compounds:
   6-chloro-17-hydroxy-1α,2α-methylenepregna-4,6-diene-3,20-dione,
   6-chloro-17-hydroxypregna-4,6-diene-3,20-dione,
   6-chloro-17-hydroxypregna-1,4,6-triene-3,20-dione,
   6-chloro-3,17-dihydroxy-1α,2α-methylenepregna-4,6-diene-20-one,
   6-chloro-3-methoxy-17-hydroxy-1α,2α-methylenepregna-4,6-diene-20-one,
   6-fluoro-17-hydroxy-1α,2α-methylenepregna-4,6-diene-3,20-dione,
   17-hydroxy-1α,2α-methylenepregna-4,6-diene-3,20-dione,
   4,6-dichloro-17-hydroxy-1α,2α-methylenepregna-4,6-diene-3,20-dione,
   6-chloro-17-hydroxy-1α,2α-methylenepregna-4,6-diene-3,20-dione acetate (cyproterone acetat),
   6-chloro-17-hydroxypregna-4,6-diene-3,20-dione acetate (chlormadione acetate),
   6-chloro-17αβ-acetoxy-17aα-methyl-1α,2α-methylene-D-homo-4,6-androstadiene-3,17-dione,
   6-chloro-17α-acetoxy-17β-methyl-1α,2α-methylene-D-homo-4,6-androstadiene-3,17a-dione,
   2-methyl-N-[4-nitro-3-(trifluoromethyl)phenyl]-propionamide (flutamide),
   2-hydroxy-2-methyl-N-[4-nitro-3-(trifluoromethyl)phenyl]-propionamide,
   2-methyl-4-[4-nitro-3-(trifluoromethyl)phenyl]-5,6-dihydro-2H-1,2,4-oxadiazin-3-one,
   6β,7β,15β,16β-dimethylien-3-oxo-17α-pregn-4-ene-21,17-carbolactone (dihydrospirorenone).

Of special interest are those combinations wherein the antihormone compounds are tamoxifen, onapristone, and dihydrospirorenone, or derivatives thereof.

Of special interest are those combinations wherein b) comprises at least one compound from the group of compounds of formula II), wherein n is an integer of 1 to 4.

Of further special interest are those combinations wherein b) comprises at least one compound from the group of compounds of general formula II), wherein Q is selected from the structures illustrated in formula (5). 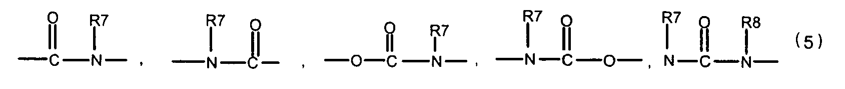 wherein R⁷ and R⁸ are as defined above.

Further of interest are those combinations wherein b) comprises at least one compound from the group of compounds of formula II), wherein A is an optionally substituted hetero ring, especially an optionally substituted pyridyl group.

Of special interest are also those combinations wherein b) comprises at least one compound from the group of compounds of of formula II), wherein n is 1 to 4; Q is selected from the structures illustrated in formula (5); A is an optionally substituted hetero ring, especially optionally substituted pyridyl group; most preferred, wherein X is direct bond, most preferred wherein R¹ and R² are a hydrogen atom, most preferred, wherein R³ is an amino group.

Also, of special interest are those combinations wherein b) comprises at least one compound from the group of compounds of of formula II), wherein Q is selected from the structures illustrated in formula (5); A is an optionally substituted hetero ring, especially optionally substituted pyridyl group; most preferred, wherein X is the structure represented by formula (6):

-(CH₂)e- (6)

wherein e is an integer of 1 to 4; most preferred wherein n is 1 and R¹ and R² are a hydrogen atom; most preferred, wherein R³ is an amino group.

Also of special interest are those combinations wherein b) comprises at least one compound from the group of compounds of formula II), wherein Q is selected from the structures illustrated in formula (5); A is an optionally substituted hetero ring, especially optionally substituted pyridyl group; most preferred, wherein X is selected from the structures illustrated in formula (7): 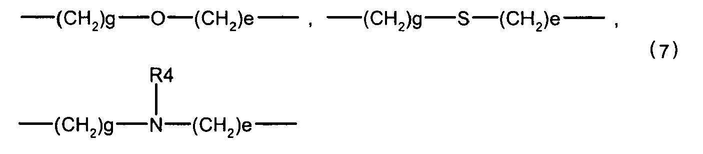 wherein e, g and R⁴ are as defined above; most preferred wherein n is 1 and R¹ and R² are a hydrogen atom; most preferred, wherein R³ is an amino group.

Interesting combinations wherein b) comprises at least one compound from the group of compounds of of formula II), wherein Q is selected from the structures illustrated in formula (5); A is an optionally substituted hetero ring, especially optionally substituted pyridyl group; most preferred, wherein X is selected from the structures illustrated in formula (8): 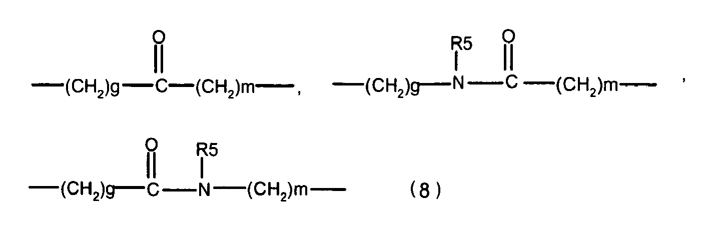 wherein g, m and R⁵ are as defined above; most preferred wherein n is 1 and R¹ and R² are a hydrogen atom; most preferred, wherein R³ is an amino group.

Of special interest are also those combinations, wherein b) comprises at least one compound from the group of compounds of formula II), wherein n is zero, and most preferred, Q is selected from the structures illustrated in formula (5); and most preferred, wherein A is an optionally substituted hetero ring; most preferred, wherein A is an optionally substituted pyridyl group; most preferred, wherein R¹ and R² are a hydrogen atom; most preferred, wherein R³ is an amino group.

Selected compounds of general formula II are for example the following compounds: 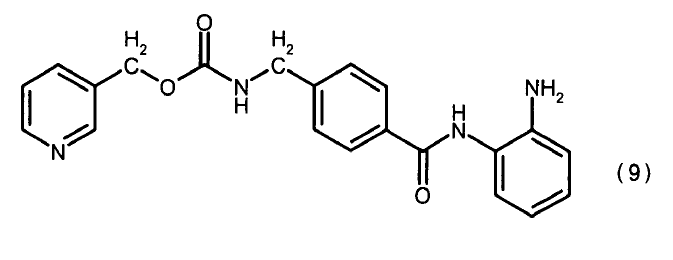 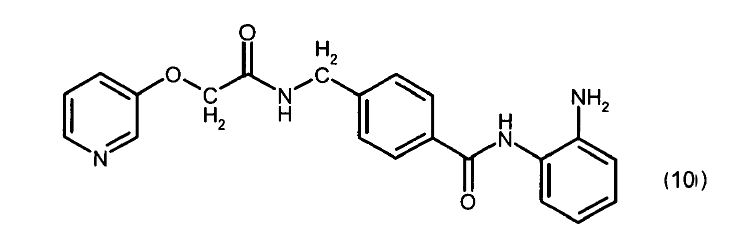 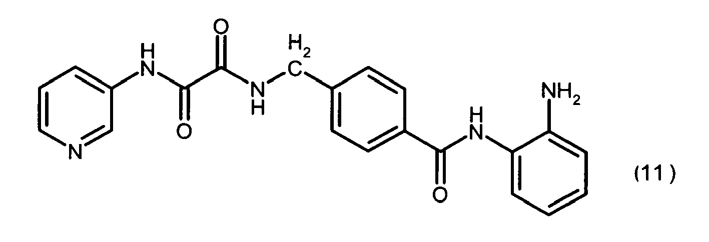 and

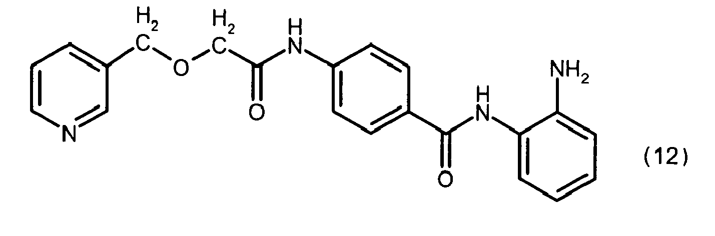

The invention also comprises the combination with anti-hormones, such as anti-oestrogens and anti-progesterones, as described in EP 0062 503, and anti-androgenes, as described in US 5,053,405 and DE 31 211 52.

The inventive combinations can be used with at least one pharmaceutically acceptable diluent or carrier and can be used as a combined preparation simultaneously, separately or sequentially.

The invention also comprises a kit, comprising the inventive pharmaceutically active combination wherein the 9cis-retine acid (CRA), 13cis-retine acid, or a derivative thereof, and compound(s) of general formula II) and the anti-hormonal compound(s) as a combined preparation are simultaneously, separately or sequentially used.

The inventive combinations can be used for enteral administration, such as nasal, buccal, rectal or, expecially, oral administration, and for parenteral administration, such as intravenous, intramuscular or subcutaneaous administration, to warm-blooded animals, especially, humans, are especially preferred. The compositions comprise the active ingredient alone or, preferably, together with a pharmaceutically acceptable carrier. The dosage of the active ingredient depends upon the disease to be treated and upon the species, gender, age, weight, and individual condition, the individual pharmacokinetic data, and the mode of administration.

The inventive combinations can also used as a method for the prophylactic or especially therapeutic management of the human or animal body, to a process for the preparation thereof (especially in the form of compositions for the treatment of tumours) and to a method of treating tumour diseases, especially those mentioned hereinabove.

In the preferred embodiment, the pharmaceutical combinations is suitable for administration to a warm-blooded animal, especially humans or commercially useful mammals suffering form a disease responsive to an inhibition of angiogenesis or of VEGF-receptor tyrosine kinase, for example psoriasis or especially a neoplastic disease, and comprises an effective quantity of a compounds for the inhibition of angiogenesis or of VEGF-receptor tyrosine kinase, or a pharmaceutically acceptable salt thereof, if salt-forming groups are present, together with at least one pharmaceutically acceptable carrier.

The inventive combinations can be used for the prophylactic or especially therapeutic management of neoplastic and other proliferative diseases of a warm-blooded animal, especially a human or a commercially useful mammal requiring such treatment, especially suffering from such a disease.

The inventive combinations comprise from approximately 1% to approximately 95% active ingredient, single-dose administration forms comprising in the preferred embodiment from approximately 5% to approximately 20% active ingredient. Unit dose forms are, for example, coated and uncoated tablets, ampoules, vials, suppositories or capsules. Further dosage forms are, for example, ointments, creams, pastes, foams, tinctures, lip-sticks, drops, sprays, dispersions, etc. Examples are capsules containing from about 0.05 g to about 1.0 g active ingredients.

The pharmaceutical combination of the present invention are prepared in a manner known per se, for example by means of conventional mixing, granulating, coating, dissolving or lyophilizing processes.

Preference is given to the use of solutions of the active ingredient, and also suspensions or dispersions, especially isotonic aqueous solutions, dispersions or suspensions which, for example in the case of lyophilized compositions comprising the active ingredients alone or together with a carrier, for example mannitol, can be made up before use. The pharmaceutical compositions may be sterilized and/or may comprise excipients, for example preservatives, stabilizers, wetting agents and/or emulsifiers, solubilizers, salts for regulating osmotic pressure and/or buffers and are prepared in a manner known per se, for example by means of conventional dissolving and lyophilizing processes. The said solutions or suspensions may comprise visosity-increasing agents, typically sodium carboxymethylcellulose, carboxymethylcellulose, dextran, polyvinylpyrrolidone, or gelatins, or also solubilizers, for example Tween 80 [polyoxyethylene(20)sorbitan mono-oleate; trademark of ICI Americas, Inc. USA].

Suspensions in oil comprise as the oil component the vegetable, synthetic, or semi-synthetic oils customary for injection purposes. In respect of such, special mention may be made of liquid fatty acid esters that contain as the acid component a long-chained fatty acid having from 8 to 22, expecially from 12 to 22, carbon atoms, for example lauric acid, tripdecylic acid, myristic acid, pentadecylic acid, palmitic acid, margaric acid, stearic acid, arachidic acid, behenic acid or corresponding unsaturated acids, for example oleaic acid, elaidic acid, erucic acid, brassidic acid or linoleic acid, if desired with the addition of anti-oxidants, for example vitamine E, β-carotene or 3,5-di-tert-butyl-4-hydroxytoluene. The alcohol component of these fatty acid esters has a maximum of 6 carbon atoms and is a monovalent or polyvalent, for example a mono-, di- or trivalent, alcohol, for example methanol, ethanol, propanol, butanol or pentanol or the isomers thereof, but especially glycol and glycerol. As fatty acid esters, therefore, the following are mentioned: ethyl oleate, isopropyl myristate, isopropyl palmitate, "Labrafil M 2375" (polyoxyethylene glycerol trioleate from Gattefossé, Paris), "Labrafil M 1944 CS" (unsaturated polyglycolized glycerides prepared by alcoholysis of apricot kernel oil and consisting of glycerides and polyethylene glycol ester; Gattefossé, France), "Labrasol" (saturated polyglycolized glycerides prepared by alcoholysis of TCM and consistitn of glycerides and polyethylene glycol ester; Gattefossé, France), and/or "Miglyol 812" (triglyceride of saturated fatty acids of chain length C₈ to C₁₂ from Hüls AG, Germany), but especially vegetable oils such as cottonseed oil, almond oil, olive oil, castor oil, sesame oil, soybean oil and more expecially groundnut oil.

The manufacture of injectable preparations is usually carried out under sterile conditions, as is filling, for example into ampoules or vials, and the sealing of the containers.

Pharmaceutical compositions for oral administration can be obtained, for example, by combining the active ingredient with one or more solid carriers, if desired granulating a resulting mixture, and processing the mixture of granules, if desired or necessary, by the inclusion of additional excipients, to form tablets or tablet cores.

Suitable carriers are especially fillers, such as sugars, for example lactose, saccharose, mannitol or sorbitol, cellulose preparations, and/or calcium phosphates, for example tricalcium phosphate or calcium hydrogen phosphate, and also binders, such as starches, for example corn, wheat, rice or potato starch, methylcellulose, hydroxypropyl methylcellulose, sodium carboxymethylcellulose, and/or polyvinylpyrrolidone, and/or, if desired, disintegrators, such as the above-mentioned starches, also carboxymethyl starch, crosslinked polyvinylpyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Tablet cores can be provided with suitable, optionally enteric, coatings through the use of, *inter alia,* condentrated sugar solutions, which may comprise gum arabic, talc, polyvinylpyrrolidone, polyethzlene glycol and/or titanium dioxide, or coating solutions in suitable organic solvents or solvent mixture, or for the preparation of enteric coatings, solutions of suitable cellulose preparations, such as acetly cellulose phthalate or hydroxypropylmethylcellulose phthalate. Dyes or pigments may be added to the tablets or tablet coatings, for example for identification purposes or to indicate different doses of active ingredient.

Pharmaceutical compostions for oral administration also include hard capsules consisting of gelatin, and also soft, sealed capsules consisting of gelatin and plasticizer, such as glycerol or sorbitol. The hard capsules may contain the active ingredient in the form of granules, for example in admixture with fillers, such as corn starch, binders, and/or glidants, such as talc or magnesium stearate, and optionally stabilizers. In soft capsules, the active ingredient is preferably dissolved or suspended in suitable liquid excipients, such as fatty oils, paraffin oil or liquid polyethylene glycols or fatty acid esters of ethylene or propylene glycol, to which stabilizers and detergents, for example of the polyoxyethylene sorbitan fatty acid ester type, may also be added.

Other oral dosage forms are, fior example syrups prepared in customary manner which comprise the active ingredient, for example, in suspended form and in a concentratin of about 5% to 20%, preferably about 10%, or in similar concentration that provides a suitable single dose, for example, when administered in measures of 5 or 10 ml. Also suitable are, for example, powdered or liquid concentrates for the preparation of shakes, for example in milk. Such concentrates may also be packaged in single-dose units.

Pharmaceutical compositions suitable for rectal administration are, for example, suppositories that consist of acombination of the active ingredient and a suppository base. Suitable suppository bases are, for example, naturral or synthetic triglycerides, paraffin hydrocarbons, polyethylene glycols or higher alkanols.

For prenteral administration, aqueous solutions of an active ingredient in water-soluble form, for example of a water-soluble salt, or aqueous injection suspensions that contain viscosity-increasing substances, for example sodium carboxymethylcellulose, sorbitol and/or dextran, and, if desired, stabilizers, are especially suitable. The active ingredient, optionally together with excipients, can also be in the form of a lyophilizate and can be made into a solution before parenteral administration by the addition of suitable solvents.

Solutions such as are used, for example, for parenteral administration can also be employed as infusion solutions.

Preferred preservatives are, for example, antioxidants, such as ascorbic acid, or micro-bicides, such as sorbic acid or benzoic acid.

The invention relates likewise to a process or a method for the treatment of one of the pathological conditions mentioned herineabove, especially a disease which responds to an inhibition of the VEGF-receptor tyrosine kinase or an inhibition of angiogenesis, especially a corresponding neoplastic disease or also psoriasis. The combination can be administered as such or especially in the form of pharmaceutical compositions, prophylactically or therapeutically, preferably in an amount effective against the said diseases, to a warm-blooded animal, for example a human, requiring such treatment. In case of an individual having a bodyweight of about 70 kg the daily dose administered is from approimately 0.1 g to approximately 5 g, preferably from approximately 0.5 g to approximately 2 g, of a compound of the present invention.

The present invention relates especially also to the use of the combination, as such or in the form of a pharmaceutical formulation with at least one pharmaceutically acceptable carrier for the therapeutic and also prophylactic management of one or more of the diseases mentioned hereinabove, especially a neoplastic disease or also psoriasis, more especially if the disease responds to an inhibition of angiogenesis or an inhibition of VEGF-receptor tyrosine kinase.

The present invention relates especially also to the use of the combination, as such or in the form of a pharmaceutical formulation with at least one pharmaceutically acceptable carrier for the therapeutic and also prophylactic management of one or more of the diseases mentione hereinabove, preferably a disease which responds to an inhibition of VGEF-receptor tyrosine kinase or an inhibition of angiogenesis, especially a neoplastic disease or also psoriasis, more especially if the said disease responds to an inhibition of VEGF-receptor tyrosine kinase or angiogenesis.

The present invention relates especially also to the use of the combination for the preparation of a pharmaceutical formulation for the therapeutic and also prophylactic managment of one or more of the diseases mentioned hereinabove, especially a neoplastic disease or also psoriasis, more especially if the disease responds to an inhibition of VEGF-receptor tyrosine kinase or angiogenesis.

The combination of this invention has differentiation-inducing effects and thus is useful as a therapeutic and/or improving combined agent to a variety of diseases such as malignant tumors, autoimmicro ne diseases, dermatologic diseases and parasitism.

As used herein, a "malignant tumor" includes hematologic malignancy such as acute leukemia, malignant lymphoma, micro Itiple myeloma and macroglobulinemia as well as solid tumors such as colon cancer, cerebral tumor, head and neck tumor, breast carcinoma, pulmonary cancer, esophageal cancer, gastric cancer, hepatic cancer, gallbladder cancer, bile duct cancer, pancreatic cancer, nesidioblastoma, renal cell carcinoma, adrenocortical cancer, urinary bladder carcinoma, prostatic cancer, testicular tumor, ovarian carcinoma, uterine cancer, chorionic carcinoma, thyroid cancer, malignant carcinoid tumor, skin cancer, malignant melanoma, osteogenic sarcoma, soft tissue sarcoma, neuroblastoma, Wilms tumor and retinoblastoma.

An autoimmicrone disease includes rheumatism, such as rheumatoide arthritis, diabetes, systemic lupus erythematodes, human autoimmicrone lymphocytotic lymphadenopathy, immicro noblastic lymphadenopathy, Crohn's disease and ulcerative colitis.

A dermatologic disease includes psoriasis, acne, eczema and atopic dermatitis.

Parasitism includes diseases such as malaria caused through vermination.

Further, the inventive combination can be used for the treatment haemeangioma, angiofribroma, diseases of the eyes, such as diabetic retinopathie, neovascular glaucoma, diseases of the kidney, such as glomerulonephritis, diabetic nephropatic diseases, malignant nephrosclerosis, thrombotic microangiopatic syndrome, disposes of transplants and glomerulopathy, fibrotic diseases, such as liver cirrhosis, mesangialic cell proliferative diseases and artheriosclerosis, injury of the nervous tissues, for inhibition of reocclusion of vascular systems after balloon catheter treatment, for artificial limbs, or after insert of mechanically devices for keeping open of vasculature, such as stents.
For the treatment of injury of the nervous tissues a rapid production of scars at the place of injury can be prevented. Thus, production of scars will be prevented before the axones can re-establish. Therefore a re-construction of nerves is possible.
Further, by using the inventive combination the ascites production within patients can be suppressed. Also, oedema resulted by VEGF can be suppressed.
Indications for the combination of this invention are not limited to these specific examples.

The active ingredient of the combination useful as a drug may be used in the form of a general pharmaceutical composition. The pharmaceutical composition maybe prepared with generally used diluents or excipients such as filler, extender, binder, moisturizing agent, disintegrator, surfactant and lubricant. The pharmaceutical composition may have a variety of dosage forms depending on its therapeutic purpose; typically tablet, pill, powder, solution, suspension, emulsion, granule, capsule, injection (e.g., solution, suspension) and suppository.

For preparing tablets, a variety of carriers well-known in the art may be used. Such a carrier includes excipients such as lactose, glucose, starch, calcium carbonate, kaoline, crystalline cellulose and silicic acid; binders such as water, ethanol, propanol, simple syrup, glucose solution, starch solution, gelatin solution, carboxymethyl cellulose, shellac, methyl cellulose and polyvinyl pyrrolidone; disintegrators such as dried starch, sodium alginate, powdered agar, calcium carmelose, starch and lactose; disintegration retarders such as sucrose, cocoa butter and hydrogenated oil; absorption promoters such as quaternary ammonium base and sodium lauryl sulfate; moisturizing agents such as glycerin and starch.; adsorbents such as starch, lactose, kaoline, bentonite, colloidal silicic acid; and glidants such as talc, stearates and polyethylene glycol. The tablet may be, if necessary, one coated with a common coating; for example, sugar-coated tablet, gelatin-coated tablet, enteric coated tablet, film-coated tablet, double-layer tablet and micro ltilayer tablet.

In forming pills, a variety of carriers well-known in the art may be used. Such a carrier includes excipients such as crystalline cellulose, lactose, starch, hydrogenated vegetable oil, kaoline and talc; binders such as powdered acacia, powdered tragacanth gum and gelatin; disintegrators such as calcium carmelose and agar.

Capsule may be prepared by blending an active ingredient with a variety of the above carriers as usual and filling the resulting blend into, for example, a hard or soft gelatin capsule or the like.

For preparing injection, solution, emulsion and suspension are sterilized and preferably isotonic with blood. It may be prepared using diluents commonly used in the art; for example, water, ethanol, macrogol, propylene glycol, ethoxylated isostearyl alcohol, polyoxyisostearyl alcohol and polyoxyethylene sorbitan fatty acid esters. The pharmaceutical preparation may contain sodium chloride necessary to prepare an isotonic solution, glucose or glycerin, as well as usual solubilizers, buffers and soothing agents.

Suppository may be formed using a variety of well-known carriers; for example, semi-synthetic glyceride, cocoa butter, higher alcohols, higher alcohol esters and polyethylene glycol.

Furthermore, the pharmaceutical combination may contain coloring agents, preservatives, perfumes, flavors, sweeteners and/or other drugs.

The amount of the active ingredient in the pharmaceutical combination of this invention may be, as appropriate, selected from a wide range with no limitations, and is generally about 1 to 70% by weight in the composition, preferably about 5 to 50% by weight.

An administration route of the pharmaceutical combination is not limited, and selected depending on patient's age, sex, severity of disease and other conditions. For example, tablet, pill, solution, suspension, emulsion, granule and capsule may be orally administered; injection may be intravenously administered solely or in combination with a common infusion fluid such as glucose, amino acids and the like, or if necessary, intramicro scularly, subcutaneously or intraperitoneally as a sole preparation. Suppository may be intrarectally administered.

Dose of the pharmaceutical combination of this invention may be selected, depending on their dosage form, patient's age, sex and severity of disease, and other conditions, as appropriate, but the amount of the active ingredient may be generally about 0.0001 to 100 mg/kg a day. It is recommended that a unit dosage form may contain about 0.001 to 1000 mg of the active ingredient.

### Biological Examples

### Example 1

### Combination of 9cis-retine acid (CRA) and 3-pyridylmethyl-N-(4-[(2-amino-phenyl)-carbamoyl]benzyl)carbamate (MS) with Tamoxifen (TAM)

CC531 and HCT116 cells were cultured as decribed in Example 1. The experiment was carried out under the same conditions as described in Example 1.
The % of apotosis induction after 72 h was determined. The data were calculated on basis of three separate experiments.

The results are shown in the following table:

| **Compound** | **CC531** | **HCT116** |
|---|---|---|
| **TAM/CRA 10-5M** | 7 ± 3 | 14 ± 3 |
| **TAM/CRA/MS 10-5M** | 34 ± 9 | 75 ± 12 |

The results show a significant apoptose induction of the combination of CRA/MS/Tamoxifen in both cell lines.

## Claims

1. A combination comprising
a) 9cis-retine acid (CRA), 13cis-retine acid, or a derivative thereof and
b) at least one compound from the group of compounds of formula II) 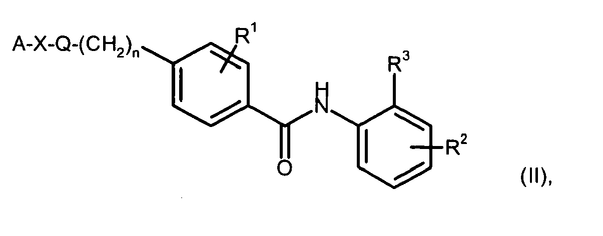 wherein
A is an optionally substituted phenyl group or an optionally substituted heterocyclic group wherein the substituent(s) for the phenyl group or the heterocyclic group is (are) 1 to 4 substituents selected from the group consisting of a halogen atom, a hydroxyl group, an amino group, a nitro group, a cyano group, an alkyl group having 1 to 4 carbons, an alkoxy group having 1 to 4 carbons, an aminoalkyl group having 1 to 4 carbons, an alkylamino group having 1 to 4 carbons, an acyl group having 1 to 4 carbons, an acylamino group having 1 to 4 carbons, an alkylthio group having 1 to 4 carbons, a perfluoroalkyl group having 1 to 4 carbons, a perfluoroalkyloxy group having 1 to 4 carbons, a carboxyl group, an alkoxycarbonyl group having 1 to 4 carbons, a phenyl group and a heterocyclic group;
X is a bond or a moiety having the following structure 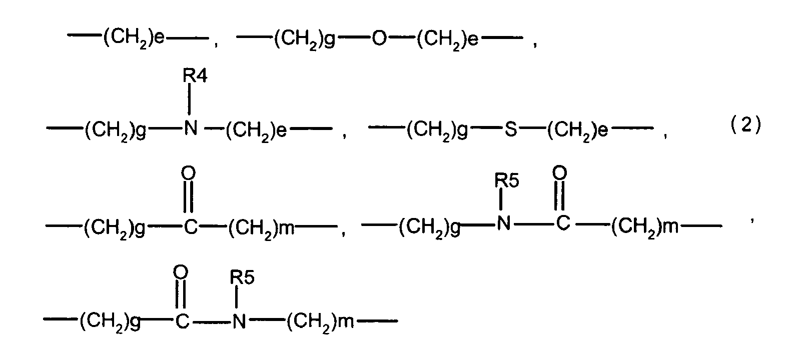 wherein e is an integer of 1 to 4; g and m are independently an integer of 0 to 4; R⁴ is a hydrogen atom or an optionally substituted alkyl group having 1 to 4 carbons, or the acyl group represented by formula (3) 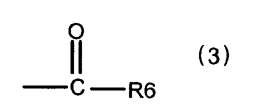 wherein R⁶ is an optionally substituted alkyl group having 1 to 4 carbons, a perfluoroalkyl group having 1 to 4 carbons, a phenyl group or a heterocyclic group; R⁵ is a hydrogen atom or an optionally substituted alkyl group having 1 to 4 carbons;
n is an integer of 0 to 4, provided that when X is a bond, n is not zero; wherein
Q is a moiety having a structure selected from those illustrated in formula (4) 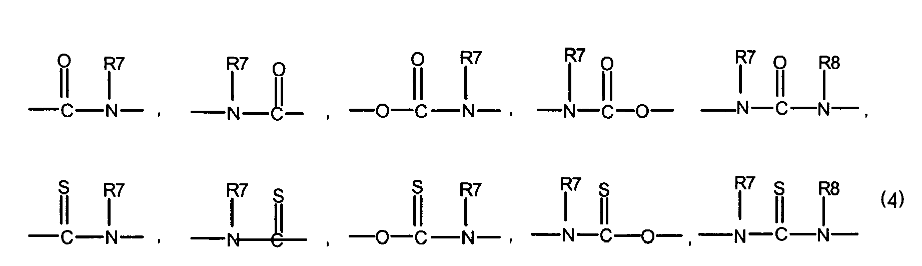 wherein R⁷ and R⁸ are independently hydrogen atom or an optionally substituted alkyl group having 1 to 4 carbons;
R¹ and R² are independently a hydrogen atom, a halogen atom, a hydroxyl group, an amino group, an alkyl group having 1 to 4 carbons, an alkoxy group having 1 to 4 carbons, an aminoalkyl group having 1 to 4 carbons, an alkylamino group having 1 to 4 carbons, an acyl group having 1 to 4 carbons, an acylamino group having 1 to 4 carbons, an alkylthio group having 1 to 4 carbons, a perfluoroalkyl group having 1 to 4 carbons, a perfluoroalkyloxy group having 1 to 4 carbons, a carboxyl group or an alkoxycarbonyl group having 1 to 4 carbons;
R³ is a hydroxyl or amino group or a pharmaceutically acceptable salt thereof,
and
c) at least one anti-hormonal compound taken from the groups comprising anti-oestrogen, anti-progesterone and anti-androgen compounds.

2. A combination according to claim 1, comprising
a) 9cis-retine acid (CRA),
and
b) at least one compound of general formula II), as claimed in claim 1,
and
c) at least one anti-hormonal compound taken from the groups comprising anti-oestrogen, anti-progesterone and anti-androgen compounds.

3. A combination according to any of claims 1-2, wherein the anti-hormonal compounds are
i) at least one anti-hormonal compound taken from the group of anti-oestrogen compounds:
(Z)-2-[p-(1,2-diphenyl-1-butenyl)phenoxy]-N, N-dimethylethylamine (tamoxifen),
1-[2-[4-(6-methoxy-2-phenyl-3,4-dihydro-1-naphthyl)phenoxy]-ethyl]-pyrrolidine hydrochloride (nafoxidine),
1-[p-(2-diethylaminoethoxy)phenyl]2-(p-methoxyphenyl)-1-phenylethanol (Mer 25),
11α-methoxy-17α-ethynyl-1,3,5(10)-estratriene-3,17β-diol, 16β-ethylestradiol,
11-(3,17β-dihydroxy-1,3,5(10)-estratrien-7α-yl)undecanoic-acid-(N-butyl-N-methyl)amide,
[6-Hydroxy-2-(4-hydroxyphenyl)benzo[b]thien-3-yl-[2-(piperidinyl)ethoxy]phenyl]methanone,
ii) at least one anti-hormonal compound taken from the group of anti progesterone compounds
11β-[(4-N, N-dimethylamino)phenyl]-17β-hydroxy-17α-propynyl-4,9(10) estradien-3-one,
11β-[(4-N,N-dimethylamino)phenyl]-17β-hydroxy-18-methyl-17α-propynyl-4,9(10)-estradien-3-one,
11β-[(4-N,N-dimethylamino)phenyl]-17aβ-hydroxy-17aα-propynyl-D-homo-4,9(10)-16-estratrien-3-one,
11β-p-methoxyphenyl-17β-hydroxy-17α-ethynyl-4,9(10)-estradien-3-one,
11β-(4-dimethylaminophenyl)-17α-hydroxy-17β-(3-hydroxypropyl)-13-methyl-4,9-gonadien-3-one,
11β-(4-dimethylaminophenyl)-17α-hydroxy-17-(3-hydroxypropyl)-13α-estra-4,9-dien-3-one (onapristone),
and
iii) at least one anti-hormonal compound taken from the group of anti-androgen compounds:
6-chloro-17-hydroxy-1 α,2α-methylenepregna-4,6-diene-3,20-dione,
6-chloro-17-hydroxypregna-4,6-diene-3,20-dione,
6-chloro-17-hydroxypregna-1,4,6-triene-3,20-dione,
6-chloro-3,17-dihydroxy-1α,2α-methylenepregna-4,6-diene-20-one,
6-chloro-3-methoxy-17-hydroxy-1α,2α-methylenepregna-4,6-diene-20-one,
6-fluoro-17-hydroxy-1α,2α-methylenepregna-4,6-diene-3,20-dione,
17-hydroxy-1α,2α-methylenepregna-4,6-diene-3,20-dione,
4,6-dichloro-17-hydroxy-1α,2α-methylenepregna-4,6-diene-3,20-dione,
6-chloro-17-hydroxy-1α,2α-methylenepregna-4,6-diene-3,20-dione acetate (cyproterone acetat),
6-chloro-17-hydroxypregna-4,6-diene-3,20-dione acetate (chlormadione acetate),
6-chloro-17αβ-acetoxy-17aα-methyl-1α,2α-methylene-D-homo-4,6-androstadiene-3,17-dione,
6-chloro-17α-acetoxy- 175-methyl-1α,2α-methylene-D-homo-4,6-androstadiene-3,17α-dione,
2-methyl-N-[4-nitro-3-(trifluoromethyl)phenyl]-propionamide (flutamide),
2-hydroxy-2-methyl-N-[4-nitro-3-(trifluoromethyl)phenyl]-propionamide,
2-methyl-4-[4-nitro-3-(trifluoromethyl)phenyl]-5,6-dihydro-2H-1,2,4-oxadiazin-3-one,
6β,7β,15β,16β-dimethylen-3-oxo-17α-pregn-4-ene-21,17-carbolactone (dihydrospirorenone).

4. A combination according to any of claims 1 - 3, wherein the antihormone compounds are tamoxifen, onapristone, and dihydrospirorenone, or derivatives thereof.

5. A combination according to any of claims 1 to 4, wherein b) comprises at least one compound from the group of compounds of formula II), wherein n is an integer of 1 to 4.

6. A combination according to claim 5, wherein b) comprises at least one compound from the group of compounds of formula II), wherein Q is selected from the structures illuctrated in formula (5): 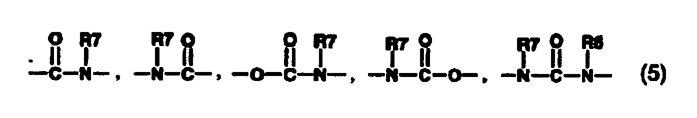 wherein R⁷ and R⁸ are as defined above.

7. A combination according to claim 6, wherein b) comprises at least one compound from the group of compounds of formula II), wherein A is an optionally substituted hetero ring.

8. A combination according to claim 7 wherein b) comprises at least one compound from the group of compounds of formula II), wherein A is an optionally substituted pyridyl group.

9. A combination according to claim 8 wherein b) comprises at least one compound from the group of compounds of formula II), wherein X is a direct bond.

10. A combination according to claim 9, wherein b) comprises at least one compound from the group of compounds of formula II), wherein R¹ and R² are a hydrogen atom.

11. A combination according to claim 10, wherein b) comprises at least one compound from the group of compounds of formula II), wherein R³ is an amino group.

12. A combination according to claim 8, wherein b) comprises at least one compound from the group of compounds of formula II), wherein X is the structure represented by formula (6):
- (CH₂)ₑ - (6)
wherein e is as defined above.

13. A combination according to claim 12, wherein b) comprises at least one compound from the group of compounds of formula II), in which n is 1; and R¹ and R² are a hydrogen atom.

14. A combination according to claim 13, wherein b) comprises at least one compound from the group of compounds of formula II), wherein additionally R³ is an amino group.

15. A combination according to claim 8, wherein b) comprises at least one compound from the group of compounds of formula II), wherein X is selected from the structures illustrated in formula (7): 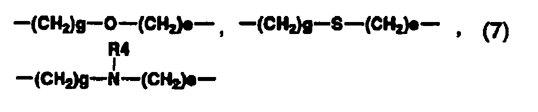 wherein e, g and R⁴ are as defined above.

16. A combination according to claim 15, wherein b) comprises at least one compound from the group of compounds of formula II), wherein n is 1 and R¹ and R² are a hydrogen atom.

17. A combination according to claim 16, wherein b) comprises at least one compound from the group of compounds of formula II), wherein R³ is an amino group.

18. A combination according to claims 8, wherein b) comprises at least one compound from the group of compounds of formula II), wherein X is selected from the structures illustrated in formula (8): 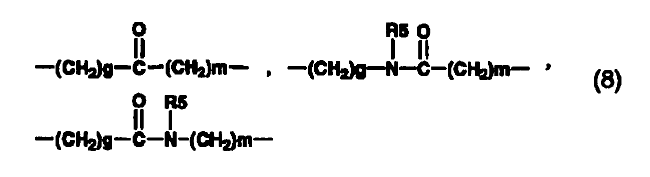 wherein g, m and R⁵ are as defined above.

19. A combination according to claim 18, wherein b) comprises at least one compound from the group of compounds of formula II), wherein n is 1; and R¹ and R² are a hydrogen atom.

20. A combination according to claim 19, wherein b) comprises at least one compound from the group of compounds of formula II), wherein R³ is an amino group.

21. A combination according to any of claims 1 to 2, wherein b) comprises at least one compound from the group of compounds of formula II), wherein n is zero.

22. A combination according to claim 21, wherein b) comprises at least one compound from the group of compounds of formula II), wherein Q is selected from the structures illustrated in formula (5): 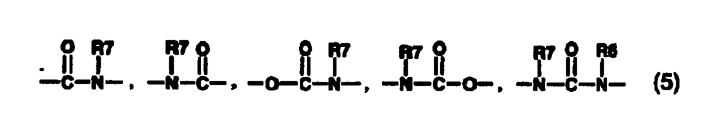 wherein R⁷ and R⁸ are as defined above.

23. A combination according to any of claim 22, wherein b) comprises at least one compound from the group of compounds of formula II), wherein A is an optionally substituted pyridyl group, and wherein R¹ and R² are a hydrogen atom, and wherein R³ is an amino group.

24. A combination according to any of claims 1 to 2, wherein b) comprises at least one compound of the following structure: 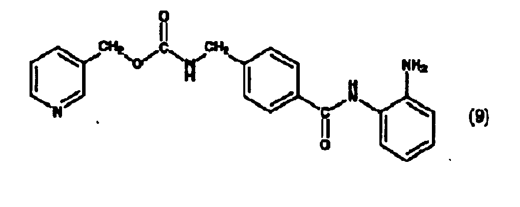 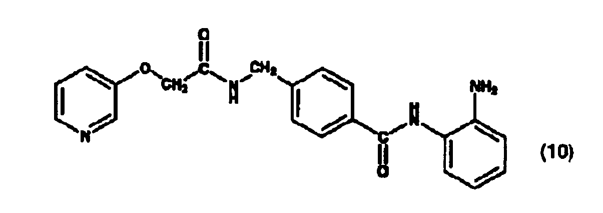 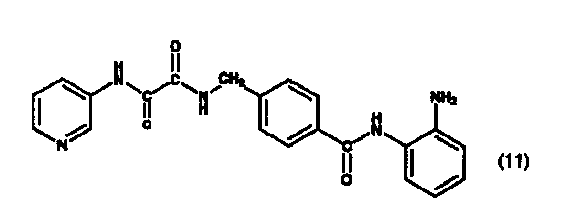 and

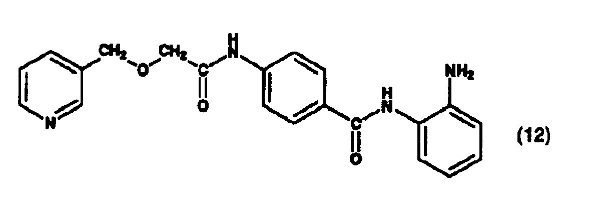

25. A combination according to any one of claims 1 to 24, comprising
a) 9cis-retine acid (CRA),
and
b) 3-pyridylmethyl-N-{4-[(2-amino-phenyl)carbamoyl]benzyl}-carbamate,
and
c) tamoxifen.

26. A kit, comprising the combination according to any of claims 1 to 25, wherein 9cis-retine acid (CRA), 13cis-retine acid, or a derivative thereof, the compound(s) of general formula II) and the anti-hormonal compound(s) as a combined preparation are simultaneously, separately or sequentially used.

27. Use of the combination according to any of claims 1 to 25, for the treatment of haemeangioma, angiofribroma, diseases of the eyes, such as diabetic retinopathie, neovascular glaucoma, diseases of the kidney, such as glomerulonephritis, diabetic nephropatic diseases, malignant nephrosclerosis, thrombotic microangiopatic syndrome, disposes of transplants and glomerulopathy, fibrotic diseases, such as liver cirrhosis, mesangialic cell proliferative diseases and artheriosclerosis, injury of the nervous tissues, for inhibition of reocclusion of vascular systems after balloon catheter treatment, for artificial limbs, or after insert of mechanically devices for keeping open of vasculature, such as stents.

28. Use of the combination according to any of claims 1 to 25, for the treatment of injury of the nervous tissues.

29. Use of the combination according to any of claims 1 to 25, for the suppression of oedema resulted by VEGF.

30. Use of a combination according to any one of claims 1 to 25, for the production of a medicament for the use in a human or an animal body.

31. A combination according to any one of claims 1 to 25, together with at least one pharmaceutically acceptable carrier, or excipient.

32. Use of a combination according to any one of claims 1 to 25, for the preparation of a pharmaceutical product for the treatment of a disease which responds to an inhibition of angiogenesis.

33. Use of a combination according to any one of claims 1 to 25, for the preparation of a pharmaceutical product for the treatment of a disease which responds to an inhibition of VEGF-receptor tyrosine kinase.

34. Use of a combination according to any one of claims 1 to 25, for the treatment of a disease wich responds to an inhibition of VEGF-receptor kinase.

35. Use of a combination according to any one of claims 1 to 25, which is suitable for administration to a warm-blooded animal, especially suffering from a disease which responds to an inhibition of angiogenesis or of VEGF-receptor tyrosine kinase, comprising an effective quantity of the combination, together with at least one pharmaceutically acceptable carrier.

36. A method for treatment of disease which responds to an inhibition of VEGF-receptor tyrosine kinasse or an inhibition of angiogenesis, which comprises administering a combination according to any one of claims 1 to 25, in a compound quantity effective against the said diseases, to a warm-blooded animal requiring such treatment in a compound quantity suitable for the treatment of the said disease.
